# EUROPEAN PATENT APPLICATION

(11) **EP 4 837 589 A1**
(43) Date of publication of application: **11.11.2026**
(21) Application number: 25751868.8
(22) Date of filing: 04.02.2025
(51) Int. Cl.: A61K 47/10, A61K 31/7088, A61K 38/17, A61K 39/395, A61K 45/00, A61K 47/02, A61K 47/32, A61K 47/34, A61K 47/36, A61P 35/00

(54) **BIOMOLECULE DELIVERY SYSTEM**

(30) Priority: 09.02.2024 JP 2024018594
(71) Applicant: Institute of Science Tokyo, Tokyo 152-8550 (JP)
(72) Inventor: HONDA Yuto, Tokyo 152-8550 (JP); NISHIYAMA Nobuhiro, Tokyo 152-8550 (JP); KO Rui, Tokyo 152-8550 (JP); HARAGUCHI Haruna, Tokyo 152-8550 (JP)
(74) Representative: Hoffmann Eitle
(86) International application number: PCT/JP2025/003590
(87) International publication number: WO 2025/169918

(57) **Abstract**

A complex of a biocompatible polymer-modified polyphenol, a metal ion, and a biomolecule; a biocompatible polymer-modified polyphenol; and a kit for delivering a biomolecule into the cytoplasm by formation of a complex with the biomolecule and administration of the complex to a subject, the kit comprising a biocompatible polymer-modified polyphenol and a metal ion.

## Description

### Technical Field

The present invention relates to a biomolecule delivery system. More specifically, the present invention relates to a complex, a biocompatible polymer-modified polyphenol, and a kit. The present application claims priority based on Japanese Patent Application No. 2024-018594, filed in Japan on February 9, 2024, the contents of which are incorporated herein by reference.

### Background Art

Biopharmaceuticals containing biomolecules such as proteins as active ingredients have attracted great anticipation as breakthrough therapeutic agents for intractable diseases including cancer. For example, intracellular antibodies that have gained attention in recent years are anticipated to serve as novel antibody medicaments because they can bind to intracellular antigens and induce cell death and cell signaling.

However, biomolecules exhibit low blood stability and low blood retention, resulting in pharmacological activities that fall short of expectations. In addition, the activity of biopharmaceuticals is limited by low cellular uptake and low translocation into the cytoplasm.

In contrast, the blood stability and blood retention of biomolecules can be improved by chemically modifying the biomolecules with a biocompatible polymer such as polyethylene glycol (PEG). However, with this method, the pharmacological activity of the biomolecule may decrease due to chemical modification with PEG. In addition, PEGylation cannot improve cellular uptake of the biomolecule or translocation thereof into the cytoplasm.

In contrast, studies have been conducted on the use of cationic molecules such as cationic peptides, polymers such as polyethyleneimine, and liposomes for cellular uptake of biomolecules and further translocation into the cytoplasm (see, for example, Non Patent Literature 1 to 3).

### Citation List

### Non Patent Literature

Non Patent Literature 1: Chatin B., et al., Liposome-based Formulation for Intracellular Delivery of Functional Proteins, Mol Ther Nucleic Acids, 4, e244, 2015.
Non Patent Literature 2: Boussif O., et al., A versatile vector for gene and oligonucleotide transfer into cells in culture and in vivo: polyethylenimine, Proc. Natl. Acad. Sci. USA, 92, 7297-7301, 1995.
Non Patent Literature 3: Akishiba M., et al., Cytosolic antibody delivery by lipid-sensitive endosomolytic peptide, Nature Chemistry, 9, 751-761, 2017.

### Summary of Invention

### Technical Problem

When a biomolecule such as an intracellular antibody and a cationic molecule are administered simultaneously, the biomolecule can be taken up into cells and further translocated into the cytoplasm by disrupting the endosomal membrane. However, cationic molecules may be highly toxic *in vivo.* Moreover, cationic molecules tend to be recognized as foreign substances *in vivo.* Therefore, methods using cationic molecules cannot provide sufficient activity of the biomolecule. An object of the present invention is to provide a technique for efficiently delivering a biomolecule into the cytoplasm.

### Solution to Problem

The present invention includes the following aspects.
[1] A complex of a biocompatible polymer-modified polyphenol, a metal ion, and a biomolecule.
[2] The complex according to [1], wherein the polyphenol is at least one selected from the group consisting of tannic acid, gallic acid, and derivatives thereof.
[3] The complex according to [1] or [2], wherein the biocompatible polymer comprises at least one selected from the group consisting of polyethylene glycol, acrylic resins, polyamino acids, polyvinylamine, polyallylamine, polynucleotides, polyacrylamide, polyethers, polyesters, polyurethanes, polysaccharides, and copolymers thereof.
[4] The complex according to any one of [1] to [3], wherein the biocompatible polymer has a weight-average molecular weight of 1,000 to 50,000.
[5] The complex according to any one of [1] to [4], wherein the metal ion is an iron(III) ion, a cobalt(II) ion, or a zirconium(IV) ion.
[6] The complex according to any one of [1] to [5], wherein the biomolecule is an antibody or an antigen-binding fragment thereof, a physiologically active protein, a nucleic acid, a lipid nanoparticle, or inorganic/organic microparticles.
[7] A biocompatible polymer-modified polyphenol.
[8] A kit for delivering a biomolecule into the cytoplasm by formation of a complex with the biomolecule and administration of the complex to a subject, the kit comprising a biocompatible polymer-modified polyphenol and a metal ion.

### Advantageous Effects of Invention

According to the present invention, a technique for efficiently delivering a biomolecule into the cytoplasm can be provided.

### Brief Description of Drawings

[Figure 1] Figure 1 is a ¹H NMR spectrum of PEG-P[Lys(TFA)]₂₅ measured in Experimental Example 1.
[Figure 2] Figure 2 is a ¹H NMR spectrum of PEG-P[Lys(TFA)]₁₀₅ measured in Experimental Example 1.
[Figure 3] Figure 3 is a GPC curve of PEG-P[Lys(TFA)]₂₅ measured in Experimental Example 1.
[Figure 4] Figure 4 is a ¹H NMR spectrum of PEG-P[Lys]₂₅ measured in Experimental Example 1.
[Figure 5] Figure 5 is a ¹H NMR spectrum of PEG-P[Lys]₁₀₅ measured in Experimental Example 1.
[Figure 6] Figure 6 is a ¹H NMR spectrum of PEG-P[Lys₁₉/(Lys-GA)₆] measured in Experimental Example 1.
[Figure 7] Figure 7 is a ¹H NMR spectrum of PEG-P[PEG-P[Lys₇₂/(Lys-GA)₃₃] measured in Experimental Example 1.
[Figure 8] Figure 8 is a GPC curve of PEG-P[Lys₁₉/(Lys-GA)₆] measured in Experimental Example 1.
[Figure 9] Figure 9 is a GPC curve of PEG-P[PEG-P[Lys₇₂/(Lys-GA)₃₃] measured in Experimental Example 1.
[Figure 10] Figure 10 is a ¹H NMR spectrum of PEG₁₀ₖ-TA measured in Experimental Example 2.
[Figure 11] Figure 11 is a GPC curve of PEG₁₀ₖ-TA measured in Experimental Example 2.
[Figure 12] Figure 12 is a ¹H NMR spectrum of PEG₅ₖ-TA measured in Experimental Example 2.
[Figure 13] Figure 13 is a GPC curve of PEG₅ₖ-TA measured in Experimental Example 2.
[Figure 14] Figure 14 is a UV-vis spectrum of a GA/Fe³⁺ complex measured in Experimental Example 3.
[Figure 15] Figure 15 is a UV-vis spectrum of a GA/Fe³⁺ complex at pH 7.4 and pH 4.0, measured in Experimental Example 3.
[Figure 16] Figure 16 is a graph showing the results of particle size measurement of a β-Gal/PEG-P[Lys₁₁/(Lys-GA)₁₄]/Fe³⁺ MPN complex by fluorescence correlation spectroscopy, measured in Experimental Example 4.
[Figure 17] Figure 17 is a graph showing the results of particle size measurement of an antibody/PEG-P[Lys₁₁/(Lys-GA)₁₄]/Fe³⁺ MPN complex by fluorescence correlation spectroscopy, measured in Experimental Example 4.
[Figure 18] Figure 18 is a graph showing the results of particle size measurement of antibody MPN complexes by fluorescence correlation spectroscopy using PEG-P[Lysₙ/(Lys-GA)ₘ] having various GA introduction ratios in Experimental Example 4.
[Figure 19] Figure 19 is a graph showing the results of particle size measurement of antibody MPN complexes by fluorescence correlation spectroscopy using PEG-P[Lysₙ/(Lys-GA)ₘ] having different degrees of Lys polymerization in Experimental Example 4.
[Figure 20] Figure 20 is a transmission electron microscopy (TEM) image of an antibody in Experimental Example 4.
[Figure 21] Figure 21 is a TEM image of an antibody/PEG-P[Lys₁₁/(Lys-GA)₁₄] in Experimental Example 4.
[Figure 22] Figure 22 is a TEM image of an antibody/PEG-P[Lys₁₁/(Lys-GA)₁₄]/Fe³⁺ in Experimental Example 4.
[Figure 23] Figure 23 is a TEM image of an antibody/PEG-P[Lys₇₂/(Lys-GA)₃₃]/Fe³⁺ MPN complex in Experimental Example 4.
[Figure 24] Figure 24 is a graph showing the results of particle size measurement of an antibody/PEG-TA/Fe³⁺ MPN complex by fluorescence correlation spectroscopy in Experimental Example 4.
[Figure 25] Figure 25 is a size histogram of an antibody/PEG-TA/Fe³⁺ MPN complex by dynamic light scattering in Experimental Example 4.
[Figure 26] Figure 26 is a graph showing the zeta potential of an antibody/PEG-TA/Fe³⁺ MPN complex in Experimental Example 4.
[Figure 27] Figure 27 is a graph showing the results of particle size measurement of an antibody/PEG-TA/Fe³⁺ MPN complex at various pHs in Experimental Example 4.
[Figure 28] Figure 28 is a graph showing the results of particle size measurement of an antibody/PEG-TA/Fe³⁺ MPN complex in a bovine serum albumin (FBS) solution in Experimental Example 4.
[Figure 29] Figure 29 is a graph showing the results of particle size measurement of an RNP/PEG₁₀ₖ-TA/Zr⁴⁺ MPN complex and an RNP/PEG₁₀ₖ-TA/Co²⁺ MPN complex by fluorescence correlation spectroscopy in Experimental Example 5.
[Figure 30] Figure 30 is a graph showing the results of particle size measurement of an RNP/PEG₅ₖ-TA/Zr⁴⁺ MPN complex and an RNP/PEG₅ₖ-TA/Co²⁺ MPN complex by fluorescence correlation spectroscopy in Experimental Example 5.
[Figure 31] Figure 31 is a graph showing the results of particle size measurement of an RNP/PEG₁₀ₖ-TA/Fe³⁺ MPN complex by fluorescence correlation spectroscopy in Experimental Example 5.
[Figure 32] Figure 32 is a graph showing the results of particle size measurement of an RNP/PEG₅ₖ-TA/Fe³⁺ MPN complex by fluorescence correlation spectroscopy in Experimental Example 5.
[Figure 33] Figure 33 is a TEM image of RNP in Experimental Example 5.
[Figure 34] Figure 34 is a TEM image of RNP/PEG₁₀ₖ-TA in Experimental Example 5.
[Figure 35] Figure 35 is a TEM image of RNP/PEG₁₀ₖ-TA/Fe³⁺ in Experimental Example 5.
[Figure 36] Figure 36 is a graph showing the results of cytotoxicity evaluation of PEG-NH₂, PEG₁₀ₖ-TA, PEG₁₀ₖ-TA/Fe³⁺, and Fe³⁺ in Experimental Example 6.
[Figure 37] Figure 37 is a confocal microscopic image showing intracellular distribution of an antibody/PEG-P[Lys₁₁/(Lys-GA)₁₄]/Fe³⁺ in Experimental Example 7.
[Figure 38] Figure 38 is a graph showing the colocalization efficiency of an antibody and lysosomes calculated from confocal microscopic images in Experimental Example 7.
[Figure 39] Figure 39 is a confocal microscopic image showing intracellular distribution of an anti-NPC antibody/PEG-P[Lys₁₁/(Lys-GA)₁₄]/Fe³⁺ in Experimental Example 7.
[Figure 40] Figure 40 is a graph showing the results of measuring the amount of cellular uptake of an antibody sample containing an antibody/PEG-TA/Fe complex in Experimental Example 7.
[Figure 41] Figure 41 is a confocal microscopic image showing intracellular distribution of an antibody/PEG-TA/Fe³⁺ after incubation for 24 hours in Experimental Example 7.
[Figure 42] Figure 42 is a confocal microscopic image showing intracellular distribution of an antibody/PEG-TA/Fe³⁺ after incubation for 48 hours in Experimental Example 7.
[Figure 43] Figure 43 is a graph showing the colocalization efficiency of an antibody and lysosomes after 24-hour incubation and after 48-hour incubation, calculated from confocal microscopic images in Experimental Example 7.
[Figure 44] Figure 44 is a confocal microscopic image showing intracellular distribution of an anti-NPC antibody/PEG-TA/Fe³⁺ in Experimental Example 7.
[Figure 45] Figure 45 is a graph showing the results of cytotoxicity evaluation of a Cas9-sgRNA RNP/PEG₁₀ₖ-TA/Fe³⁺ MPN complex in Experimental Example 8.
[Figure 46] Figure 46 is a graph showing the results of evaluation of gene knockout efficiency of a Cas9-sgRNA RNP/PEG₁₀ₖ-TA/Fe³⁺ MPN complex in Experimental Example 8.
[Figure 47] Figure 47 is a graph showing the results of comparing organ accumulation of an antibody, an antibody/PEG-TA, an antibody/PEG-TA/Fe³⁺, and an antibody/TA/Fe³⁺ in a 4T1-Luc subcutaneous tumor mouse model in Experimental Example 9.
[Figure 48] Figure 48 is a graph showing the results of comparing blood retention of an antibody, an antibody/PEG-TA, an antibody/PEG-TA/Fe³⁺, and an antibody/TA/Fe³⁺ in a 4T1-Luc subcutaneous tumor mouse model in Experimental Example 9.
[Figure 49] Figure 49 is an IVIS image of a 4T1 subcutaneous tumor mouse model administered with an anti-NPC antibody, an anti-NPC antibody/PEG-TA, an anti-NPC antibody/PEG-TA/Fe³⁺, or an anti-NPC antibody/TA/Fe³⁺ in Experimental Example 9.
[Figure 50] Figure 50 is a fluorescence microscopy image showing the observed distribution of an anti-NPC antibody, an anti-NPC antibody/PEG-TA, an anti-NPC antibody/PEG-TA/Fe³⁺, and an anti-NPC antibody/TA/Fe³⁺ in 4T1 subcutaneous tumor sections in Experimental Example 9.
[Figure 51] Figure 51 is a graph showing the results of quantifying antibody-derived fluorescence intensity in cell nuclei in the 4T1 subcutaneous tumor of Figure 50.
[Figure 52] Figure 52 is a graph showing the results of measuring the antitumor effects of anti-S100A4 antibody/PEG-TA/Fe³⁺ and comparative samples in a 4T1-Luc subcutaneous tumor mouse model in Experimental Example 9.
[Figure 53] Figure 53 is a graph showing the results of measuring changes in body weight when an anti-S100A4 antibody/PEG-TA/Fe³⁺ or a comparative sample was administered to a 4T1-Luc subcutaneous tumor mouse model in Experimental Example 9.
[Figure 54] Figure 54 is a graph showing the results of measuring Luc gene knockout efficiency when an RNP/PEG-TA/Fe³⁺ was administered to a 4T1-Luc subcutaneous tumor mouse model in Experimental Example 10.
[Figure 55] Figure 55 is a graph showing the results of UV absorption of TA, TA (quinone), PEG₅ₖ-TA (Mₙ = 6,700) and PEG₁₀ₖ-TA (Mₙ = 11,700), measured using a spectrophotometer in Experimental Example 11.
[Figure 56] Figure 56 is a graph showing spectra of TA, PEG-NH₂ (Mn: 10k), PEG₁₀ₖ-TA, and PEG-NH₂ (Mn: 10k) + TA, measured using FT-IR in Experimental Example 11.
[Figure 57] Figure 57 is a graph showing spectra of TA, PEG-NH₂ (Mn: 5k), PEG₅ₖ-TA, and PEG-NH₂ (Mn: 5k) + TA, measured using FT-IR in Experimental Example 11.
[Figure 58] Figure 58 is a ¹H NMR spectrum of GalNac-PEG₁₀ₖ-NH₂ measured in Experimental Example 12.
[Figure 59] Figure 59 is a ¹H NMR spectrum of GalNac-PEG₁₀ₖ-TA measured in Experimental Example 12.
[Figure 60] Figure 60 is a ¹H NMR spectrum of PEG₁₀ₖ-TET measured in Experimental Example 13.
[Figure 61] Figure 61 is an SEC spectrum of PEG₁₀ₖ-COOH, PEG₁₀ₖ-TA, PEG₁₀ₖ-TET-TA, and PEG₁₀ₖ-TEP-TA measured in Experimental Example 13.
[Figure 62] Figure 62 is a ¹H NMR spectrum of PEG₁₀ₖ-TEP measured in Experimental Example 13.
[Figure 63] Figure 63 is a ¹H NMR spectrum of PEG₁₀ₖ-S measured in Experimental Example 13.
[Figure 64] Figure 64 is an SEC spectrum of PEG₁₀ₖ-S-TA measured in Experimental Example 13.
[Figure 65] Figure 65 is an absorption spectrum of a mixture of a polyphenol molecule and iron ions, measured in Experimental Example 14.
[Figure 66] Figure 66 is a graph showing the results of particle size measurement of a β-Gal/PEG₁₀ₖ-TA/Fe³⁺ MPN complex and a β-Gal/GalNac-PEG₁₀ₖ-TA/Fe³⁺ MPN complex measured by fluorescence correlation spectroscopy in Experimental Example 15.
[Figure 67] Figure 67 is a graph showing the results of particle size measurement of LNP, LNP/PEG₅ₖ-TA, and LNP/PEG₅ₖ-TA/Fe³⁺ measured using a Zetasizer Ultra (red) in Experimental Example 16.
[Figure 68] Figure 68 is a graph showing the results of particle size measurement of each LNP MPN complex when the mixing amounts of PEG₅ₖ-TA and FeCl₃·6H₂O were varied, measured using a Zetasizer Ultra (red) in Experimental Example 16.
[Figure 69] Figure 69 is a graph showing the results of particle size measurement of PS particles, PS/PEG₅ₖ-TA/Fe³⁺, and PS/PEG₁₀ₖ-TA/Fe³⁺ measured using a Zetasizer Ultra (red) in Experimental Example 16.
[Figure 70] Figure 70 is a graph showing the results of particle size measurement of TUG1, a TUG1/PEG₁₀ₖ-TA complex, and a TUG1/PEG₁₀ₖ-TA/Zr⁴⁺ complex measured by fluorescence correlation spectroscopy in Experimental Example 16.
[Figure 71] Figure 71 is a graph showing the results of particle size measurement of TUG1, a TUG1/PEG-P[Lys₁₁/(Lys-GA)₁₄] complex, and a TUG1/PEG-P[Lys₁₁/(Lys-GA)₁₄]/Zr⁴⁺ complex measured by fluorescence correlation spectroscopy in Experimental Example 16.
[Figure 72] Figure 72 is a graph showing the results of particle size measurement of RNP-loaded MPN complexes prepared using PEG-TET-TA and PEG-TEP-TA, measured by fluorescence correlation spectroscopy in Experimental Example 17.
[Figure 73] Figure 73 is a graph showing the results of particle size measurement of an RNP-loaded MPN complex prepared using PEG-S-TA, measured by fluorescence correlation spectroscopy in Experimental Example 17.
[Figure 74] Figure 74 shows TEM images of antibody MPN complex samples in Experimental Example 18.
[Figure 75] Figure 75 shows the results of measuring the buffering effect of antibody MPN complexes in Experimental Example 18.
[Figure 76] Figure 76 shows the results of measuring blood stability of antibody-loaded MPN complexes in Experimental Example 18.
[Figure 77] Figure 77 shows the results of evaluation of hepatotoxicity of anti-S100A4 antibody-loaded MPN complexes in Experimental Example 18.
[Figure 78] Figure 78 is a graph showing changes in tumor size over time in orthotopically transplanted breast cancer model mice administered with anti-S100A4 antibody-loaded MPN complexes in Experimental Example 18.
[Figure 79] Figure 79 is a graph showing changes in body weight over time in orthotopically transplanted breast cancer model mice administered with anti-S100A4 antibody-loaded MPN complexes in Experimental Example 18.
[Figure 80] Figure 80 shows the results of immunostaining of the tumor suppressor gene (p53) in thin tumor sections from subcutaneously transplanted breast cancer model mice administered with anti-S100A4 antibody-loaded MPN complexes, acquired in Experimental Example 18.
[Figure 81] Figure 81 shows TUNEL assay images of thin tumor sections from subcutaneously transplanted breast cancer model mice administered with anti-S100A4 antibody-loaded MPN complexes, acquired in Experimental Example 18.
[Figure 82] Figure 82 is a graph showing the results of measuring blood retention of a β-Gal-loaded MPN complex in Experimental Example 19.
[Figure 83] Figure 83 is a graph showing the results of measuring the cellular uptake ratio of β-Gal-loaded MPN complexes in the presence and absence of an inhibitor in Experimental Example 20.
[Figure 84] Figure 84 is a graph showing the results of measuring organ accumulation 6 hours after administration of RNP-loaded MPN complexes to tumor-bearing model mice in Experimental Example 21.
[Figure 85] Figure 85 is a graph showing the results of measuring tumor accumulation 6 hours after administration of RNP-loaded MPN complexes to tumor-bearing model mice in Experimental Example 21.
[Figure 86] Figure 86 is a graph showing the results of measuring blood retention after administration of an RNP-loaded MPN complex to tumor-bearing model mice in Experimental Example 21.
[Figure 87] Figure 87 is a graph showing the results of measuring Luc luminescence intensity after administration of RNP-loaded MPN complexes to HeLa-Luc cells in Experimental Example 22.
[Figure 88] Figure 88 is a graph showing the results of measuring genome editing efficiency in the liver of Ai9 tdTomato mice administered with RNP-loaded MPN complexes in Experimental Example 23.
[Figure 89] Figure 89 is a graph showing the results of measuring genome editing efficiency in muscle of Ai9 tdTomato mice administered with RNP-loaded MPN complexes in Experimental Example 23.
[Figure 90] Figure 90 is a graph showing the results of measuring genome editing efficiency in the brain of Ai9 tdTomato mice administered with RNP-loaded MPN complexes in Experimental Example 23.

### Description of Embodiments

### [Complex]

In one embodiment, the present invention provides a complex of a biocompatible polymer-modified polyphenol, a metal ion, and a biomolecule. As described later in the Examples, by administering the complex of the present embodiment, a biomolecule can be taken up into cells, allowed to escape from endosomes, and delivered into the cytoplasm. Accordingly, it can also be said that the complex of the present embodiment is a delivery system for delivering the biomolecule into the cytoplasm.

The complex of the present embodiment may be added to a medium *in vitro* or may be administered to a living body *in vivo.* Examples of the living body include humans and non-human animals. The non-human animal is not particularly limited and examples thereof include rodents such as mice, rats, and hamsters; non-human primates such as monkeys and chimpanzees; and mammals such as rabbits, sheep, cattle, and pigs. Examples of the route of administration to a living body include intravenous injection.

In the complex of the present embodiment, the biomolecule preferably exerts its physiological activity by being delivered into the cytoplasm, and is preferably usable as a biopharmaceutical. The biomolecule is not particularly limited as long as it has physiological activity, and examples thereof include antibodies or antigen-binding fragments thereof, physiologically active proteins, nucleic acids, lipid nanoparticles, and inorganic/organic microparticles.

The antibody may be a human-type antibody. Since human-type antibodies exhibit low immunogenicity even when administered to humans, side effects such as anaphylactic shock can be suppressed. Examples of the human-type antibody include chimeric antibodies, humanized antibodies, and fully human antibodies.

A chimeric antibody means an antibody in which the variable region is derived from a non-human animal and at least a part of the constant region is derived from a human. A humanized antibody means an antibody in which only the complementarity-determining regions (CDRs) of the heavy chain and light chain are derived from a non-human animal and the constant regions and framework regions are derived from a human. A fully human antibody means an antibody that is entirely derived from a human, including the complementarity-determining regions. Examples of the antigen-binding fragment of the antibody include F(ab')₂, Fab', Fab, Fv, and scFv.

The physiologically active protein is not particularly limited as long as it has physiological activity, and also includes enzymes and complexes of a CRISPR/Cas protein and gRNA (ribonucleoprotein; RNP).

The nucleic acid is not particularly limited as long as it has physiological activity, and examples thereof include DNA and RNA. Examples of the RNA include mRNA and shRNA.

A lipid nanoparticle (LNP) is a particle having a diameter of about 10 nm to 1,000 nm and containing lipids as main components. Lipid nanoparticles are used as non-viral drug delivery systems (DDSs) for delivery of nucleic acid drugs and the like. The lipid nanoparticle may encapsulate a drug. As described later in the Examples, the complex of the present embodiment can be formed by mixing a biocompatible polymer-modified polyphenol, a metal ion, and a lipid nanoparticle.

The inorganic/organic microparticles mean inorganic microparticles, organic microparticles, and composite microparticles thereof. The inorganic/organic microparticles preferably have a diameter of about 10 nm to 1,000 nm. Examples of the inorganic/organic microparticles include polystyrene particles, silica particles, and gold nanoparticles. As described later in the Examples, the complex of the present embodiment can be formed by mixing a biocompatible polymer-modified polyphenol, a metal ion, and inorganic/organic microparticles.

The biocompatible polymer-modified polyphenol is a compound in which a biocompatible polymer and a polyphenol are bonded to each other. In the complex of the present embodiment, examples of the polyphenol include tannic acid, gallic acid, and derivatives thereof. Examples of derivatives of tannic acid or gallic acid include compounds in which one or more hydrogen atoms or hydroxyl groups in tannic acid or gallic acid are substituted with substituents. Here, examples of the substituent include a hydroxyl group, an amino group, a monovalent linear saturated hydrocarbon group having 1 to 10 carbon atoms, and a halogen atom. Examples of the monovalent linear saturated hydrocarbon group having 1 to 10 carbon atoms include a methyl group, an ethyl group, a propyl group, and a butyl group. Examples of the halogen atom include a fluorine atom and a chlorine atom.

The biocompatible polymer means a polymer that does not cause, or is less likely to cause, significant adverse effects or harmful effects such as strong inflammatory response or injury when administered to a living body. In the complex of the present embodiment, the biocompatible polymer includes polyethylene glycol, acrylic resins, polyamino acids, polyvinylamine, polyallylamine, polynucleotides, polyacrylamide, polyethers, polyesters, polyurethanes, polysaccharides, and copolymers thereof. The biocompatible polymer may partially have any group introduced during the synthesis process thereof. Examples of such a group include a part of a polymerization initiator.

As described later in the Examples, a molecule having functionality may be introduced into the biocompatible polymer. Examples of the molecule having functionality include N-acetylgalactosamine (GalNAc), which is a hepatic parenchymal cell-targeting molecule, and cationic molecules that improve cellular uptake. Examples of the cationic molecule include ethylenediamine-based molecules, and more specifically include triethylenetriamine (TET), tetraethylenepentamine (TEP), and spermine (S).

The biocompatible polymer may have a weight-average molecular weight of 1,000 to 50,000. The upper limit of the weight-average molecular weight of the biocompatible polymer may be 30,000, 20,000, or 10,000. The lower limit of the weight-average molecular weight of the biocompatible polymer may be 2,000, 3,000, 4,000, or 5,000. These upper limits and lower limits may be combined as appropriate.

Here, as the weight-average molecular weight of the biocompatible polymer, a value measured by size exclusion chromatography (SEC) analysis can be used. Specifically, after dissolving the biocompatible polymer in a solvent, the biocompatible polymer is passed together with a mobile phase solution through a column using a packing material having a large number of pores, separated in the column according to molecular weight, and detected using a differential refractometer, an ultraviolet-visible spectrophotometer, a viscometer, a light scattering detector, or the like as a detector. Dedicated SEC instruments are widely commercially available, and measurement is generally performed in terms of standard polyethylene glycol. In the present specification, the weight-average molecular weight is measured in terms of the standard polyethylene glycol.

The dispersity (Mw/Mn) of the biocompatible polymer is preferably 1.0 or more and less than 2.0, more preferably 1.0 to 1.5, and still more preferably 1.0 to 1.3. When the dispersity of the biocompatible polymer is within the above range, the complex of the present embodiment can exhibit high tumor accumulation.

The tumor accumulation is considered to be exerted by selective accumulation in a tumor utilizing enhanced vascular permeability of the tumor, that is, by an enhanced permeability and retention effect (EPR effect), and the complex of the present embodiment can thereby be selectively delivered to the tumor.

When a polyphenol and a biomolecule are mixed, a complex is spontaneously formed. This complex is considered to be formed by hydrophobic interactions and/or hydrogen bonds, and the complex can be formed without chemically modifying the biomolecule.

When a biocompatible polymer-modified polyphenol and a biomolecule are mixed, a complex is spontaneously formed. This results in a form in which the biocompatible polymer appears to be attached to the biomolecule via the polyphenol.

As described later in the Examples, the complex of the present embodiment is spontaneously formed by mixing a biocompatible polymer-modified polyphenol, a metal ion, and a biomolecule in a buffer solution. The biocompatible polymer-modified polyphenol, the metal ion, and the biomolecule may be mixed simultaneously, or the three may be mixed in any order. In either case, the complex of the present embodiment is spontaneously formed. When the three are mixed sequentially, they are preferably mixed in the order of the biomolecule, the biocompatible polymer-modified polyphenol, and the metal ion. The buffer solution is not particularly limited, and phosphate buffered saline (PBS) can be used, for example.

The biocompatible polymer-modified polyphenol is preferably mixed such that the molar ratio of biomolecule:biocompatible polymer-modified polyphenol is 1:20 to 1:5,000, more preferably 1:25 to 1:3,000. The metal ion is preferably mixed such that the molar ratio of polyphenol in the biocompatible polymer-modified polyphenol:metal ion is 5:1 to 1:5, more preferably 3:1 to 1:3.3.

In the complex of the present embodiment, the metal ion is not particularly limited as long as it can form a metal ion-polyphenol complex (MPN) having buffering ability by crosslinking the polyphenol described above, and specific examples thereof include an iron(III) ion, a cobalt(II) ion, and a zirconium(IV) ion. The metal ion can be used by dissolving a metal salt that generates the above metal ion upon ionization in a solvent such as a buffer solution.

As described later in the Examples, metal ions such as iron(III) ions, cobalt(II) ions, and zirconium(IV) ions form pH-responsive complexes with polyphenols and exhibit buffering ability.

The complex of the present embodiment is taken up into cells while enclosed in vesicles called endosomes. A proton pump called V-ATPase is present in the endosomal membrane and transports protons into the endosome until the pH in the endosome reaches about 5 to 6. However, since the complex of the present embodiment has buffering ability, a decrease in pH in the endosome is suppressed, and a larger influx of protons into the endosome is required to decrease the pH. In addition, anions also flow into the endosome in order to maintain charge balance inside and outside the endosome, thereby increasing the salt concentration and osmotic pressure in the endosome. In order to relieve this high osmotic pressure, a large amount of water further flows into the endosome, which can no longer withstand the volume, resulting in rupture of the endosomal membrane. As a result, the complex of the present embodiment is considered to be capable of escaping from endosomes (lysosomes). Such an effect is called a proton sponge effect. That is, the complex of the present embodiment exhibits a proton sponge effect.

### [Compound]

In one embodiment, the present invention provides a biocompatible polymer-modified polyphenol. The biocompatible polymer-modified polyphenol of the present embodiment is a novel compound. The biocompatible polymer and the polyphenol are the same as those described above.

More specific examples of the compound of the present embodiment include tannic acid-modified polyethylene glycol (PEG-TA) represented by Formula (1) below and PEG-Poly[(L-Lysine)ₙ/(L-Lysine-Gallic Acid)ₘ] represented by Formula (2) below.

In Formulae (1) and (2), p represents the number of polyethylene glycol units. In Formula (2), n represents the number of lysine residues, and m represents the number of gallic acid-modified lysine residues. p may be about 45 to 4,500. In addition, n may be about 10 to 200. In addition, m may be about 5 to 100. As described later in the Examples, by using the compounds represented by Formulae (1) and (2), a biomolecule can be taken up into cells, allowed to escape from endosomes, and delivered into the cytoplasm.

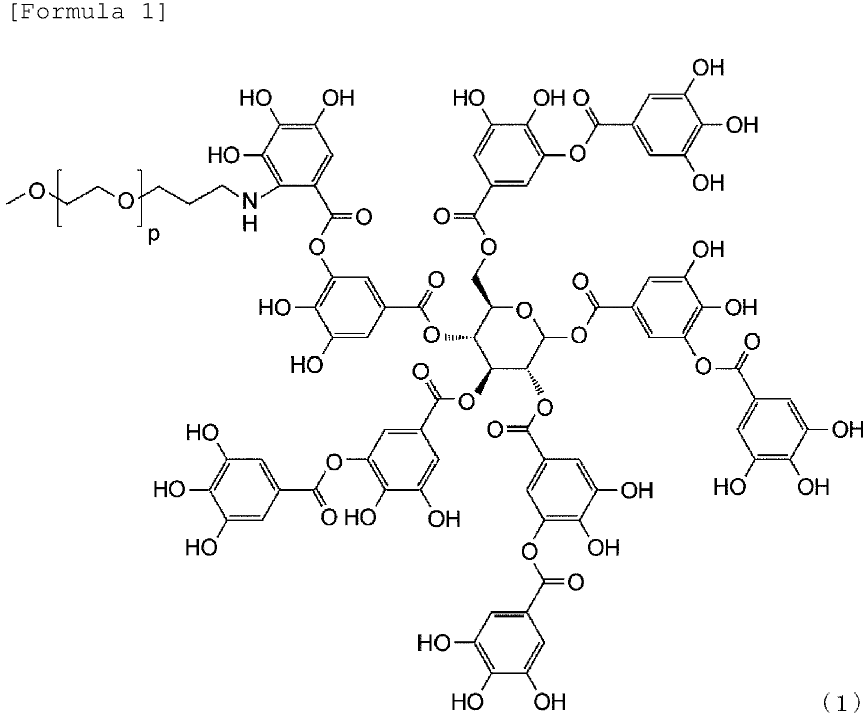

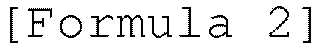

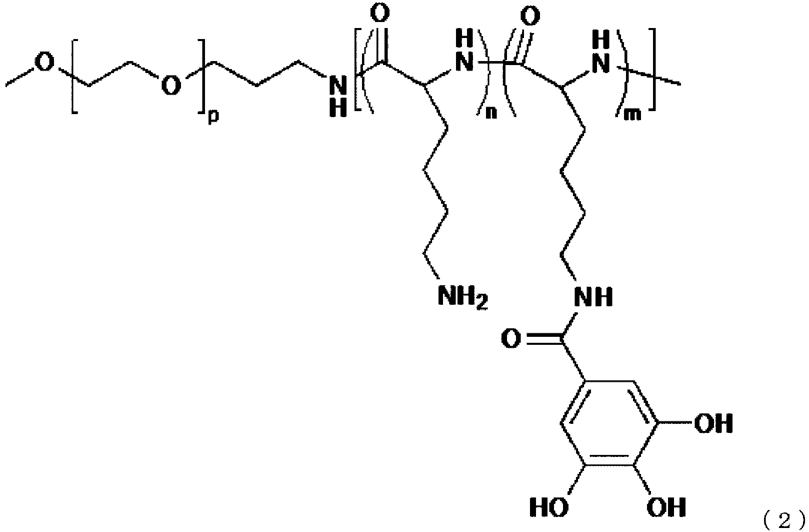

As described later in the Examples, a part of the galloyl group structure of tannic acid-modified polyethylene glycol may be in a quinone structure. Formula (3) below shows the chemical formula of tannic acid-modified polyethylene glycol in which a part of the galloyl group structure is in a quinone structure. In Formula (3) below, n represents the number of polyethylene glycol units.

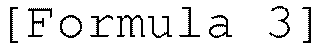

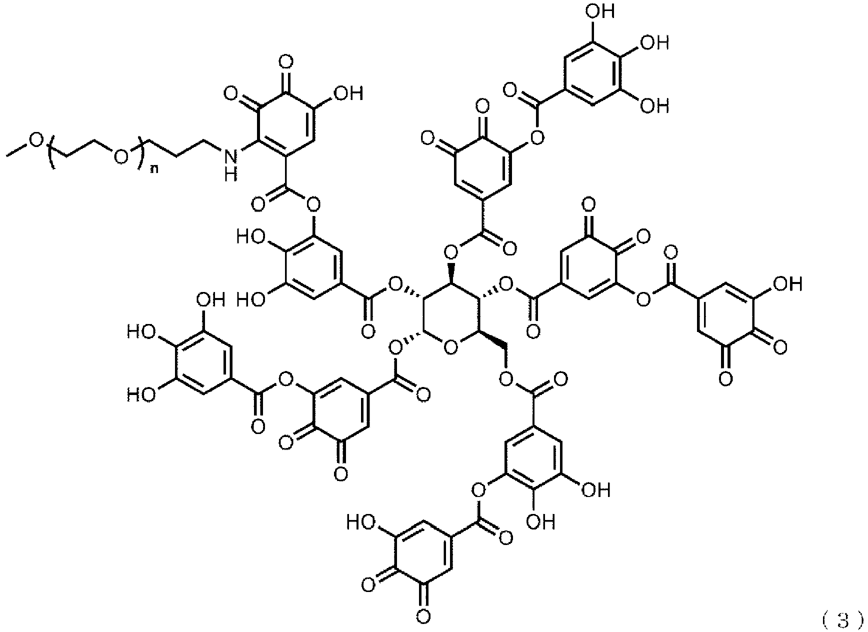

As described later in the Examples, a molecule having functionality may be introduced into the compound of the present embodiment. Examples of the molecule having functionality include N-acetylgalactosamine (GalNAc), which is a hepatic parenchymal cell-targeting molecule, and cationic molecules that improve cellular uptake. Examples of the cationic molecule include ethylenediamine-based molecules, and more specifically include triethylenetriamine (TET), tetraethylenepentamine (TEP), and spermine (S).

### [Kit]

In one embodiment, the present invention provides a kit for delivering a biomolecule into the cytoplasm by formation of a complex with the biomolecule and administration of the complex to a subject, the kit comprising a biocompatible polymer-modified polyphenol and a metal ion.

As described later in the Examples, the kit of the present embodiment can efficiently deliver a biomolecule into the cytoplasm of a target cell.

In the kit of the present embodiment, the biocompatible polymer, the polyphenol, the metal ion, the biomolecule, and the like are the same as those described above.

As described above, when a biocompatible polymer-modified polyphenol, a metal ion, and a biomolecule are mixed in a buffer solution, a complex is spontaneously formed. By administering this complex to a subject, the biomolecule can be efficiently delivered into the cytoplasm of a target cell.

The complex may be added *in vitro* to a medium of a target cell, or may be administered *in vivo* to a target living body. The living body is the same as that described above. Examples of the route of administration to a living body include intravenous injection.

### [Other Embodiments]

In one embodiment, the present invention provides a method for delivering a biomolecule into the cytoplasm, the method comprising a step of administering to a subject a complex of a biocompatible polymer-modified polyphenol, a metal ion, and the biomolecule. As described later in the Examples, the method of the present embodiment can efficiently deliver a biomolecule into the cytoplasm of a target cell.

In the method of the present embodiment, the biocompatible polymer, the polyphenol, the metal ion, the biomolecule, and the like are the same as those described above.

As described above, when a biocompatible polymer-modified polyphenol, a metal ion, and a biomolecule are mixed in a buffer solution, a complex is spontaneously formed. By administering this complex to a subject, the biomolecule can be efficiently delivered into the cytoplasm of a target cell.

The complex may be added *in vitro* to a medium of a target cell, or may be administered *in vivo* to a target living body. The living body is the same as that described above. Examples of the route of administration to a living body include intravenous injection.

### Examples

Next, the present invention will be described in more detail with reference to Examples, but the present invention is not limited to the following Examples.

### [Experimental Example 1]

### <Synthesis of PEG₁₀ₖ-Poly[L-Lysineₙ/(L-Lysine-Gallic Acid)ₘ]>

### (1.1 Overview)

Schemes (1) and (2) below show a method for synthesizing PEG₁₀ₖ-Poly[(L-Lysine)ₙ/(L-Lysine-Gallic Acid)ₘ] (hereinafter referred to as "PEG-P[(Lys)ₙ/(Lys-GA)ₘ]", where n+m represents the degree of polymerization of Lys, and m represents the number of introduced gallic acid (GA)).

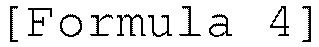

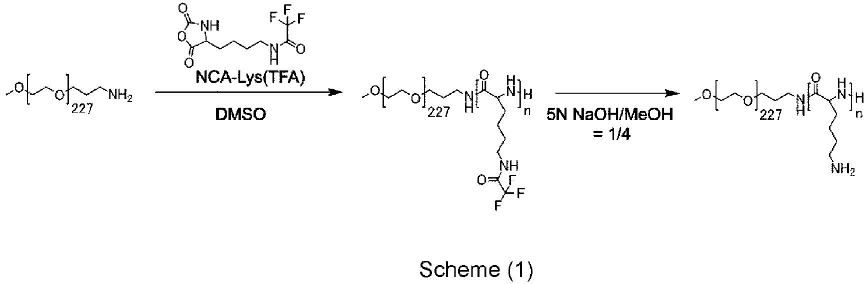

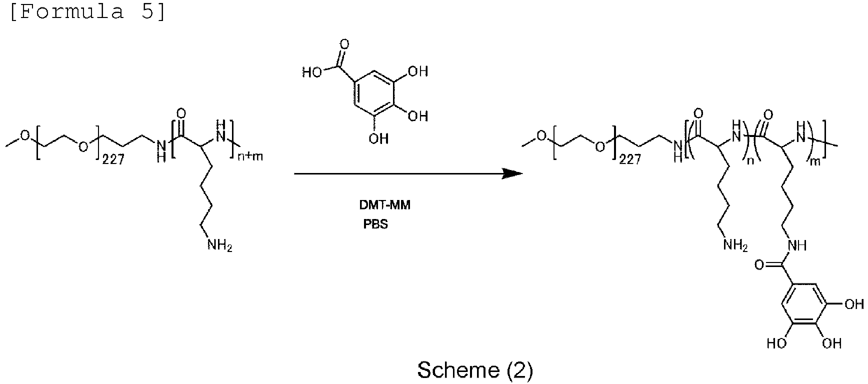

As shown in Scheme (1), PEG-P[Lys(TFA)]ₙ₊ₘ was synthesized by N-carboxyanhydride (NCA) polymerization using PEG₁₀ₖ-NH₂ as an initiator and Lys(TFA)-NCA as a monomer. The TFA groups of the side chains were deprotected under basic conditions to obtain PEG-PLysₙ₊ₘ.

Subsequently, as shown in Scheme (2), the carboxyl group of gallic acid was bonded to an amino group of PEG-PLysₙ₊ₘ to obtain PEG-P[Lysₙ/(Lys-GA)ₘ].

### (1.2 Reagents)

The reagents used are shown below. Unless otherwise specified, commercially available reagents and solvents were used as purchased.
· α-Methoxy-ω-amino-poly(ethylene glycol) (PEG-NH₂) [Mn: 10k] (NOF Corporation)
· Benzene (Nacalai Tesque)
· N-₂-Trifluoroacetyl-L-lysine-N-carboxyanhydride (Lys(TFA)-NCA) (Chuo Kaseihin)
· Dimethyl sulfoxide (DMSO) (FUJIFILM Wako Pure Chemical Corporation), distilled under an argon atmosphere before use (b.p. 189°C)
· Diethyl ether (Kanto Chemical)
· Methanol (Kanto Chemical)
· 5 mol/L NaOH (FUJIFILM Wako Pure Chemical Corporation)
· Dimethyl sulfoxide (DMSO) (Nacalai Tesque)
· D-PBS(-) (FUJIFILM Wako Pure Chemical Corporation)
· 4-(4,6-Dimethoxy-1,3,5-triazin-2-yl)-4-methyl-morpholinium chloride n-hydrate (DMT-MM) (FUJIFILM Wako Pure Chemical Corporation)
· Gallic acid (GA) (Tokyo Chemical Industry)
· L(+)-Ascorbic acid sodium salt (sodium L(+)-ascorbate) (FUJIFILM Wako Pure Chemical Corporation)
· D-Sorbitol (Tokyo Chemical Industry)
· 4-(2-Hydroxyethyl)-1-piperazineethanesulfonic acid (HEPES) (Dojindo Laboratories)
· Sodium chloride (NaCl) (FUJIFILM Wako Pure Chemical Corporation)
· Cy5-NHS (Lumiprobe)
· 1-Methyl-2-pyrrolidinone (Sigma-Aldrich)
· Lithium bromide (Sigma-Aldrich)

### (1.3 Measurement Instruments)

· Nuclear magnetic resonance (NMR) instrument (BRUKER AVANCE III 400 (400 MHz, BRUKER BioSpin))
· Gel permeation chromatography (GPC) instrument (JASCO International)
   Columns: TSK-gel SuperAW3000 + SuperAW4000 (Tosoh) and Superdex 200 Increase 10/300 GL (GE Healthcare)
   Detectors: RI-2031, UV-2030

### (1.4 Synthesis Method)

### <Synthesis of PEG-P[Lys(TFA)]ₙ₊ₘ>

500 mg (0.050 mmol) of PEG-NH₂ was weighed into a 300 mL two-neck round-bottom flask, dissolved in 2.0 mL of benzene, and then freeze-dried. Under an argon atmosphere, 348 mg (1.3 mmol, 26 equivalents) (n+m = 25) and 1,600 mg (6.0 mmol, 120 equivalents) (n+m = 105) of Lys(TFA)-NCA were weighed into a 100 mL two-neck round-bottom flask. 5 mL of DMSO was added to PEG-NH₂. In addition, 10 mL of DMSO was added to each Lys(TFA)-NCA to dissolve them. The Lys(TFA)-NCA solution was added to the PEG-NH₂ solution, followed by stirring at room temperature for 72 hours under an argon atmosphere. Each reaction solution was added dropwise to 300 mL of diethyl ether and purified by reprecipitation. Thereafter, the product was dried under reduced pressure to obtain white solid PEG-PLys(TFA) in amounts of 745 mg (n+m = 25) and 1,750 mg (n+m = 105), with yields of 91% (n+m = 25) and 86% (n+m = 105).

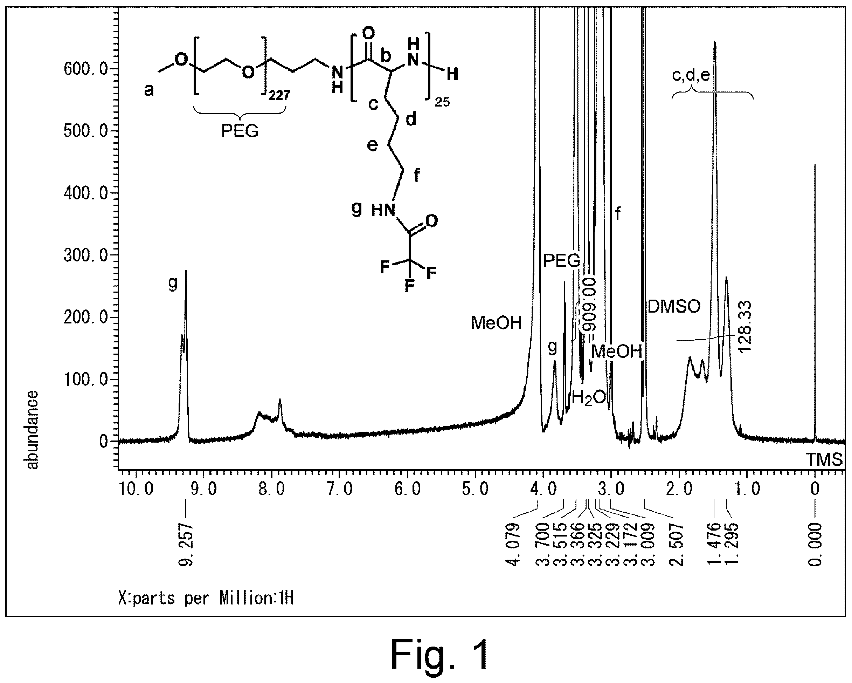

Figure 1 shows a ¹H-NMR spectrum of PEG-P[Lys(TFA)]₂₅ (solvent: DMSO-d⁶). The results of ¹H-NMR analysis of PEG-P[Lys(TFA)]₂₅ are also shown below.

¹H-NMR spectrum of PEG-P[Lys(TFA)]₂₅

[Formula 6] ¹H-NMR (DMSO-d⁶, 25°C): δ 3. 4-3. 9 (909H, -CH₂CH ₂O-), δ 1. 2 5-1. 99 (120H, -CH₂CH₂CH₂CH₂NH₃), δ 2 . 97 (40H, -CH₂CH₂NH₃), δ 4. 30 (20H, -COCHNH-).

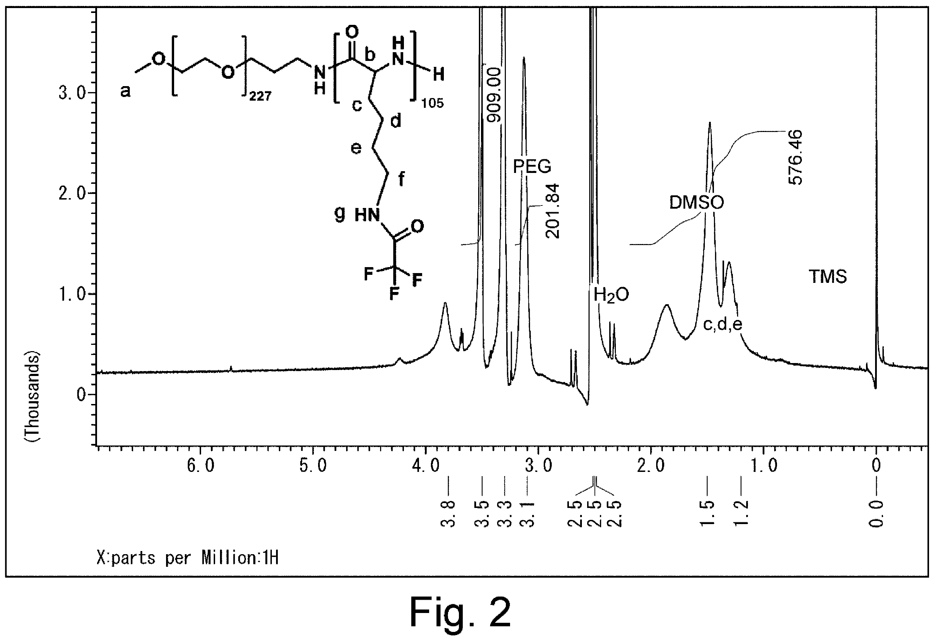

Figure 2 shows a ¹H-NMR spectrum of PEG-P[Lys(TFA)]₁₀₅ (solvent: DMSO-d⁶). The results of ¹H-NMR analysis of PEG-P[Lys(TFA)]₁₀₅ are also shown below.

¹H-NMR spectrum of PEG-P[Lys(TFA)]₁₀₅

¹H-NMR (DMSO-d⁶, 25°C): Assignments are the same as those for the above ¹H-NMR spectrum of PEG-P[Lys(TFA)]₂₅.

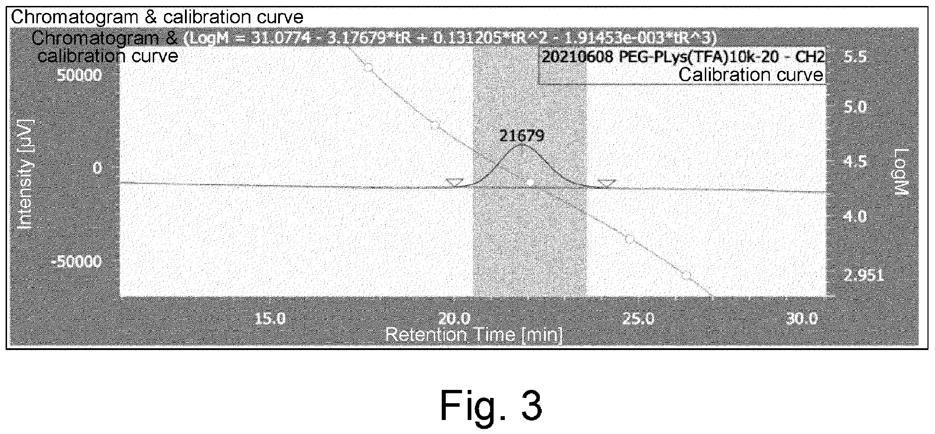

Figure 3 shows a GPC curve of PEG-P[Lys(TFA)]₁₀₅. The GPC curve was obtained using TSK-gel superAW3000 and superAW4000 as columns, NMP (50 mM LiBr) as an eluent, RI-2031 as a detector, a flow rate of 0.30 mL/min, and a measurement temperature of 40°C. Table 1 below also shows the Mw/Mn of both polymers, PEG-P[Lys(TFA)]₂₅ and PEG-PLys(TFA)₁₀₅.

**[Table 1]**

| **Polymer** | **Mw/Mn** |
|---|---|
| PEG-P[Lys(TFA)]₂₅ | 1.07 |
| PEG-P[Lys(TFA)]₁₀₅ | 1.10 |

### <Deprotection of PEG-P[Lys(TFA)]ₙ₊ₘ>

500 mg of PEG-P[Lys(TFA)]₂₅ and 500 mg of PEG-P[Lys(TFA)]₁₀₅ were respectively weighed into 50 mL round-bottom flasks, added to a mixed solution of 2 mL of 5 M NaOH and 8 mL of methanol, and stirred overnight at room temperature. The reaction solution was placed in a dialysis membrane (MWCO: 3.5 kDa) and dialyzed twice each against 2 L of 0.1 M HCl and subsequently 2 L of pure water. The solution was freeze-dried to obtain white solid PEG-P[Lys]ₙ₊ₘ in amounts of 367 mg (n+m = 25) and 331 mg (n+m = 105), with yields of 92% (n+m = 25) and 90% (n+m = 25), respectively.

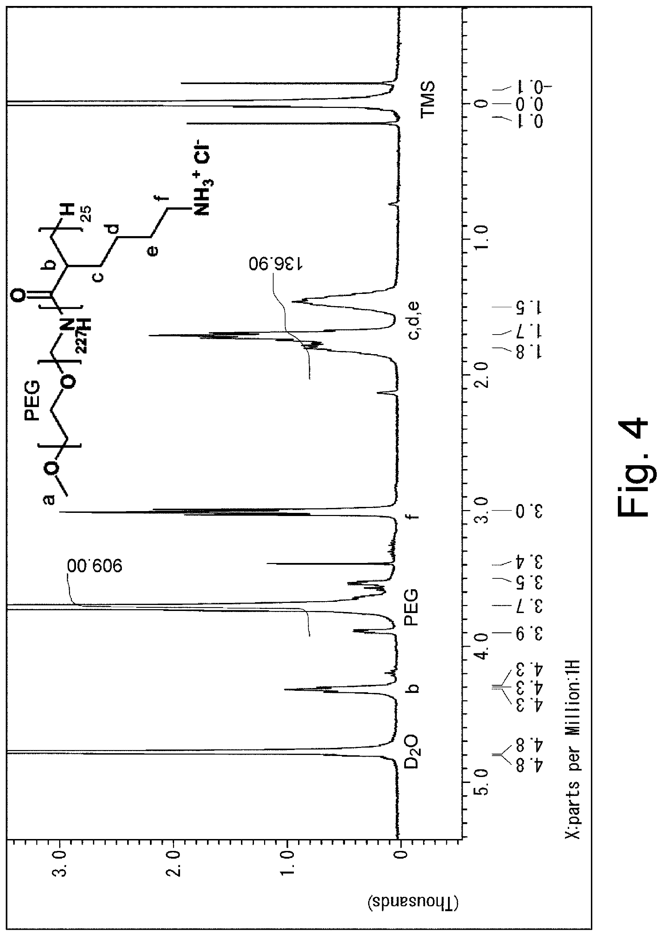

Figure 4 shows a ¹H-NMR spectrum of PEG-P[Lys]₂₅ (solvent: D₂O). The results of ¹H-NMR analysis of PEG-P[Lys]₂₅ are also shown below.

¹H-NMR spectrum of PEG-P[Lys]₂₅

[Formula 7] ¹H-NMR (D₂O, 25°C): δ 3. 4-3.9 (909H, -CH₂CH₂O-) , δ 1. 25-1.99 (120H, -CH₂CH₂CH₂CH₂NH₃), δ 2. 9 7 ( 4 OH, -CH₂CH₂NH₃), δ 4. 30 (20H, -COCHNH-).

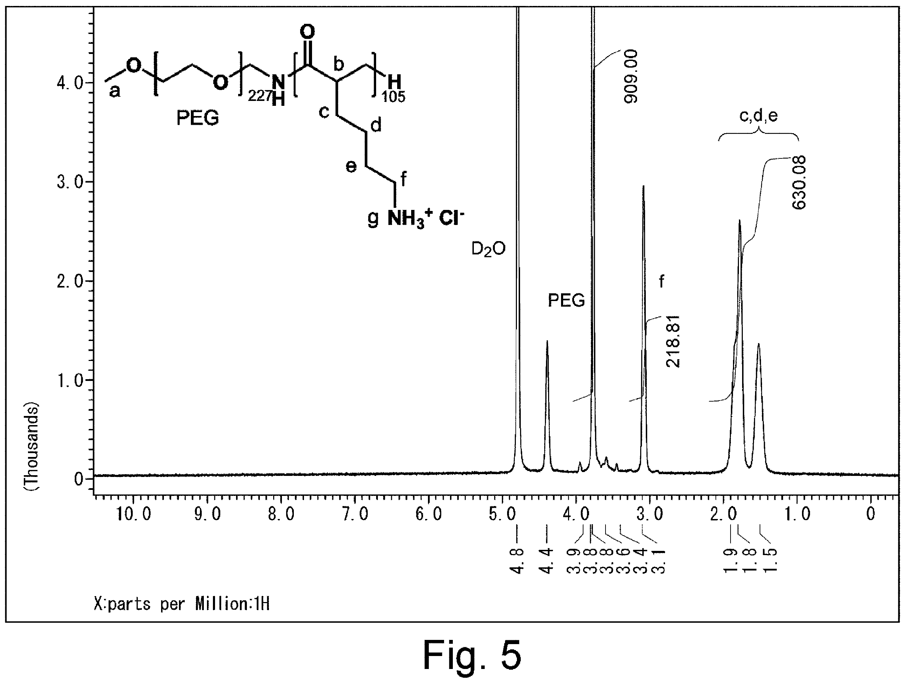

Figure 5 shows a ¹H-NMR spectrum of PEG-P[Lys]₁₀₅ (solvent: D₂O). The results of ¹H-NMR analysis of PEG-P[Lys]₁₀₅ are also shown below.

¹H-NMR spectrum of PEG-P[Lys]₁₀₅

¹H-NMR (D₂O, 25°C): Assignments are the same as those for the above ¹H-NMR spectrum of PEG-PLys₂₅.

### <Binding of GA to PEG-P[Lys]ₙ₊ₘ>

PEG-PLysₙ₊ₘ, gallic acid (GA), and sodium L(+)-ascorbate were weighed into a vial. The amount of GA added was set to 25%, 50%, or 75% of the molar amount of Lys in PEG-P[Lys]ₙ₊ₘ, with the molar ratio of Lys in PEG-P[Lys]ₙ₊ₘ taken as 100%. In addition, sodium L(+)-ascorbate was used in an amount of 30 chemical equivalents relative to PEG-P[Lys]ₙ₊ₘ. DMT-MM was weighed into a separate screw tube in an amount of 1.2 equivalents relative to GA. Next, D-PBS(-) was added to dissolve the respective weighed reagents. While stirring the polymer solution in an ice bath, the DMT-MM solution was added all at once, followed by stirring at 4°C for 6 hours to carry out a galloyl group introduction reaction of the polymer. After the reaction, diluted hydrochloric acid was added to lower the pH of the galloyl group-introduced polymer solution to 4. Next, ultrafiltration (MWCO: 3 kDa) was performed 3 to 5 times using ultrapure water at 4°C to remove impurities. The purified polymer solution was transferred to a centrifuge tube, and the solvent was removed by freeze-drying to obtain light brown solid PEG-P[Lysₙ/(Lys-GA)ₘ]. The yields thereof were calculated to be 60 to 80%.

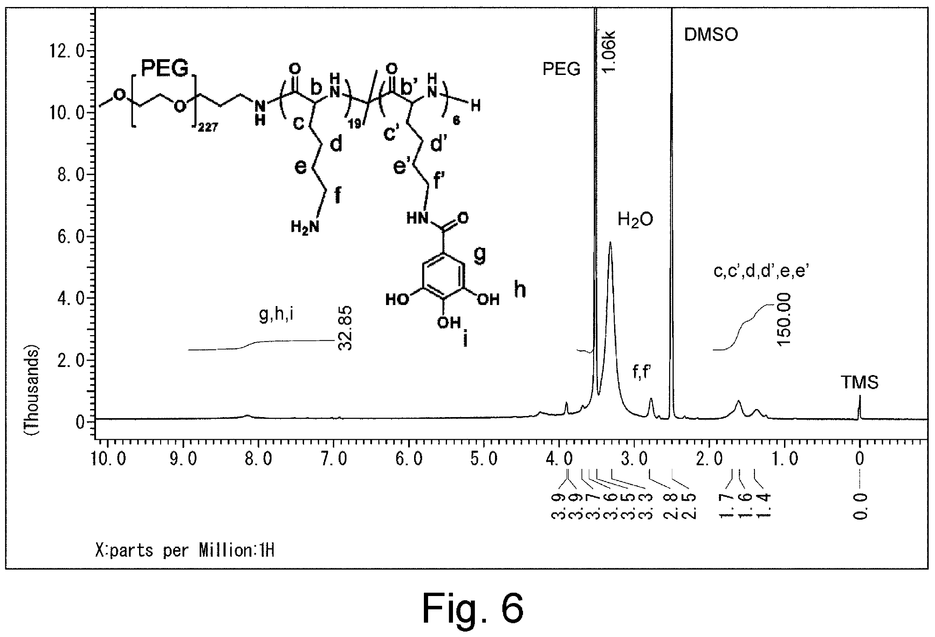

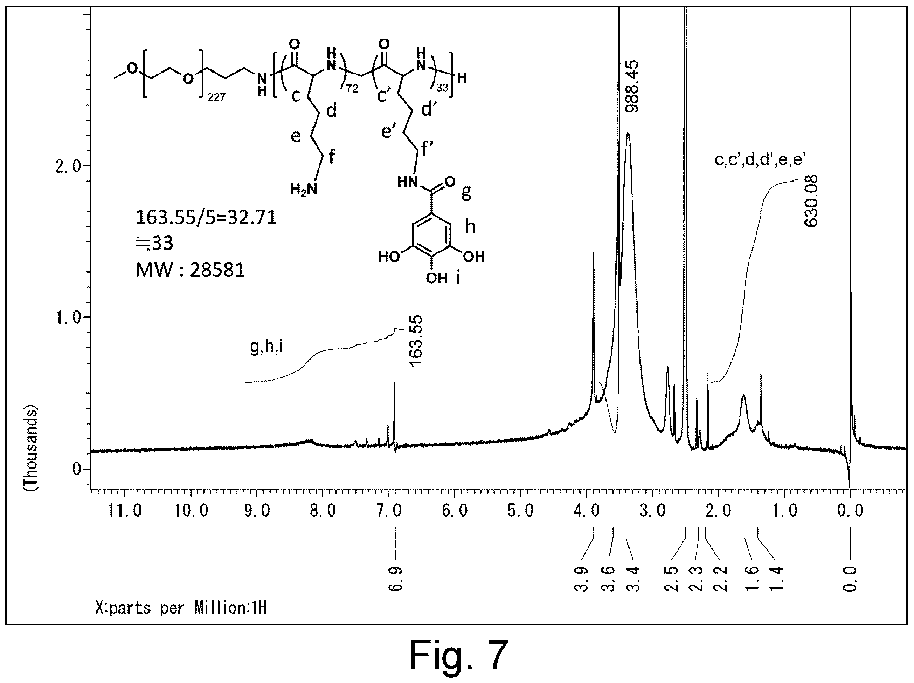

Figures 6 and 7 show ¹H-NMR spectra of PEG-P[Lysₙ/(Lys-GA)ₘ] (solvent: DMSO-d⁶). The results of ¹H-NMR analysis of PEG-P[Lysₙ/(Lys-GA)ₘ] are also shown below. From the ¹H-NMR spectra, the GA introduction ratio relative to the Lys side chains of PEG-P[Lys]ₙ₊ₘ was calculated.

¹H-NMR spectrum of PEG-P[PEG-P[Lys₁₉/(Lys-GA)₆]

[Formula 8] ¹H-NMR (DMSO-d⁶, 25°C): δ 0. 87-2. 22(120H, -CH₂ CH₂CH₂CH₂NH₃), δ 6. 90-9. 00(32H, -C₆H₃(OH)₃).

¹H-NMR spectrum of PEG-P[PEG-P[Lys₇₂/(Lys-GA)₃₃]

¹H-NMR (DMSO-d⁶, 25°C): Assignments are the same as those for the above ¹H-NMR spectrum of PEG-P[PEG-P[Lys₁₉/(Lys-GA)₆].

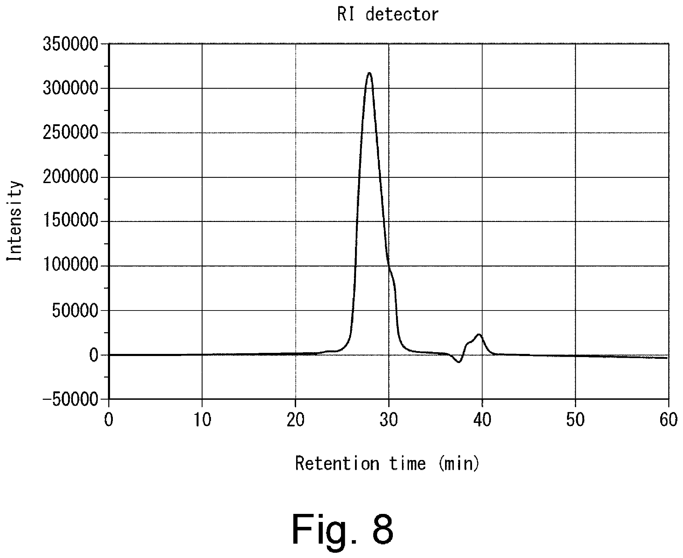

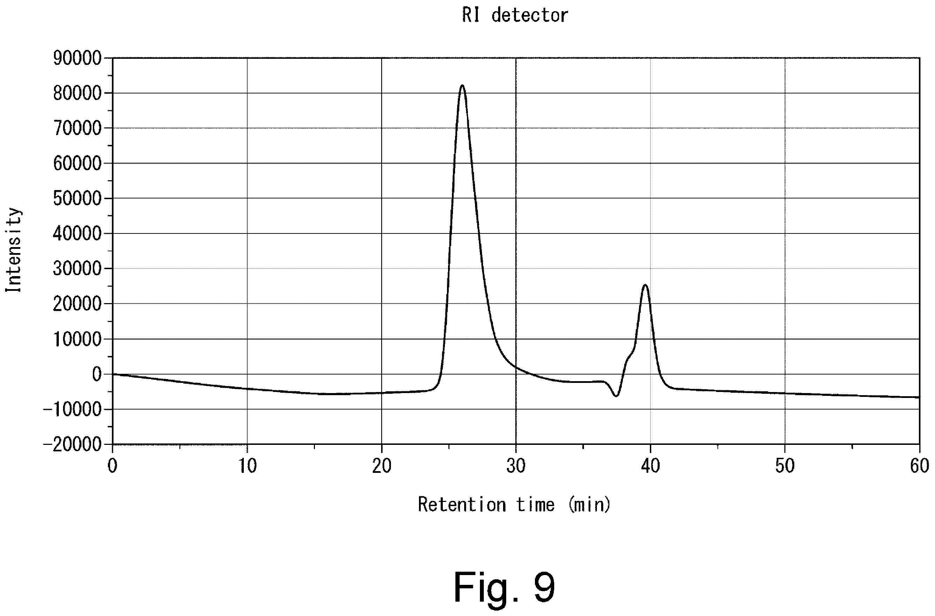

Figure 8 shows a GPC curve of PEG-P[PEG-P[Lys₁₉/(Lys-GA)₆]. Figure 9 shows a GPC curve of PEG-P[PEG-P[Lys₇₂/(Lys-GA)₃₃]. The GPC curve was obtained using Superdex 200 Increase 10/300 GL as a column, 10 mM HEPES and 140 mM NaCl (pH 7.4) as an eluent, UV-2030 as a detector, detection at a wavelength of 220 nm, a flow rate of 0.75 mL/min, and room temperature.

### (1.5 Analysis)

### <PEG-P[Lys(TFA)]ₙ₊ₘ>

From the GPC curve, the M_{w}/Mₙ of PEG-P[Lys(TFA)]₂₅ was calculated to be 1.07. In addition, the M_{w}/Mₙ of PEG-P[Lys(TFA)]₁₀₅ was calculated to be 1.10. From these results, it was confirmed that these polymers had a narrow molecular weight distribution.

### <PEG-PLysₙ₊ₘ>

From the ratio of the integral values of the initiator-derived peak and the Lys-derived peak in the ¹H-NMR spectra, the degree of polymerization of PLys was calculated to be DP = 25 and DP = 105.

### <PEG-P[PEG-P[Lysₙ/(Lys-GA)ₘ]]>

From the ratio of the integral values of the PLys-derived peak and the GA-derived peak in the ¹H-NMR spectra, the number of introduced GA was calculated to be 6 (n = 25) and 33 (n = 105), and the number-average molecular weight was calculated to be Mn = 16,300 (n = 25) and Mn = 30,940 (n = 105). In addition, from the GPC curve, it was confirmed that the obtained polymer had a unimodal narrow molecular weight distribution. In addition, with respect to PEG-P[PEG-P[Lysₙ/(Lys-GA)ₘ]] having a Lys degree of polymerization of 25, synthesis of PEG-P[PEG-P[Lysₙ/(Lys-GA)ₘ]] having GA introduction ratios of 52% (number of introduced GA: 14) and 62% (number of introduced GA: 16) was also achieved.

### [Experimental Example 2]

### <Synthesis of PEG-TA>

### (2.1 Overview)

Schemes (3) and (4) below show a method for synthesizing tannic acid-modified PEG(PEG-TA).

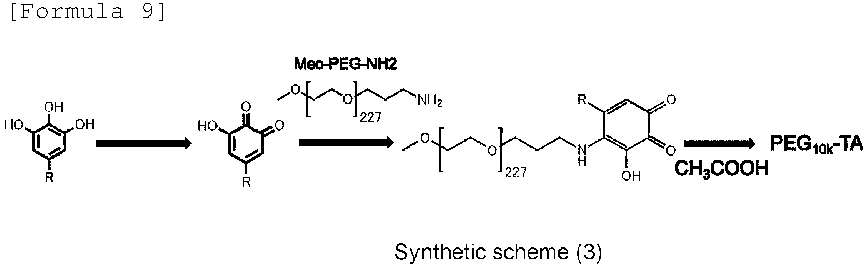

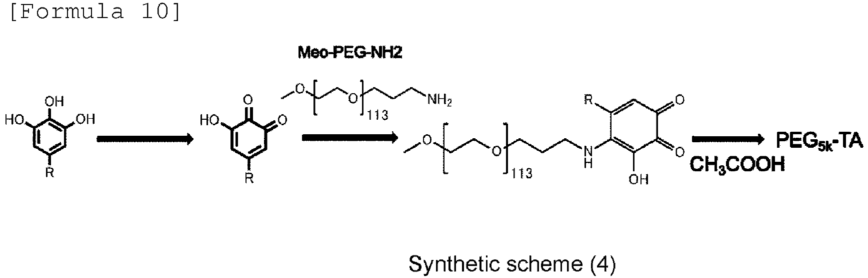

PEG-NH₂ having a molecular weight of 10k and PEG-NH₂ having a molecular weight of 5k were each reacted with tannic acid (TA) by a Michael addition reaction, after which ketone groups of the side chains were reduced under acidic conditions, followed by dialysis to obtain PEG-TA.

### (2.2 Reagents)

The reagents used are shown below.
· α-Methoxy-ω-amino-poly (ethylene glycol) (PEG₁₀ₖ-NH₂) [Mn: 10k] (NOF Corporation)
· α-Methoxy-ω-amino-poly (ethylene glycol) (PEG₅ₖ-NH₂) [Mn:5k] (NOF Corporation)
· Tannic acid (FUJIFILM Wako Pure Chemical Corporation)
· Sodium hydrogen carbonate (NaHCO₃) (FUJIFILM Wako Pure Chemical Corporation)
· Acetic acid (CH₃COOH) (Nacalai Tesque)
· {2-[4-(2-Hydroxyethyl)-1-piperazinyl]ethanesulfonic acid} (HEPES) (Nacalai Tesque)
· Sodium chloride (NaCl) (FUJIFILM Wako Pure Chemical Corporation)

### (2.3 Measurement Instruments)

· Nuclear magnetic resonance (NMR) instrument (BRUKER AVANCE III 400 (400 MHz, BRUKER BioSpin))
· Gel permeation chromatography (GPC) instrument (JASCO International)
   Column: Superdex 75 Increase 10/300 GL (Cytiva)
   Detectors: RI-2031, UV-2030
· NanoDrop One nanospectrophotometer (Thermo Fisher Scientific)

### (2.4 Synthesis Method)

### <Synthesis of PEG₁₀ₖ-TA>

### <Binding of TA to PEG₁₀ₖ-NH₂>

52 mg (0.030 mmol) of TA was weighed into a 200 mL beaker and dissolved in 80 mL of 50 mM NaHCO₃ (pH 8.5). To this was added dropwise 100 mg (0.010 mmol) of PEG₁₀ₖ-NH₂ dissolved in 40 mL of 50 mM NaHCO₃ (pH 8.5), followed by stirring at room temperature for 5 hours. Thereafter, the reaction solution transferred into a dialysis membrane (MWCO: 6 to 8 kDa) was dialyzed for 1 hour against 2 L of pure water subjected to ultrasonication for 10 minutes and Ar bubbling for 5 minutes.

### <Reduction of Side-Chain Ketone Groups of TA>

The reaction solution was transferred to a 200 mL beaker, and 2 mL of acetic acid was added dropwise followed by stirring for 5 minutes. Thereafter, the reaction solution transferred into a dialysis membrane (MWCO: 6 to 8 kDa) was dialyzed twice against 2 L of pure water subjected to ultrasonication for 10 minutes and Ar bubbling for 5 minutes. The resulting solution was freeze-dried to obtain yellow solid PEG₁₀ₖ-TA in a yield of 68 mg.

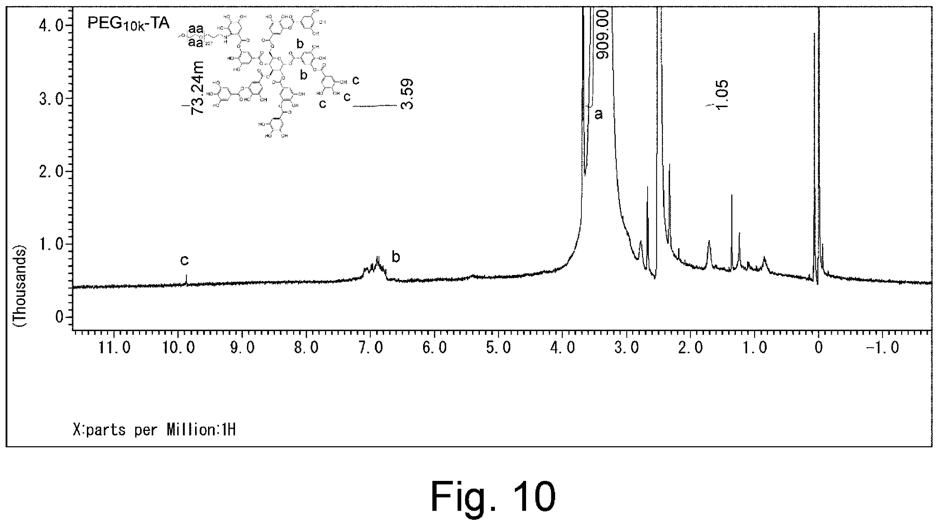

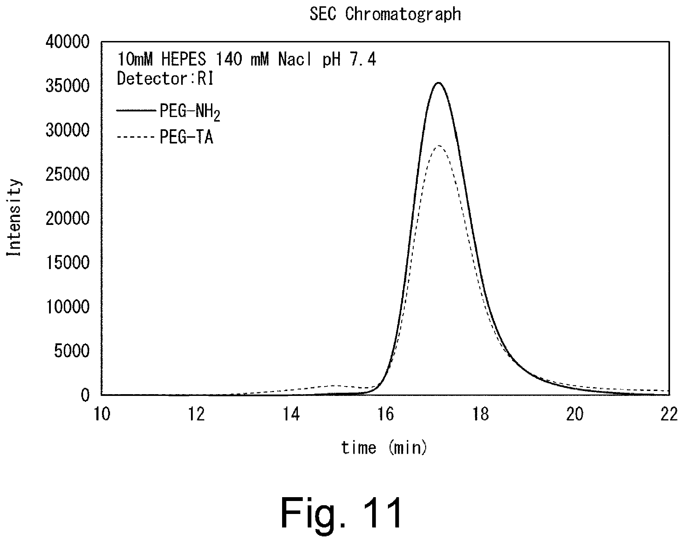

Figure 10 shows a ¹H-NMR spectrum of PEG₁₀ₖ-TA. Figure 11 shows a GPC curve of PEG₁₀ₖ-TA. The GPC curve was obtained using Superdex 75 Increase 10/300 GL as a column, 10 mM HEPES and 140 mM NaCl (pH 7.4) as an eluent, UV-2030 as a detector, a detection wavelength of 254 nm, a flow rate of 0.75 mL/min, and room temperature. The vertical axis in Figure 11 represents signal intensity (relative value).

### <Synthesis of PEG₅ₖ-TA>

### <Binding of TA to PEG₅ₖ-NH₂>

52 mg (0.030 mmol) of TA was weighed into a 200 mL beaker and dissolved in 80 mL of 50 mM NaHCO₃ (pH 8.5). To this was added dropwise 50 mg (0.010 mmol) of PEG₅ₖ-NH₂ dissolved in 40 mL of 50 mM NaHCO₃ (pH 8.5), followed by stirring at room temperature for 5 hours. Thereafter, the reaction solution transferred into a dialysis membrane (MWCO: 6 to 8 kDa) was dialyzed for 1 hour against 2 L of pure water subjected to ultrasonication for 10 minutes and Ar bubbling for 5 minutes.

### <Reduction of Side-Chain Ketone Groups of TA>

The reaction solution was transferred to a 200 mL beaker, and 2 mL of acetic acid was added dropwise followed by stirring for 5 minutes. Thereafter, the reaction solution transferred into a dialysis membrane (MWCO: 6 to 8 kDa) was dialyzed twice against 2 L of pure water subjected to ultrasonication for 10 minutes and Ar bubbling for 5 minutes. The resulting solution was freeze-dried to obtain yellow solid PEG₅ₖ-TA in a yield of 30 mg.

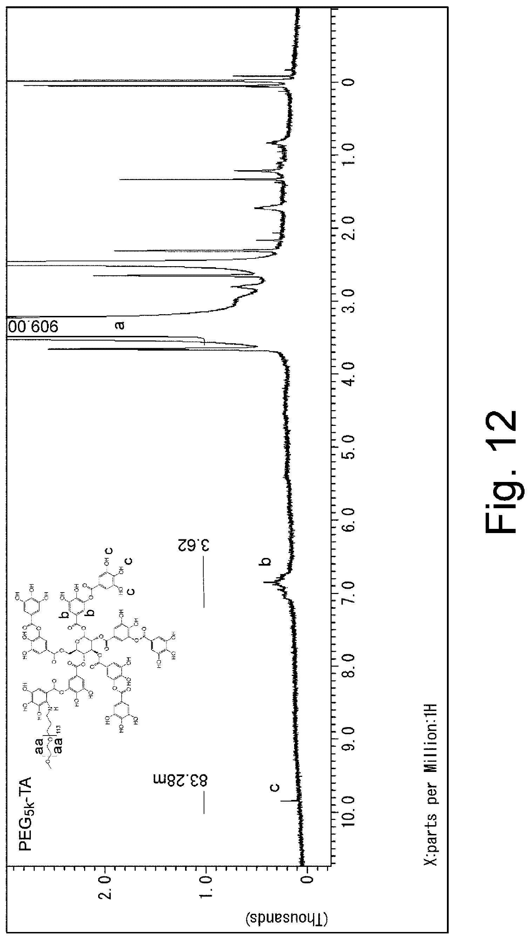

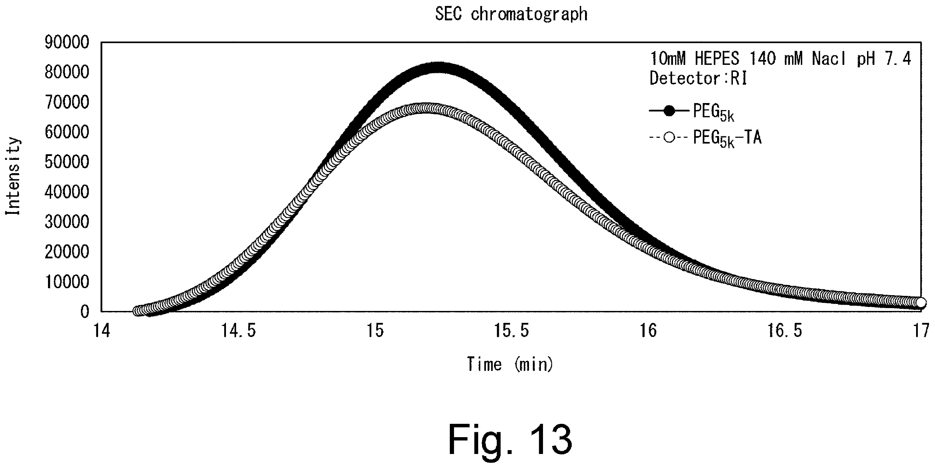

Figure 12 shows a ¹H-NMR spectrum of PEG₅ₖ-TA. Figure 13 shows a GPC curve of PEG₅ₖ-TA. The GPC curve was obtained using Superdex 75 Increase 10/300 GL as a column, 10 mM HEPES and 140 mM NaCl (pH 7.4) as an eluent, UV-2030 as a detector, a detection wavelength of 254 nm, a flow rate of 0.75 mL/min, and room temperature. The vertical axis in Figure 13 represents signal intensity (relative value).

### (2.5 Analysis)

### <PEG₁₀ₖ-TA>

From the GPC curve, it was confirmed that PEG₁₀ₖ-TA had a narrow molecular weight distribution. In the ¹H-NMR spectrum, an initiator-derived peak and a GA group-derived peak were confirmed. In addition, the TA introduction ratio was calculated to be 92% from the absorbance at 277 nm, an absorption wavelength derived from the GA groups.

### <PEG₅ₖ-TA>

From the GPC curve, it was confirmed that PEG₅ₖ-TA had a narrow molecular weight distribution. In the ¹H-NMR spectrum, an initiator-derived peak and a GA group-derived peak were confirmed. In addition, the TA introduction ratio was calculated to be 81% from the absorbance at 277 nm, an absorption wavelength derived from the GA groups. In the following Examples, when referred to as PEG-TA, PEG₁₀ₖ-TA was used in all cases.

### [Experimental Example 3]

### <Confirmation of Complex Formation Between GA Groups and Fe³⁺ and pH Responsiveness>

### (3.1 Overview)

GA groups and metal ions form a metal ion-polyphenol complex (MPN) having buffering ability by crosslinking. Therefore, complex formation between GA groups and iron ions, and the pH responsiveness thereof, were confirmed using a spectrophotometer.

### (3.2 Reagents)

· Gallic acid (GA) (Tokyo Chemical Industry)
· Iron(III) chloride hexahydrate (FeCl₃·6H₂O) (Fe³⁺) (FUJIFILM Wako Pure Chemical Corporation)
· D-PBS(-) (FUJIFILM Wako Pure Chemical Corporation)
· {2-[4-(2-Hydroxyethyl)-1-piperazinyl]ethanesulfonic acid} (HEPES) (Nacalai Tesque)

### (3.3 Measurement Instruments)

· JASCO V-650 spectrophotometer (JASCO)

### (3.4 Confirmation of Complex Formation)

### <Final Concentrations of GA and FeCl₃·6H₂O>

· GA: 3 mM
· FeCl₃·6H₂O (Fe³⁺): 3 mM and 9 mM

These were prepared by dissolution in D-PBS(-).

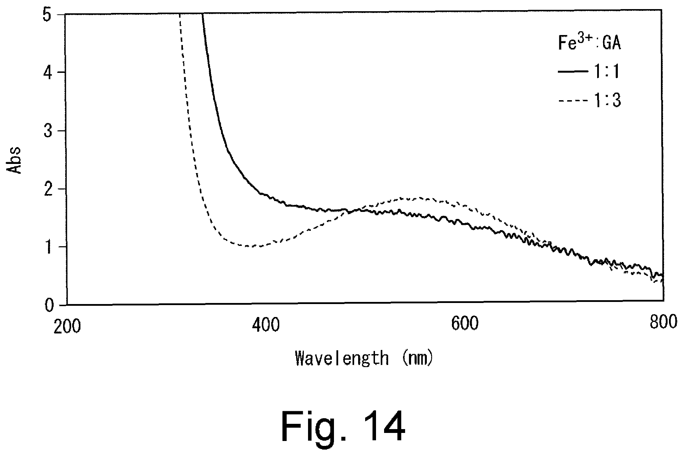

GA and Fe³⁺ were mixed at two mixing ratios ([GA:Fe³⁺] = 1:1 and [GA:Fe³⁺] = 1:3) to prepare solutions. After incubation at room temperature for 2 hours, absorption spectra were measured using a spectrophotometer. Figure 14 is a graph showing the results of measuring the absorption spectra.

From the absorption spectra, an increase in absorbance around 550 nm was observed in the [GA:Fe³⁺] = 1:3 solution, confirming that GA and Fe³⁺ formed a complex at [GA:Fe³⁺] = 1:3.

### (3.5 Confirmation of pH Responsiveness)

### <Final Concentrations of GA and FeCl₃·6H₂O>

· GA: 3 mM
· Fe³⁺: 9 mM

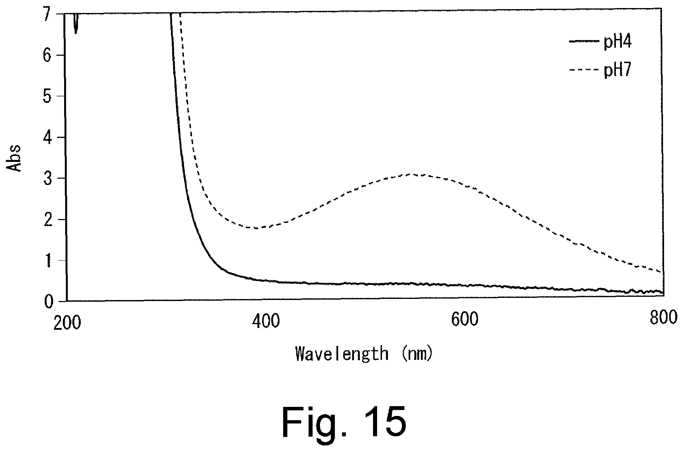

GA and Fe³⁺ were dissolved in D-PBS(-) (pH 7.4) and in D-PBS(-) adjusted to pH 4.0, and after incubation at room temperature for 2 hours, absorption spectra were measured using a spectrophotometer. Figure 15 is a graph showing the results of measuring the absorption spectra.

From the absorption spectra, the absorbance around 550 nm in the pH 4.0 solution was decreased as compared with the pH 7.4 solution, confirming that the complex of GA and Fe³⁺ had pH responsiveness.

### [Experimental Example 4]

### <Evaluation of Physicochemical Properties of Protein Metal-Polyphenol Network (MPN) Complexes>

### (4.1 Overview)

When a protein MPN complex is formed from a protein, a polymer-modified polyphenol, and iron ions, the particle size increases. Accordingly, particle size measurement was performed by fluorescence correlation spectroscopy using β-galactosidase (β-Gal) and an antibody as proteins. PEG-P[Lysₙ/(Lys-GA)ₘ] and PEG-TA were used as the polymer-modified polyphenols. At the same time, complex formation was also confirmed using transmission electron microscopy (TEM). Furthermore, properties of the protein MPN complex, such as pH responsiveness and surface zeta potential, were also evaluated. In addition, in order to evaluate stability in a blood environment, changes in particle size in an FBS solution were also measured.

### (4.2 Reagents)

Unless otherwise specified, commercially available reagents and solvents were used as purchased.
· β-Galactosidase (β-Gal, Mw: 540 kDa) (FUJIFILM Wako Pure Chemical Corporation)
· Normal human IgG (antibody, Mw: 150 kDa) (FUJIFILM Wako Pure Chemical Corporation)
· Cy5-NHS (Lumiprobe)
· PEG-P[Lysₙ/ (Lys-GA)ₘ] (m + n = 25, 105)
· PEG-TA (Mn = 11,700)
· D-PBS(-) (FUJIFILM Wako Pure Chemical Corporation)
· 5 mol/L HCl (FUJIFILM Wako Pure Chemical Corporation)
· 5 mol/L NaOH (FUJIFILM Wako Pure Chemical Corporation)
· Sodium hydrogen carbonate: Tokyo Chemical Industry
· 4-(2-Hydroxyethyl)-1-piperazineethanesulfonic acid (HEPES) (Dojindo Laboratories)
· Sodium chloride (NaCl) (FUJIFILM Wako Pure Chemical Corporation)
· Iron(III) chloride hexahydrate (FeCl₃·6H₂O) (Fe³⁺) (FUJIFILM Wako Pure Chemical Corporation)
· Fetal bovine serum (FBS) (Biosera)

### (4.3 Measurement Instruments)

· LSM710 (Carl Zeiss)
· Fluorescence spectrophotometer FP-8300 (JASCO)
· Zetasizer Nano ZS (Malvern Panalytical)
· NanoDrop One nanospectrophotometer (Thermo Fisher Scientific)
· JEM-1400 (JEOL)

### (4.4 Introduction of Fluorescent Dye into Protein)

20 mg of antibody and 15 mg of β-Gal were weighed into vials and dissolved in 10 mL of 50 mM NaHCO₃ (pH 8.5). To this was added 0.7 mg of Cy5-NHS dissolved in DMSO, followed by stirring at room temperature for 2 hours. The reaction solution was added to an ultrafiltration tube (MWCO: 30 kDa) and centrifuged at 8000 × g for 20 minutes to remove unreacted Cy5-NHS. At that time, PBS(-) was used as an exchange solution. The final concentration was determined by measuring the absorbance at 280 nm, at which protein-derived absorption was observed. Removal of unreacted fluorescent dye was also confirmed by electrophoresis. Thus, Cy5-β-Gal (hereinafter, β-Gal) and Cy5-IgG antibody (hereinafter, antibody) were obtained.

### (4.5 Evaluation of Formation of β-Gal-Loaded MPN Complex Using PEG-P[Lysₙ/(Lys-GA)ₘ])

### <Final Concentrations of β-Gal, PEG-P[Lysₙ/(Lys-GA)ₘ], and FeCl₃·6H₂O>

· β-Gal: 500 nM (stock concentration: 2.0 µM)
• PEG-P[Lys₁₁/(Lys-GA)₁₄]: 50 µM (stock concentration: 200 µM)
• FeCl₃·6H₂O (Fe³⁺): 220 µM (stock concentration: 2.2 mM)

Each was prepared by dissolution in D-PBS(-).

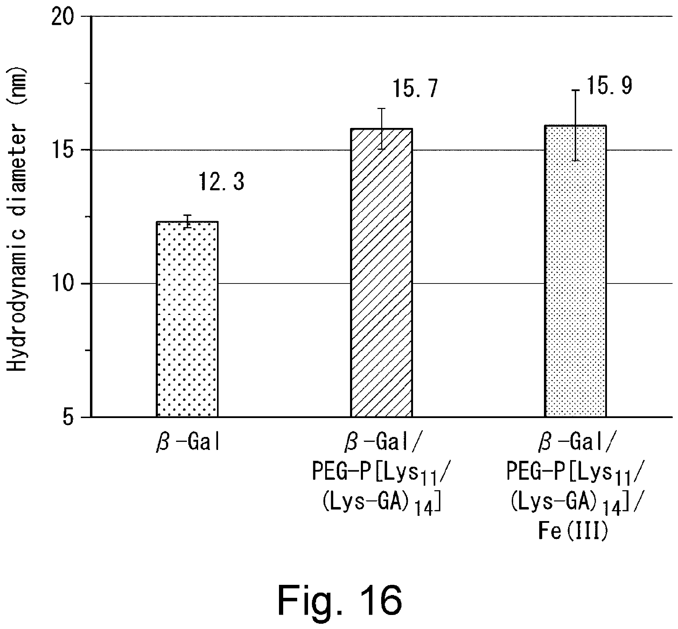

The β-Gal solution, PEG-P[Lys₁₁/(Lys-GA)₁₄] solution, and Fe³⁺ solution were mixed by pipetting. Thereafter, after the concentration was adjusted by adding PBS(-), particle size was measured by fluorescence correlation spectroscopy using LSM710. Figure 16 shows the results of particle size measurement.

Because the particle size of the β-Gal/PEG-P[Lys₁₁/(Lys-GA)₁₄]/Fe³⁺ complex was significantly increased compared with the particle sizes of the β-Gal and β-Gal/PEG-P[Lys₁₁/(Lys-GA)₁₄] solutions, formation of the β-Gal/PEG-P[Lys₁₁/(Lys-GA)₁₄]/Fe³⁺ complex was demonstrated.

### (4.6 Evaluation of Formation of Antibody-Loaded MPN Complex Using PEG-P[Lysₙ/(Lys-GA)ₘ])

### <Final Concentrations of Antibody, PEG-P[Lys/(Lys-GA)ₘ, and Fe³⁺>

- Antibody: 200 nM
- PEG-P[Lys₁₁/(Lys-GA)₁₄]: 20 to 600 µM (stock concentration: 2,000 µM)
- Fe³⁺: 90 to 2,800 µM (stock concentration: 2.2 mM)

Each was prepared by dissolution in D-PBS(-).

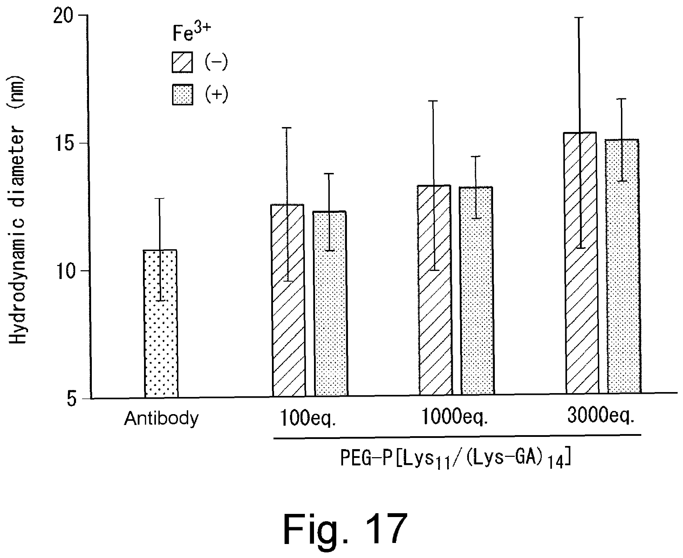

The antibody solution, PEG-P[Lys₁₁/(Lys-GA)₁₄] solution, and Fe³⁺ solution were mixed by pipetting. In doing so, the molar ratio of PEG-P[Lys₁₁/(Lys-GA)₁₄]/antibody was set to 100, 1,000, and 3,000. In addition, the molar ratio of iron ions was set to 1/3 equivalent relative to GA in PEG-P[Lys₁₁/(Lys-GA)₁₄]. Finally, after the concentration was adjusted by adding PBS(-), particle size was measured by fluorescence correlation spectroscopy using LSM710. Figure 17 shows the results of particle size measurement.

Because the particle size of the antibody/PEG-P[Lys₁₁/(Lys-GA)₁₄]/Fe³⁺ complex was significantly increased compared with the particle sizes of the antibody and antibody/PEG-P[Lys₁₁/(Lys-GA)₁₄] solutions, formation of the antibody/PEG-P[Lys₁₁/(Lys-GA)₁₄]/Fe³⁺ complex was suggested. In addition, a tendency for the particle size to increase with increasing amounts of PEG-P[Lys₁₁/(Lys-GA)₁₄] and Fe³⁺ added was observed.

### (4.7 Evaluation of Formation of Antibody-Loaded MPN Complexes When Varying the GA Introduction Ratio on the Polymer)

### <Final Concentrations of Antibody, PEG-P[Lysₙ/(Lys-GA)ₘ, and Fe³⁺>

- Antibody: 200 nM
- PEG-P[Lys₁₉/(Lys-GA)₆]: 20 µM (stock concentration: 2,000 µM)
- PEG-P[Lys₁₁/(Lys-GA)₁₄]: 20 µM (stock concentration: 2,000 µM)
- PEG-P[Lys₉/(Lys-GA)₁₆]: 20 µM (stock concentration: 2,000 µM)
- Fe³⁺: 25 to 110 µM (stock concentration: 2.2 mM)

Each was prepared by dissolution in D-PBS(-).

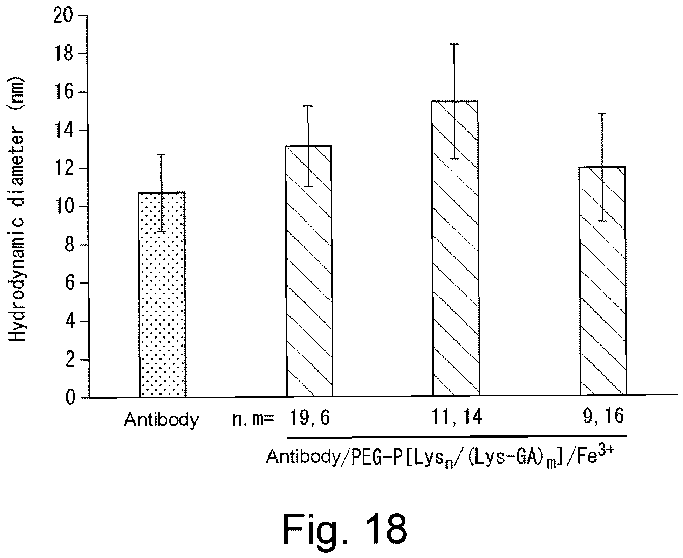

The antibody solution, each PEG-P[Lysₙ/(Lys-GA)ₘ] solution, and Fe³⁺ solution were mixed by pipetting. In doing so, the molar ratio of PEG-P[Lysₙ/(Lys-GA)ₘ]/antibody was set to 100. In addition, the molar ratio of iron ions was set to 1/3 equivalent relative to GA in PEG-P[Lysₙ/(Lys-GA)ₘ]. Finally, after the concentration was adjusted by adding PBS(-), particle size was measured by fluorescence correlation spectroscopy using LSM710. Figure 18 shows the results of particle size measurement.

The particle size of the antibody/PEG-P[Lysₙ/(Lys-GA)ₘ]/Fe³⁺ complex was significantly increased compared with the particle size of the antibody solution regardless of the GA introduction ratio of PEG-P[Lysₙ/(Lys-GA)ₘ] used. On the other hand, the greatest increase in particle size of the MPN complex was observed when PEG-P[Lys₁₁/(Lys-GA)₁₄] was used.

### (4.8 Evaluation of Formation of Antibody-Loaded MPN Complexes When Varying the Degree of Polymerization of Lys on the Polymer)

### <Final Concentrations of Antibody, PEG-P[Lysₙ/(Lys-GA)ₘ], and Fe³⁺>

- Antibody: 200 nM
- PEG-P[Lys₁₁/(Lys-GA)₁₄]: 20 µM (stock concentration: 2,000 µM)
- PEG-P[Lys₇₂/(Lys-GA)₃₃]: 20 µM (stock concentration: 2,000 µM)
- Fe³⁺: 90 to 200 µM (stock concentration: 2.2 mM)

Each was prepared by dissolution in D-PBS(-).

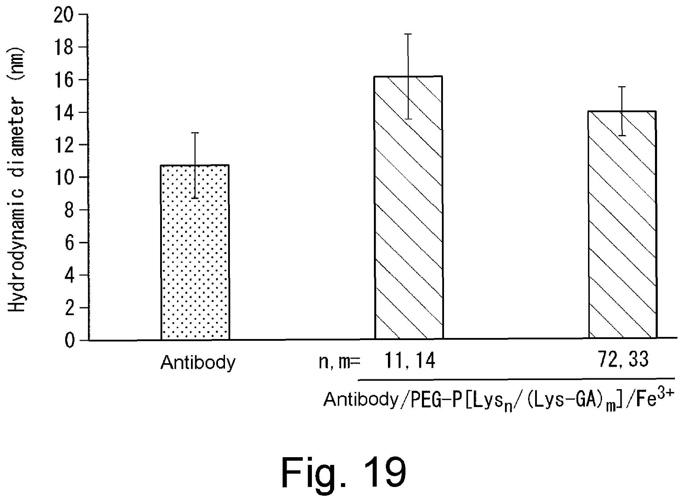

The antibody solution, each PEG-P[Lysₙ/(Lys-GA)ₘ] solution, and Fe³⁺ solution were mixed by pipetting. In doing so, the molar ratio of PEG-P[Lysₙ/(Lys-GA)ₘ]/antibody was set to 100. In addition, the molar ratio of iron ions was set to 1/3 equivalent relative to GA in PEG-P[Lysₙ/(Lys-GA)ₘ]. Finally, after the concentration was adjusted by adding PBS(-), particle size was measured by fluorescence correlation spectroscopy using LSM710. Figure 19 shows the results of particle size measurement.

The particle size of the antibody/PEG-P[Lysₙ/(Lys-GA)ₘ]/Fe³⁺ complex was significantly increased compared with the particle size of the antibody solution when PEG-P[Lysₙ/(Lys-GA)ₘ] having a Lys degree of polymerization of either 25 or 105 was used. On the other hand, a greater increase in particle size of the MPN complex was observed when PEG-P[Lys₁₁/(Lys-GA)₁₄] was used than when PEG-P[Lys₇₂/(Lys-GA)₃₃] was used.

### (4.9 Evaluation of Formation of Antibody-Loaded MPN Complex Using TEM)

### <Final Concentrations of Antibody, PEG-P[Lysₙ/(Lys-GA)ₘ, and Fe³⁺>

- Antibody: 6.6 µM
- PEG-P[Lys₁₁/(Lys-GA)₁₄]: 660 µM (stock concentration: 2,000 µM)
- PEG-P[Lys₇₂/(Lys-GA)₃₃]: 660 µM (stock concentration: 2,000 µM)
- Fe³⁺: 3.0 to 7.2 mM (stock concentration: 20 mM)

Each was prepared by dissolution in 1 mM HEPES buffer (pH 7.4).

The antibody solution, each PEG-P[Lysₙ/(Lys-GA)ₘ] solution, and Fe³⁺ solution were mixed by pipetting. In doing so, the molar ratio of PEG-P[Lysₙ/(Lys-GA)ₘ]/antibody was set to 100. In addition, the molar ratio of iron ions was set to 1/3 equivalent relative to GA in PEG-P[Lysₙ/(Lys-GA)ₘ]. Finally, the concentration was adjusted by adding 1 mM HEPS buffer of pH 7.4. Thereafter, 5 µL of the sample solution was dropped onto a copper grid, and the solution was removed after 1 minute. Thereafter, staining was performed using a gadolinium acetate solution, followed by washing with ultrapure water and then air drying for 1 day. TEM images of the sample-coated microgrids were captured using JEM1400.

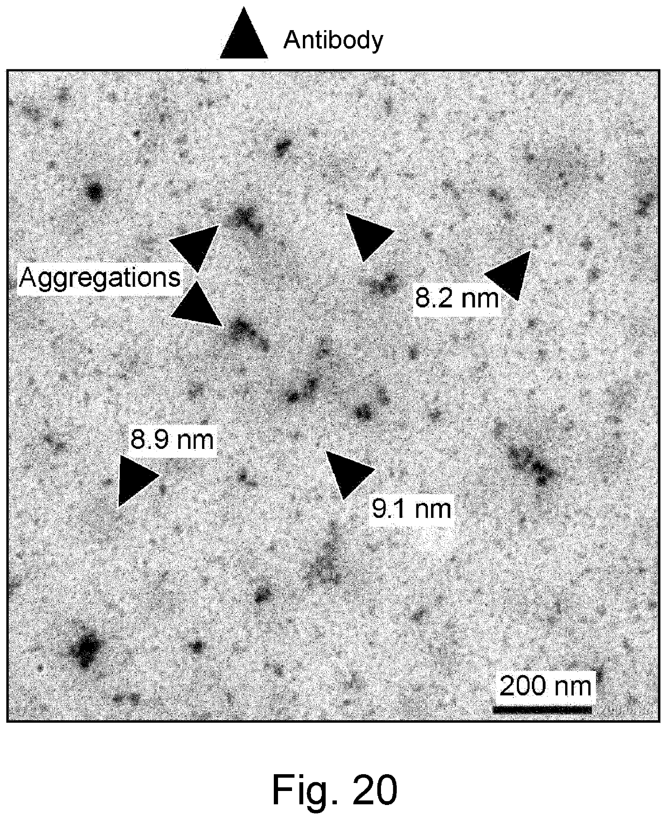

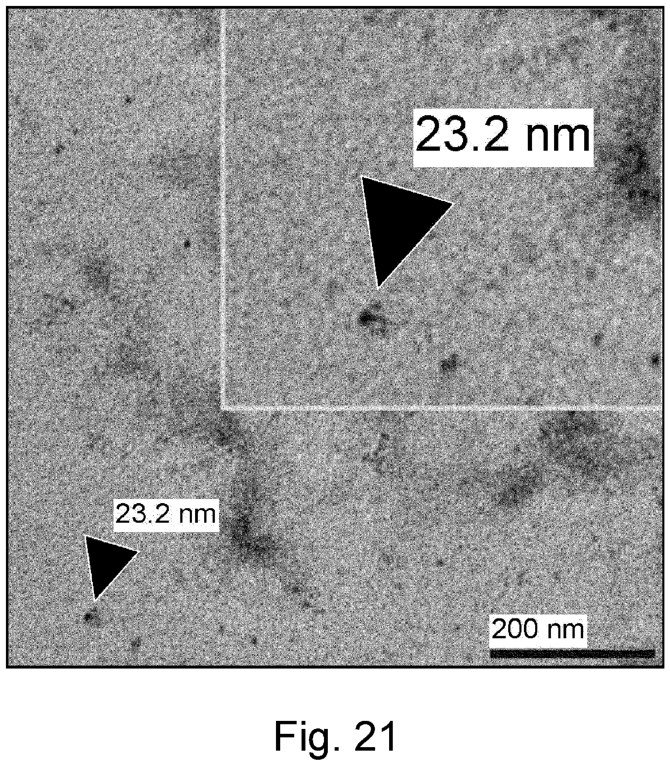

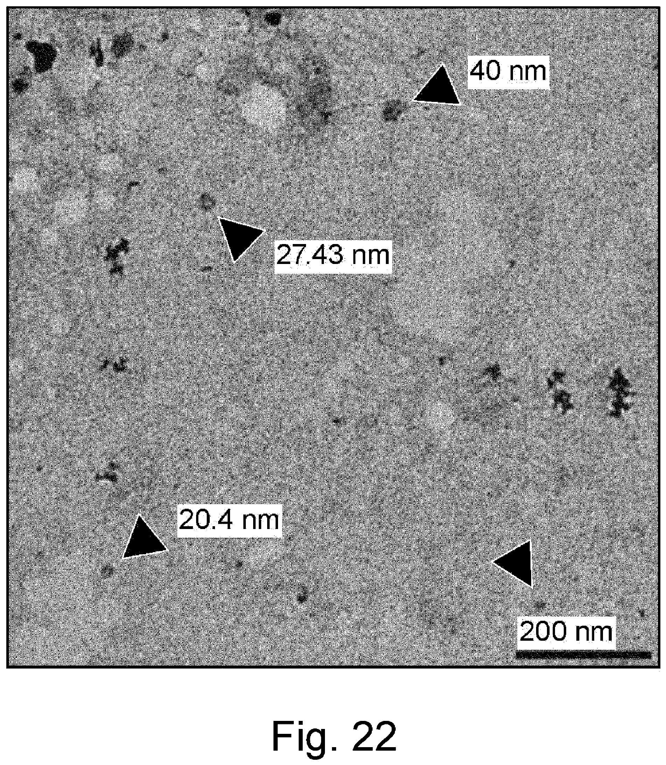

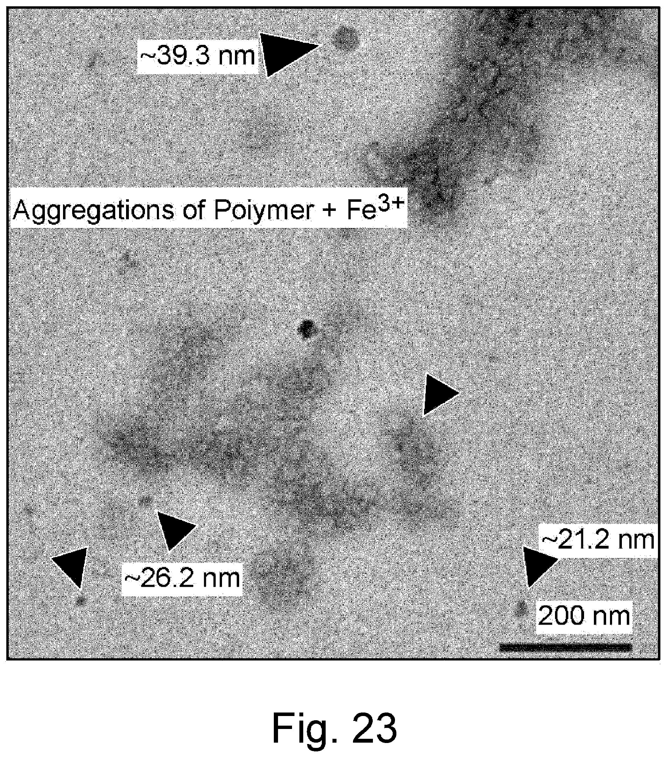

Figure 20 is a transmission electron microscopy (TEM) image of the antibody. Figure 21 is a TEM image of antibody/PEG-P[Lys₁₁/(Lys-GA)₁₄]. Figure 22 is a TEM image of antibody/PEG-P[Lys₁₁/(Lys-GA)₁₄]/Fe³⁺. Figure 23 is a TEM image of the antibody/PEG-P[Lys₇₂/(Lys-GA)₃₃]/Fe³⁺ MPN complex.

In the TEM images of the antibody/PEG-P[Lysₙ/(Lys-GA)ₘ]/Fe³⁺ complex, relatively large spherical particles were observed compared with the antibody alone, suggesting formation of the antibody-loaded MPN complex.

### (4.10 Evaluation of Formation of Antibody-Loaded MPN Complex Using PEG-TA)

### <Final Concentrations of Antibody, PEG-TA, and Fe³⁺>

- Antibody: 660 nM
- PEG-TA: 16 µM (stock concentration: 80 µM)
- Fe³⁺: 55 µM (stock concentration: 2.2 mM)

The antibody and PEG-TA were prepared by dissolution in D-PBS(-), and Fe³⁺ was prepared by dissolution in ultrapure water.

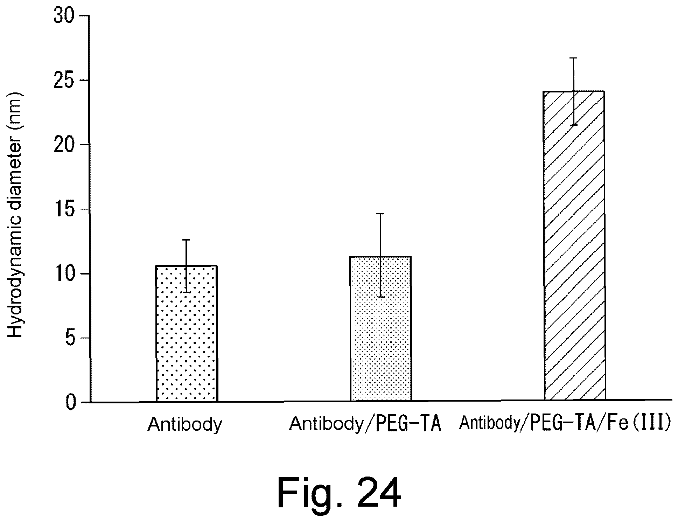

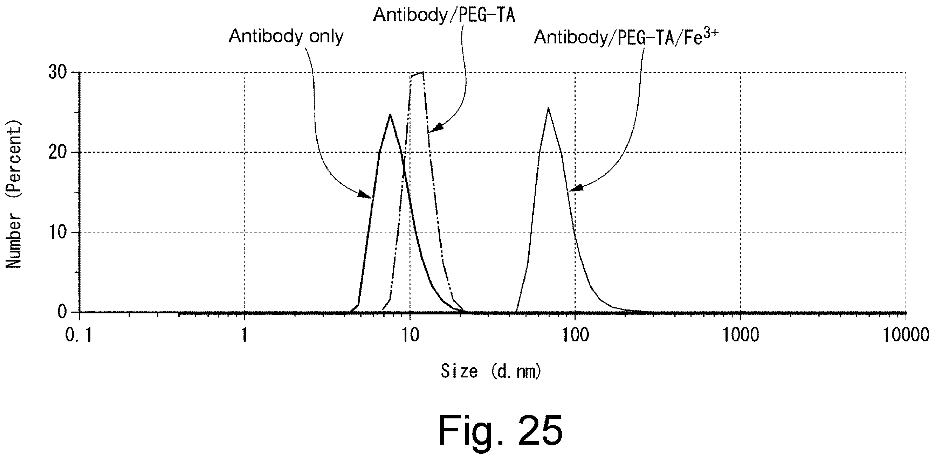

The antibody solution, PEG-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA. Finally, after the concentration was adjusted by adding PBS(-), particle size was measured by fluorescence correlation spectroscopy using LSM710. Figure 24 shows the results of particle size measurement. Particle size was measured using Zetasizer NanoZS. Figure 25 shows the results of particle size measurement.

The particle size of the antibody/PEG-TA/Fe³⁺ MPN complex was significantly increased compared with the particle sizes of the antibody alone and antibody/PEG-TA. These results demonstrated formation of the antibody/PEG-TA/Fe³⁺ MPN complex.

### (4.11 Measurement of Zeta Potential of Antibody-Loaded MPN Complex Formation)

### <Final Concentrations of Antibody, PEG-TA, and Fe³⁺>

- Antibody: 660 nM
- PEG-TA: 16 µM (stock concentration: 80 µM)
- Fe³⁺: 55 µM (stock concentration: 2.2 mM)

The antibody and PEG-TA were prepared by dissolution in 10 mM HEPES, and Fe³⁺ was prepared by dissolution in ultrapure water.

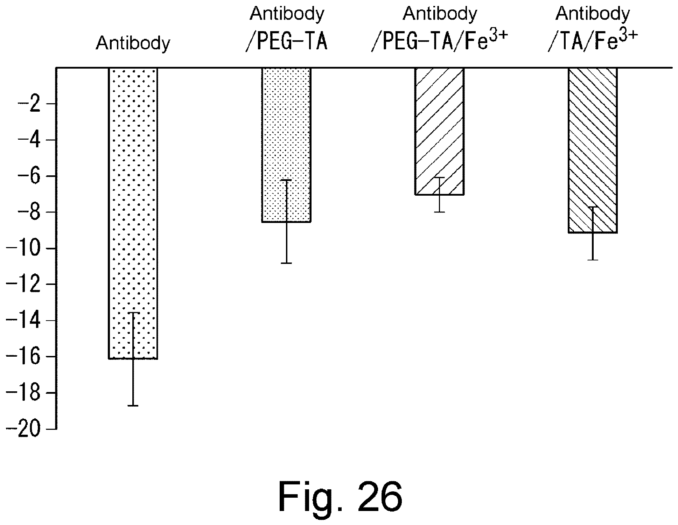

The antibody solution, PEG-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA. Subsequently, the concentration was adjusted by adding 10 mM HEPES, and the zeta potential was measured using Zetasizer NanoZS. Figure 26 shows results of zeta potential measurement.

The antibody alone exhibited a strong negative charge of -16 mV. However, upon formation of the antibody/PEG-TA/Fe³⁺ MPN complex, the zeta potential became -8 mV, indicating a reduction in negative charge and a value closer to 0.

### (4.12 Evaluation of pH Responsiveness of Antibody-Loaded MPN Complex)

### <Final Concentrations of Antibody, PEG-TA, and Fe³⁺>

- Antibody: 660 nM
- PEG-TA: 16 µM (stock concentration: 80 µM)
- Fe³⁺: 55 µM (stock concentration: 2.2 mM)

Each was prepared by dissolution in D-PBS(-) (pH 7.4), 50 mM HEPES buffer (pH 6.8), or 50 mM MES buffer (pH 6.0 and pH 5.5). Fe³⁺ was prepared by dissolution in ultrapure water.

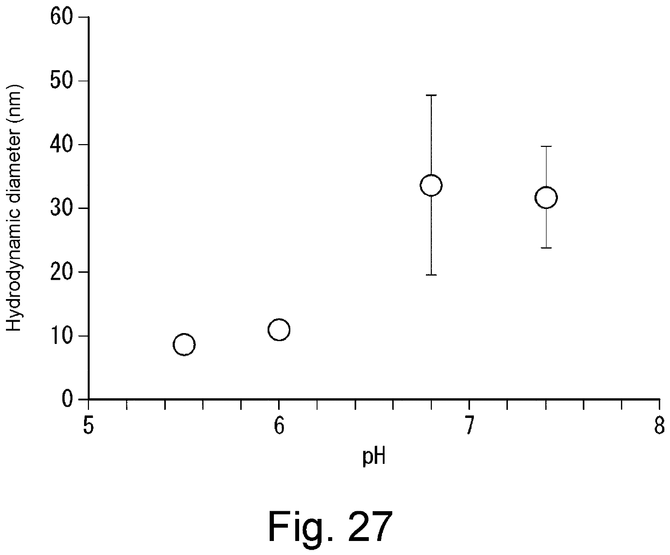

The antibody solution, PEG-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA. Subsequently, the concentration was adjusted by adding D-PBS(-), and particle size was measured by fluorescence correlation spectroscopy using LSM710. Figure 27 shows the results of particle size measurement.

The particle size of the antibody/PEG-TA/Fe³⁺ MPN complex at pH 6.8 was comparable to that of the antibody/PEG-TA/Fe³⁺ MPN complex at pH 7.4. In addition, the particle sizes of the antibody/PEG-TA/Fe³⁺ MPN complex at pH 5.5 and pH 6.0 were comparable to the particle size of the antibody alone, thus confirming that the antibody/PEG-TA/Fe³⁺ MPN complex had intracellular pH responsiveness.

### (4.13 Evaluation of Complex Formation in FBS)

### <Final Concentrations of Antibody, PEG-TA, and Fe³⁺>

- Antibody: 660 nM
- PEG-TA: 16 µM (stock concentration: 80 µM)
- Fe³⁺: 55 µM (stock concentration: 2.2 mM)

The antibody and PEG-TA were prepared by dissolution in D-PBS(-). Fe³⁺ was prepared by dissolution in ultrapure water. Subsequently, preparation was performed by addition to a mixed solution of FBS/D-PBS(-) (10/90, 30/70 (vol)) so as to achieve the above concentrations.

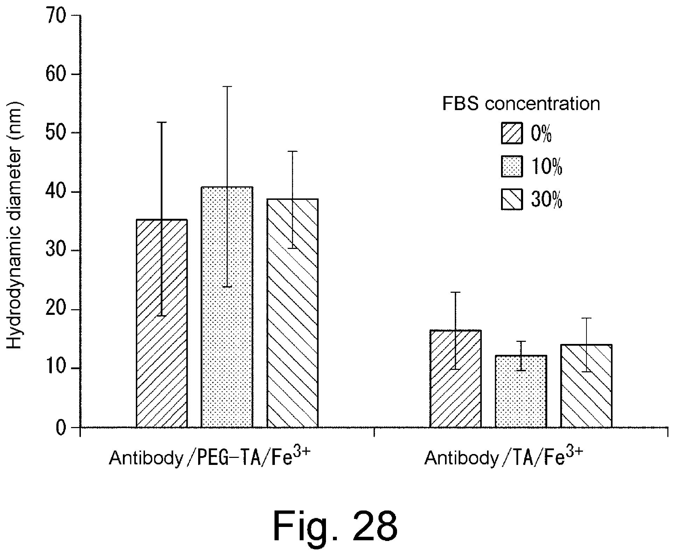

The antibody solution, PEG-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA. Subsequently, the concentration was adjusted by adding PBS(-), and particle size was measured by fluorescence correlation spectroscopy using LSM710. Figure 28 shows the results of particle size measurement.

Because the particle size of the antibody/PEG-TA/Fe³⁺ MPN complex did not change in FBS solutions of various concentrations, it was confirmed to be stable in FBS solutions.

### [Experimental Example 5]

### <Evaluation of Physicochemical Properties of Nucleic Acid Complex (RNP)-Loaded MPN Complexes>

### (5.1 Overview)

When an MPN complex is formed by mixing Cas9-sgRNA RNP, PEG-TA, and metal ions, the particle size increases compared with that of RNP alone. Accordingly, particle size measurement was performed by fluorescence correlation spectroscopy using Cas9-GFP. Zr⁴⁺, Fe³⁺, and Co²⁺, which differ in valence, were used as the metal ions.

### (5.2 Reagents)

- Cas9-GFP protein (Sigma-Aldrich)
- sgRNA (Luciferase-2) (GenScript)
- PEG₁₀ₖ-TA (Mₙ = 11,700)
- PEG₅ₖ-TA (Mₙ = 6,700)
- Zirconium(IV) oxide (ZrO₂) (Zr⁴⁺) (FUJIFILM Wako Pure Chemical Corporation)
- Cobalt(II) nitrate hexahydrate (Co(NO₃)₂·6H₂O) (Co²⁺) (FUJIFILM Wako Pure Chemical Corporation)
- D-PBS(-) (FUJIFILM Wako Pure Chemical Corporation)
- Iron(III) chloride hexahydrate (FeCl₃·6H₂O) (Fe³⁺) (FUJIFILM Wako Pure Chemical Corporation)

### (5.3 Measurement Instruments)

- LSM710 (Carl Zeiss)
- JEM-1400 (JEOL)

### (5.4 Evaluation of Formation of RNP MPN Complexes Using Zr⁴⁺ and Co²⁺)

### <Final Concentrations of Cas9-sgRNA, PEG-TA, ZrO₂, and Co(NO₃)₂·6H₂O>

- Cas9-GFP: 0.2 µM
- sgRNA: 0.2 µM
- PEG₁₀ₖ-TA: 20 µM
- PEG₅ₖ-TA: 20 µM
- ZrO₂ (Zr⁴⁺): 67 µM
- Co(NO₃)₂·6H₂O(Co²⁺): 22 µM

Cas9-sgRNA and PEG-TA were prepared by dissolution in D-PBS(-). Zr⁴⁺ and Co²⁺ were prepared by dissolution in pure water.

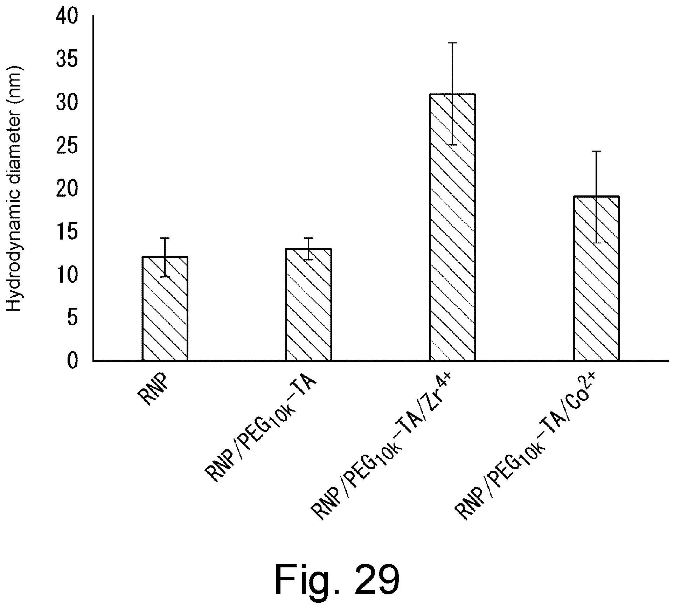

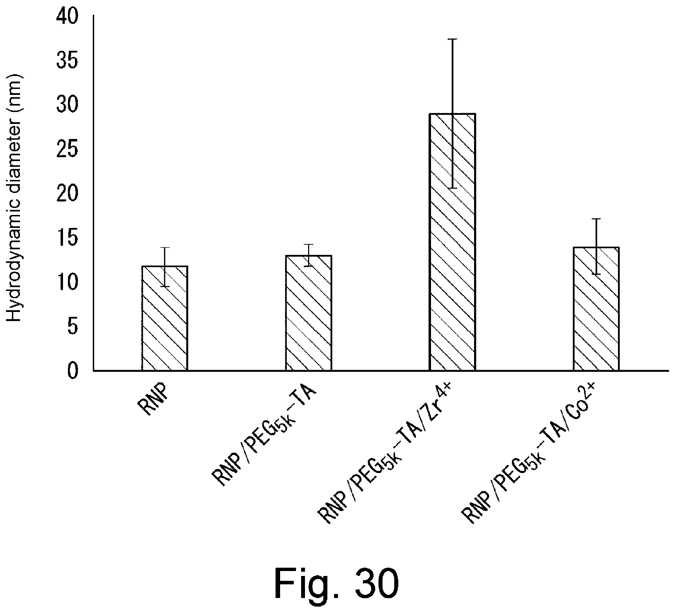

Cas9-GFP and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, PEG₁₀ₖ-TA or PEG₅ₖ-TA was added, followed by standing at room temperature for 15 minutes to prepare both RNP/PEG-TA solutions. To this was added Zr⁴⁺ or Co²⁺, followed by standing at room temperature for 15 minutes to prepare both RNP/PEG-TA/Zr⁴⁺ solutions and both RNP/PEG-TA/Co²⁺. In doing so, the molar ratio of PEG-TA/RNP was set to 25. In addition, the molar ratio of metal ions was set to 3.3 equivalents relative to TA in PEG-TA. Finally, particle size was measured by fluorescence correlation spectroscopy using LSM710. Figures 29 and 30 show the results of particle size measurement.

The particle sizes of RNP/PEG-TA/Zr⁴⁺ and RNP/PEG-TA/Co²⁺ were significantly increased compared with the particle sizes of RNP and RNP/PEG-TA, regardless of the molecular weight of PEG. These results suggested formation of the RNP/PEG-TA/Zr⁴⁺ MPN complex and the RNP/PEG-TA/Co²⁺ MPN complex. On the other hand, the metal ion Zr⁴⁺, which has a higher valence, exhibited a greater increase in particle size than when Co²⁺ was used, suggesting formation of a more stable complex.

### (5.5 Evaluation of Formation of RNP MPN Complexes Using Fe³⁺)

### <Final Concentrations of Cas9-sgRNA, PEG-TA, and FeCl₃·6H₂O>

- Cas9-GFP: 0.2 µM
- sgRNA: 0.2 µM
- PEG₁₀ₖ-TA: 20 µM
- FeCl₃·6H₂O (Fe³⁺): 67 µM

Cas9-sgRNA and PEG-TA were prepared by dissolution in D-PBS(-). FeCl₃·6H₂O was prepared by dissolution in pure water.

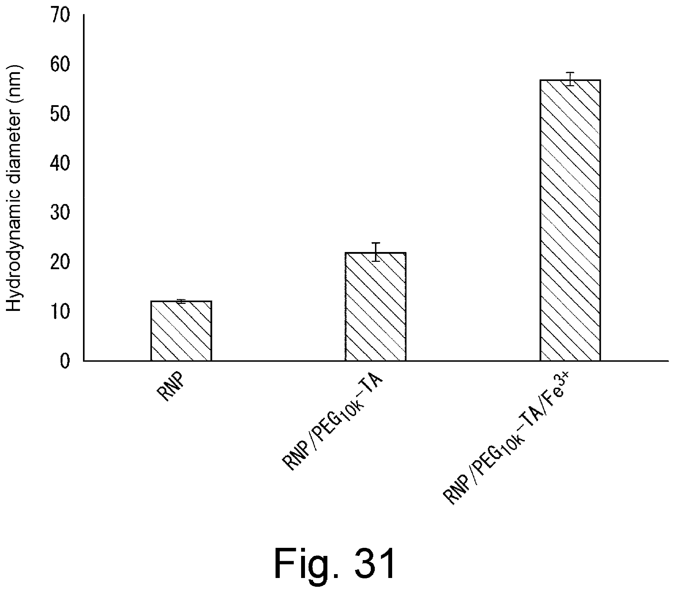

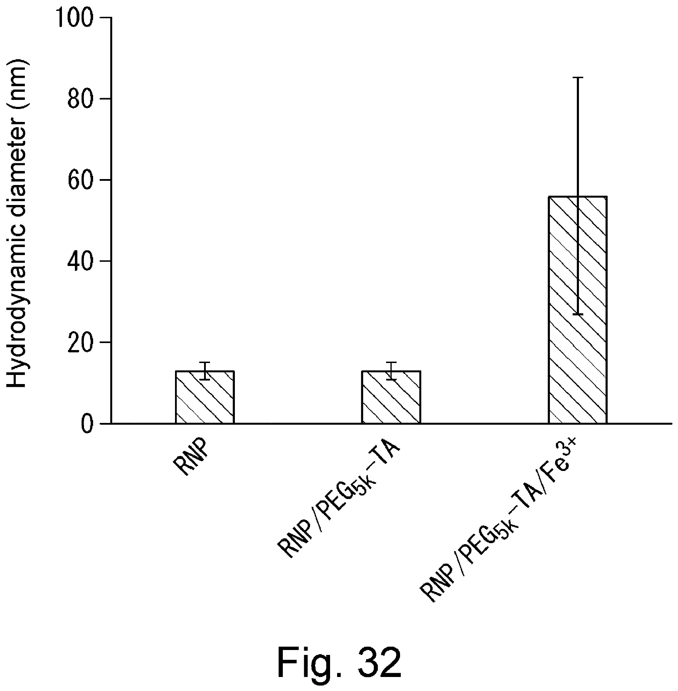

Cas9-GFP and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, PEG₁₀ₖ-TA and PEG₅ₖ-TA were added, followed by standing at room temperature for 15 minutes to prepare both RNP/PEG-TA solutions. Fe³⁺ was further added, followed by standing at room temperature for 15 minutes to prepare both RNP/PEG₁₀ₖ-TA/Fe³⁺ MPN complex solutions. In doing so, the molar ratio of PEG-TA/RNP was set to 100. In addition, the molar ratio of iron ions was set to 3.3 equivalents relative to TA in PEG-TA. Finally, particle size was measured by fluorescence correlation spectroscopy using LSM710. Figures 31 and 32 show the results of particle size measurement.

The particle sizes of both RNP/PEG-TA/Fe³⁺ were significantly increased compared with the particle sizes of RNP and RNP/PEG-TA. These results suggested formation of both RNP/PEG-TA/Fe³⁺ MPN complexes.

### (5.6 Evaluation of Formation of RNP-Loaded MPN Complex Using TEM)

### <Final Concentrations of Cas9-sgRNA, PEG-TA, and FeCl₃·6H₂O>

- Cas9-GFP: 0.2 µM
- sgRNA: 0.2 µM
- PEG₁₀ₖ-TA: 20 µM
- FeCl₃·6H₂O (Fe³⁺): 67 µM

Cas9-sgRNA and PEG-TA were prepared by dissolution in 1 mM HEPES buffer (pH 7.4). Fe³⁺ was prepared by dissolution in pure water.

Cas9-GFP and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, PEG₁₀ₖ-TA and PEG₅ₖ-TA were added, followed by standing at room temperature for 15 minutes to prepare both RNP/PEG-TA solutions. Fe³⁺ was further added, followed by standing at room temperature for 15 minutes to prepare both RNP/PEG₁₀ₖ-TA/Fe³⁺ MPN complex solutions. In doing so, the molar ratio of PEG-TA/RNP was set to 100. In addition, the molar ratio of iron ions was set to 3.3 equivalents relative to TA in PEG-TA. Subsequently, 5 µL of the sample solution was dropped onto a copper grid, and the solution was removed after 1 minute. The sample was then stained using a gadolinium acetate solution. The sample was then washed with ultrapure water and air-dried for 1 day. TEM images were then captured using a transmission electron microscope.

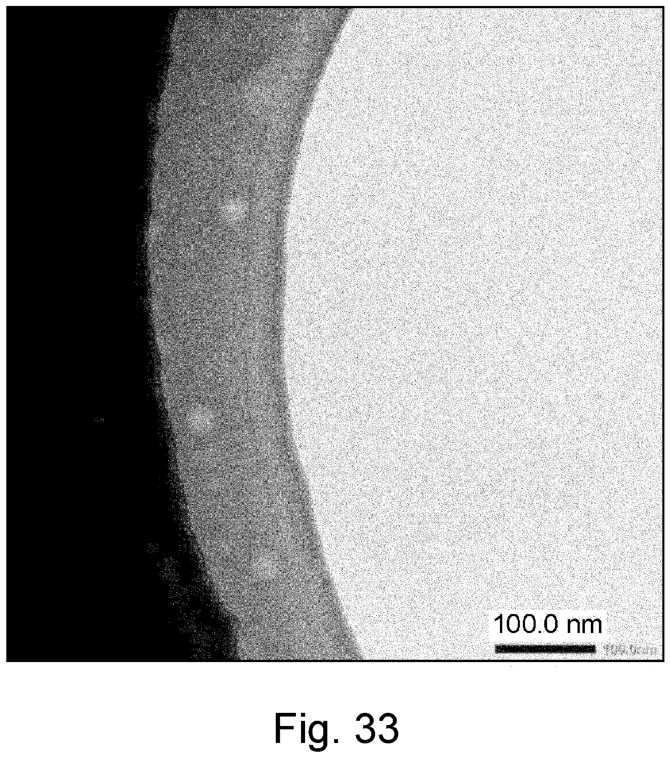

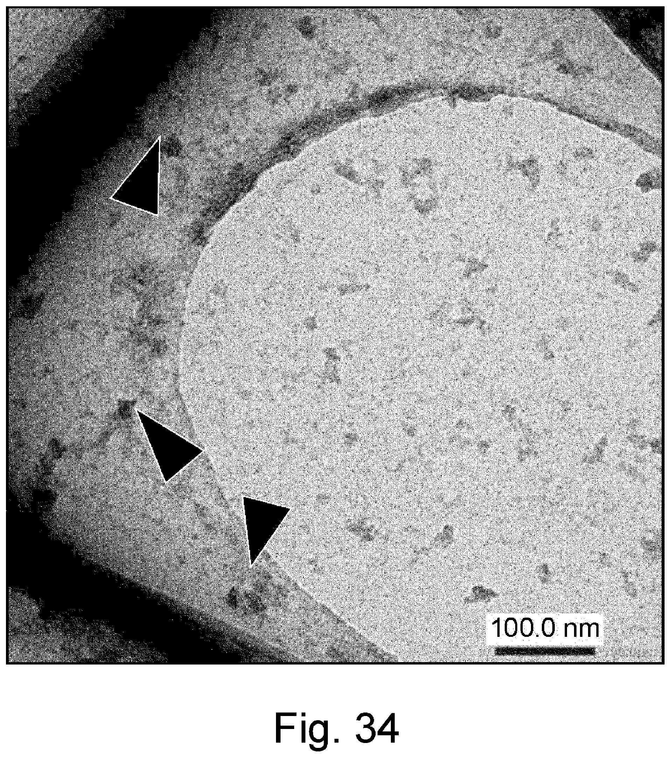

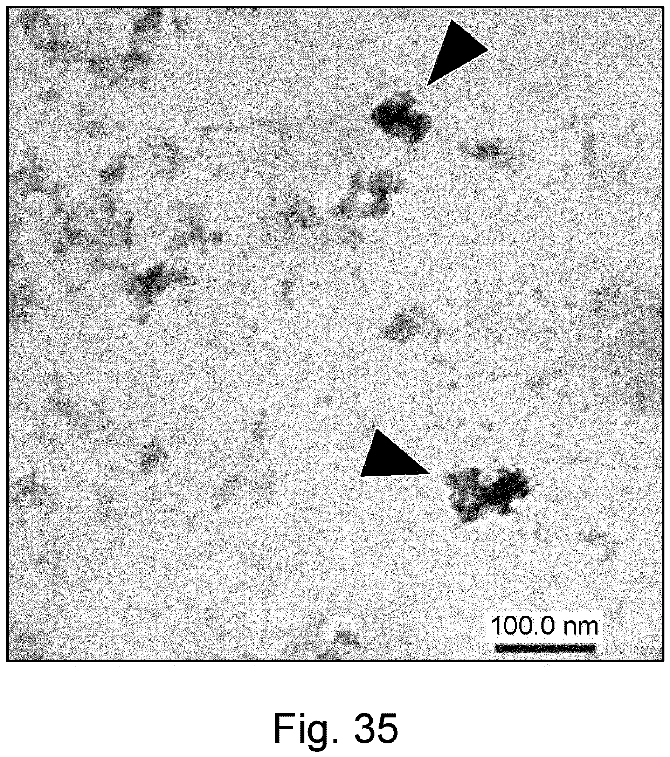

Figure 33 is a TEM image of RNP. Figure 34 is a TEM image of RNP/PEG₁₀ₖ-TA. In Figure 34, the arrowheads indicate RNP/PEG₁₀ₖ-TA. Figure 35 is a TEM image of RNP/PEG₁₀ₖ-TA/Fe³⁺. In Figure 35, the arrowheads indicate RNP/PEG₁₀ₖ-TA/Fe³⁺. In the TEM images, the RNP/PEG₁₀ₖ-TA/Fe³⁺ complex was observed as relatively large particles of about 70 to 100 nm compared with RNP alone. These results suggested formation of the RNP-loaded MPN complex.

### [Experimental Example 6]

### <Evaluation of Cytotoxicity of PEG-TA>

### (6.1 Overview)

The cytotoxicity of the synthesized PEG₁₀ₖ-TA and PEG₁₀ₖ-TA/Fe³⁺ was evaluated by a CCK assay using Cell Counting Kit-8 (Dojindo Laboratories).

### (6.2 Reagents)

- α-Methoxy-ω-amino-poly(ethylene glycol) (PEG₁₀ₖ-NH₂) [Mn: 10k] (NOF Corporation)
- PEG₁₀ₖ-TA (Mₙ = 11,700)
- Iron(III) Chloride Hexahydrate (FeCl₃·6H₂O)
- Iron(III) chloride hexahydrate (FeCl₃·6H₂O) (Fe³⁺) (FUJIFILM Wako Pure Chemical Corporation)
- D-PBS(-) (FUJIFILM Wako Pure Chemical Corporation)
- Cell Counting Kit-8 (Dojindo Laboratories)
- Roswell Park Memorial Institute medium (RPMI) (Sigma-Aldrich)
- Fetal bovine serum (FBS) (Biosera)
- Trypsin-EDTA solution (Sigma-Aldrich)
- Penicillin/streptomycin (Sigma-Aldrich)
- HeLa-Luc cells (aneuploid epithelial-like cell line stably expressing luciferase) (American Type Culture Collection, ATCC)

### (6.3 Measurement Instruments)

- Multi-plate reader (Spark) (Tecan)

### (6.4 Cytotoxicity Evaluation)

### <Final Concentrations of PEG₁₀ₖ-NH₂, PEG₁₀ₖ-TA, and FeCl₃·6H₂O>

- PEG₁₀ₖ-NH₂: 5 to 250 µM
- PEG₁₀ₖ-TA (Mn = 11,700): 5 to 250 µM
- FeCl₃·6H₂O (Fe³⁺): 16.5 to 825 µM

These were prepared by dissolution in D-PBS(-). Fe³⁺ was prepared by dissolution in pure water.

From the above, a PEG₁₀ₖ-NH₂ solution, a PEG₁₀ₖ-TA solution, and a FeCl₃·6H₂O solution were prepared. Furthermore, PEG₁₀ₖ-TA/Fe³⁺ solutions were prepared by adding Fe³⁺ to each PEG₁₀ₖ-TA solution such that PEG₁₀ₖ-TA:Fe³⁺ = 3:10.

### <Cytotoxicity Evaluation by CCK Assay>

HeLa-Luc cells were seeded in a 96-well plate at 3.0 × 10³ cells/plate and incubated at 37°C under 5% CO₂ for 24 hours. 100 µL each of the above PEG₁₀ₖ-NH₂ solution, RNP/PEG₁₀ₖ-TA solution, PEG₁₀ₖ-TA/Fe³⁺ solution, and FeCl₃·6H₂O solution were added to each well, followed by incubation for 72 hours. After washing with 150 µL/well of D-PBS(-), 110 µL/well of a CCK measurement solution prepared by mixing 10 µL of CCK and 100 µL of PBS(-) was added, followed by incubation for 1 hour. Thereafter, the absorbance at 460 nm was measured using a plate reader.

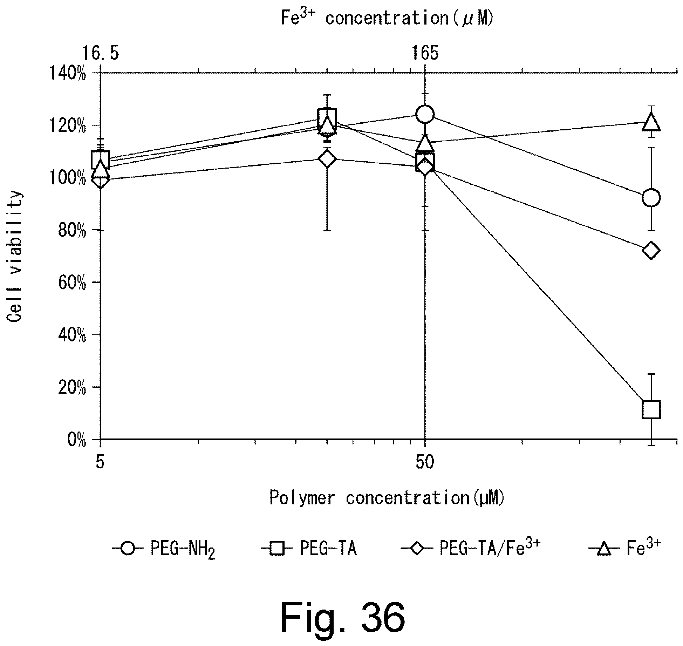

Then, cytotoxicity was evaluated by normalizing the absorbance of wells in which cells treated with each sample were present to the absorbance of wells in which untreated cells were present. Figure 36 is a graph showing the results of measuring cell survival rate by a CCK assay. The results confirmed that although cytotoxicity was observed with PEG-TA alone, the toxicity was suppressed by the addition of Fe³⁺.

### [Experimental Example 7]

### <Evaluation of Antibody MPN Complex in Cultured Cells>

### (7.1 Overview)

The intracellular distribution of an antibody-loaded MPN complex was observed using a confocal microscope to confirm the cellular uptake pathway. In addition, in the present Experimental Example, the antigen recognition function of the loaded antibody was also evaluated by using an anti-Nuclear Pore Complex Proteins Antibody (anti-NPC antibody) capable of targeting the cell nucleus.

### (7.2 Reagents and Cell Lines)

- Cy5-labeled antibody (antibody)
- Alexa Fluor^{(R)} 647 anti-Nuclear Pore Complex Proteins Antibody (anti-NPC antibody) (BioLegend)
- PEG-P[Lysₙ/(Lys-GA)ₘ] (m+n = 25, 105)
- PEG₅ₖ-TA (Mₙ = 11,700)
- PEG₁₀ₖ-TA (Mₙ = 5,700)
- D-PBS(-) (FUJIFILM Wako Pure Chemical Corporation)
- Roswell Park Memorial Institute medium (RPMI) (Sigma-Aldrich)
- Fetal bovine serum (FBS) (Biosera)
- Trypsin-EDTA solution (Sigma-Aldrich)
- Penicillin/streptomycin (Sigma-Aldrich)
- 5 mol/L HCl (FUJIFILM Wako Pure Chemical Corporation)
- CT26 cells (mouse colon cancer-derived cell line) (American Type Culture Collection, ATCC)
- 4T1-Luc cells (mouse breast cancer-derived cell line) (American Type Culture Collection, ATCC)
- LysoTracker (registered trademark) Red DND-99 (Thermo Fisher Scientific)
- Hoechst 33342 (Thermo Fisher Scientific)
- Paraformaldehyde (Nacalai Tesque), used as a 4% paraformaldehyde/D-PBS(-) solution

### (7.3 Measurement Instruments)

- Countess (Thermo Fisher Scientific)
- LSM710 (Carl Zeiss)
- Flow cytometer (Guava easyCyte 6-2L): (Merck Millipore, Ex/Em: 642/661 nm)

### (7.4 Observation of Intracellular Distribution of Antibody-Loaded MPN Complex by Confocal Microscopy)

### <Final Concentrations of Antibody, PEG-P[Lysₙ/(Lys-GA)ₘ, and Fe³⁺>

- Antibody: 3.3 µM
- PEG-P[Lys₁₁/(Lys-GA)₁₄]: 330 µM (stock concentration: 2,000 µM)
- Fe³⁺: 1.5 mM (stock concentration: 20 mM)

Each was prepared by dissolution in PBS(-).

The antibody solution, each PEG-P[Lys₁₁/(Lys-GA)₁₄] solution, and Fe³⁺ solution were mixed by pipetting. In doing so, the molar ratio of PEG-P[Lys₁₁/(Lys-GA)₁₄]/antibody was set to 100. In addition, the molar ratio of iron ions was set to 1/3 equivalent relative to GA in PEG-P[Lys₁₁/(Lys-GA)₁₄].

### <Observation by Confocal Microscopy>

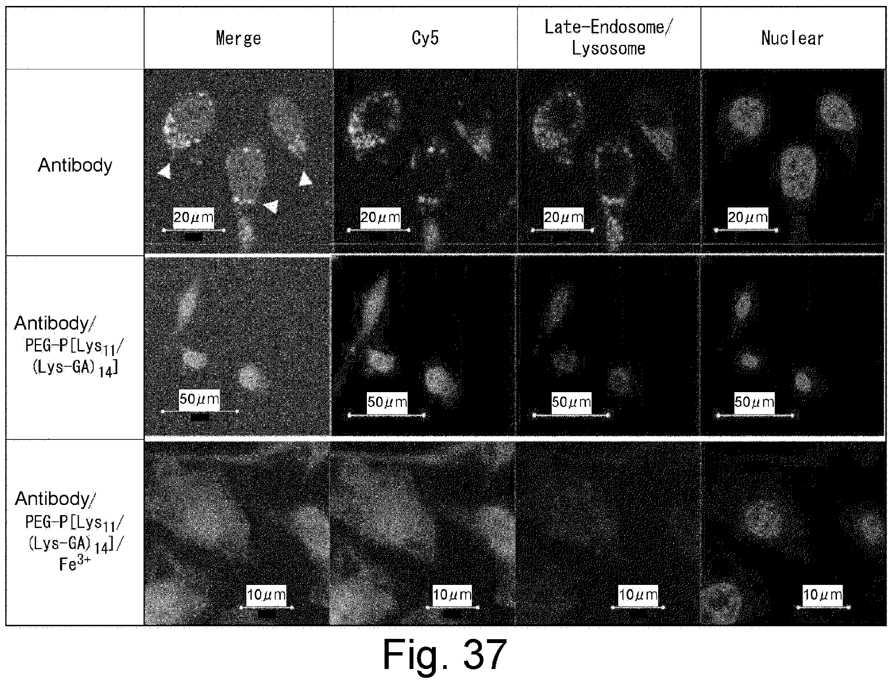

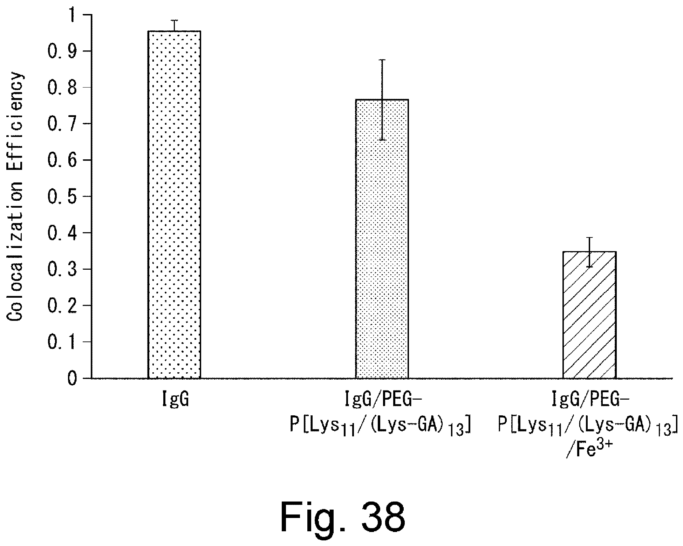

CT26 cells were seeded in 35 mm² glass based dishes at 5.0 × 10⁴ cells/dish and precultured at 37°C under 5% CO₂ for 24 hours. 500 µL of the above antibody sample solution and 500 µL of RPMI medium were added to each dish, followed by incubation for 48 hours. After washing with 1 mL of D-PBS(-), 1 mL of a 100 nM LysoTracker Red DND-99/(D-PBS(-) :RPMI = 1:9) solution was added, followed by incubation for 30 minutes. After washing with 1 mL of D-PBS(-), incubation was performed with a 4% paraformaldehyde/D-PBS(-) solution for 4 minutes. After washing with 1 mL of D-PBS(-), 1 mL of a 5.0 µg/mL Hoechst/D-PBS(-) solution was added, followed by incubation for 5 minutes. After washing twice with 1 mL of D-PBS(-), 2 mL of RPMI was added, and observation was performed using a confocal microscope. Figure 37 shows the captured microscopic images. Figure 38 is a graph showing the results of calculating the colocalization efficiency of fluorescence derived from Lysotracker and fluorescence derived from Cy5 labeled to the antibody.

With the antibody alone and antibody/PEG-P[Lys₁₁/(Lys-GA)₁₄], fluorescence derived from the antibody was observed to be colocalized with lysosomes. In contrast, in the antibody/PEG-P[Lys₁₁/(Lys-GA)₁₄]/Fe³⁺ MPN complex, fluorescence derived from the antibody was observed to be dispersed throughout the cytoplasm. In addition, the colocalization efficiency of fluorescence derived from Cy5 and Lysotracker was markedly decreased for the antibody/PEG-P[Lys₁₁/(Lys-GA)₁₄]/Fe³⁺ MPN complex compared with the antibody alone and antibody/PEG-P[Lys₁₁/(Lys-GA)₁₄]. These results indicate that the antibody [Lys₁₁/(Lys-GA)₁₄]/Fe³⁺ MPN complex can escape from lysosomes and translocate into the cytoplasm after being taken up into cells.

### (7.5 Functional Evaluation of Anti-NPC Antibody-Loaded MPN Complex by Confocal Microscopy)

### <Final Concentrations of Antibody, PEG-P[Lysₙ/(Lys-GA)ₘ], and Fe³⁺>

- Anti-NPC antibody: 1.7 µM
- PEG-P[Lys₁₁/(Lys-GA)₁₄]: 170 µM (stock concentration: 2,000 µM)
- Fe³⁺: 750 mM (stock concentration: 20 mM)

Each was prepared by dissolution in PBS(-).

The anti-NPC antibody solution, PEG-P[Lys₁₁/(Lys-GA)₁₄] solution, and Fe³⁺ solution were mixed by pipetting. In doing so, the molar ratio of PEG-P[Lys₁₁/(Lys-GA)₁₄]/antibody was set to 100. In addition, the molar ratio of iron ions was set to 1/3 equivalent relative to GA in PEG-P[Lys₁₁/(Lys-GA)₁₄].

### <Observation by Confocal Microscopy>

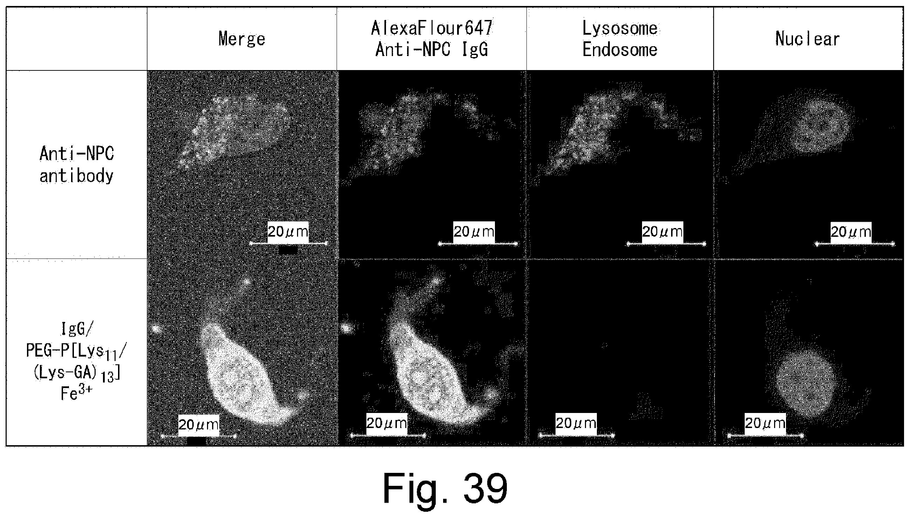

CT26 cells were seeded in 35 mm² glass based dishes at 5.0 × 10⁴ cells/dish and precultured at 37°C under 5% CO₂ for 24 hours. 100 µL of the above antibody sample solution and 100 µL of RPMI medium were added to each dish, followed by incubation for 48 hours. After washing with 1 mL of D-PBS(-), 1 mL of a 100 nM LysoTracker (registered trademark) Red DND-99/(D-PBS(-):RPMI = 1:9) solution was added, followed by incubation for 30 minutes. After washing with 1 mL of D-PBS(-), incubation was performed with a 4% paraformaldehyde/D-PBS(-) solution for 4 minutes. After washing with 1 mL of D-PBS(-), 1 mL of a 5.0 µg/mL Hoechst/D-PBS(-) solution was added, followed by incubation for 5 minutes. After washing twice with 1 mL of D-PBS(-), 2 mL of RPMI was added, and observation was performed using a confocal microscope. Figure 39 shows the captured microscopic images.

With the anti-NPC antibody alone, fluorescence derived from the antibody was colocalized with lysosomes, and no colocalization of the anti-NPC antibody with the nucleus was observed. In contrast, with the anti-NPC antibody MPN complex, the anti-NPC antibody was observed to be dispersed throughout the cytoplasm and colocalized with the nucleus. These results indicate that the anti-NPC antibody/PEG-P[Lys₁₁/(Lys-GA)₁₄]/Fe³⁺ MPN complex can escape from lysosomes, translocate into the cytoplasm, and target the nucleus after being taken up into cells.

### (7.6 Evaluation of Cellular Uptake Amount of Antibody-Loaded MPN Complex)

### <Final Concentrations of Antibody, PEG-TA, and Fe³⁺>

- Antibody: 1.7 µM
- PEG₁₀ₖ-TA: 42.5 µM (stock concentration: 200 µM)
- PEG₅ₖ-TA: 42.5 µM (stock concentration: 200 µM)
- Fe³⁺: 141 µM (stock concentration: 20 mM)

The antibody and each PEG-TA were prepared by dissolution in 10 mM HEPES, and Fe³⁺ was prepared by dissolution in ultrapure water.

The antibody solution, PEG-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA.

### <Observation by Confocal Microscopy>

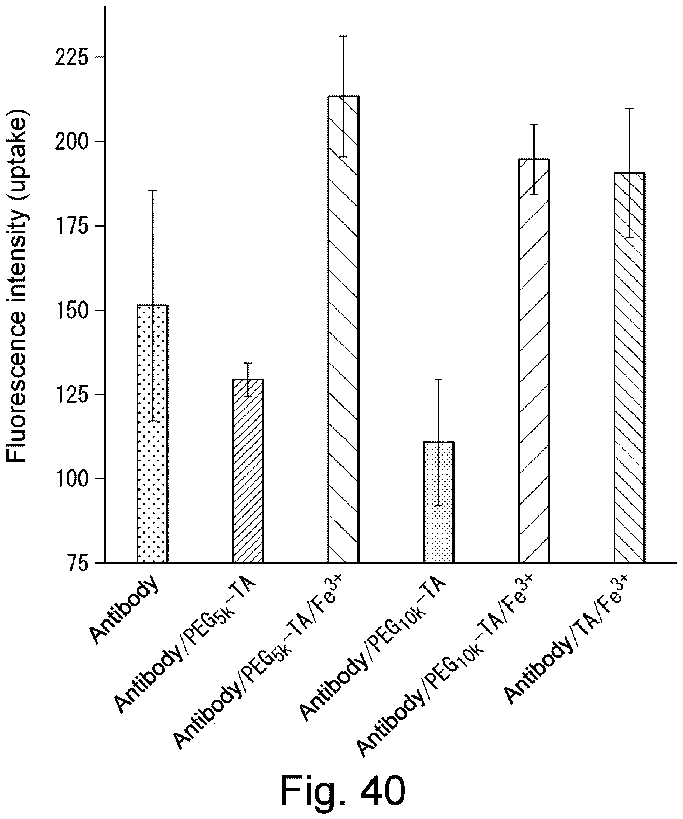

4T1-Luc cells were seeded in a 24-well plate at 2.5 × 10⁴ cells/plate and precultured at 37°C under 5% CO₂ for 24 hours. 50 µL of the above antibody sample solution and 150 µL of RPMI medium were added to each dish, followed by incubation for 24 hours. Thereafter, the solution was removed and washing with PBS(-) was performed twice, after which 150 µL of trypsin was added and incubation was performed at 37°C for 7 minutes, followed by addition of 150 µL of RPMI medium and measurement by flow cytometry (FCM). Figure 40 is a graph showing the results of measuring, by flow cytometry, the fluorescence of the Cy5-labeled antibody taken up into cells.

Cellular uptake of antibody/PEG₅ₖ-TA and antibody/PEG₁₀ₖ-TA was decreased compared with the antibody alone. In contrast, cellular uptake of the antibody/PEG₅ₖ-TA/Fe³⁺ MPN complex and the antibody/PEG₁₀ₖ-TA/Fe³⁺ MPN complex was increased compared with the antibody alone. These results indicate that cellular uptake is increased by forming the MPN complex.

### (7.7 Functional Evaluation of Anti-NPC Antibody-Loaded MPN Complex by Confocal Microscopy)

### <Final Concentrations of Antibody and PEG-TA>

- Antibody: 1.7 µM
- PEG₁₀ₖ-TA: 42.5 µM (stock concentration: 200 µM)
- Fe³⁺: 141 µM (stock concentration: 20 mM)

The antibody and each PEG-TA were prepared by dissolution in 10 mM HEPES, and Fe³⁺ was prepared by dissolution in ultrapure water.

The antibody solution, PEG₁₀ₖ-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA.

### <Observation by Confocal Microscopy>

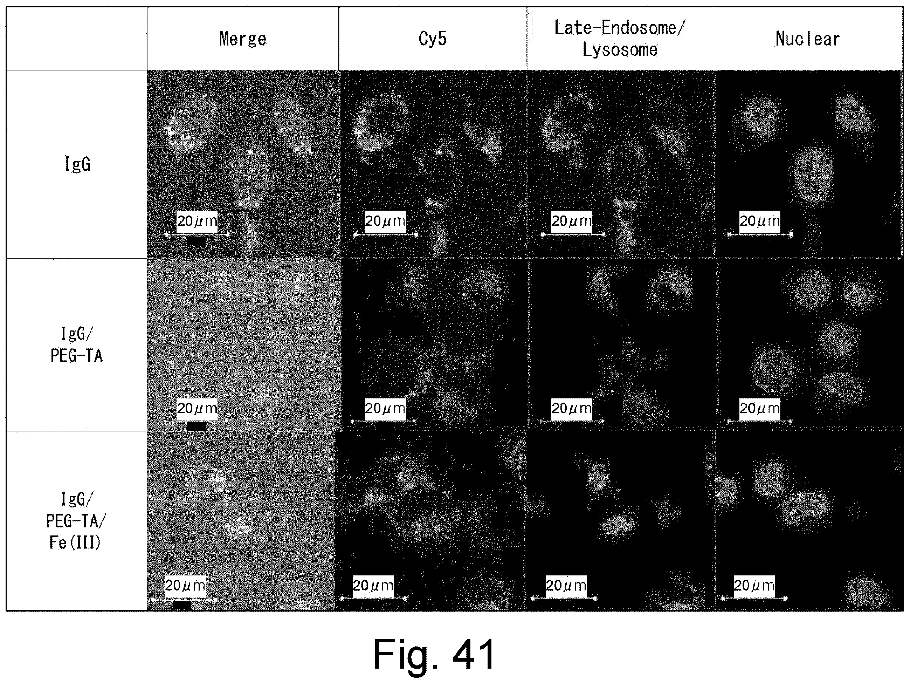

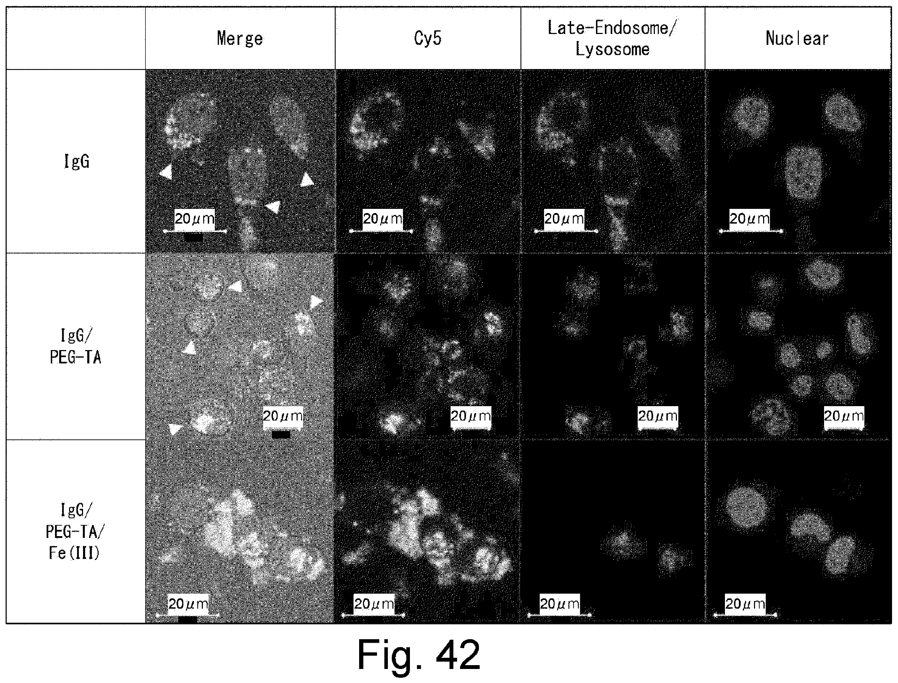

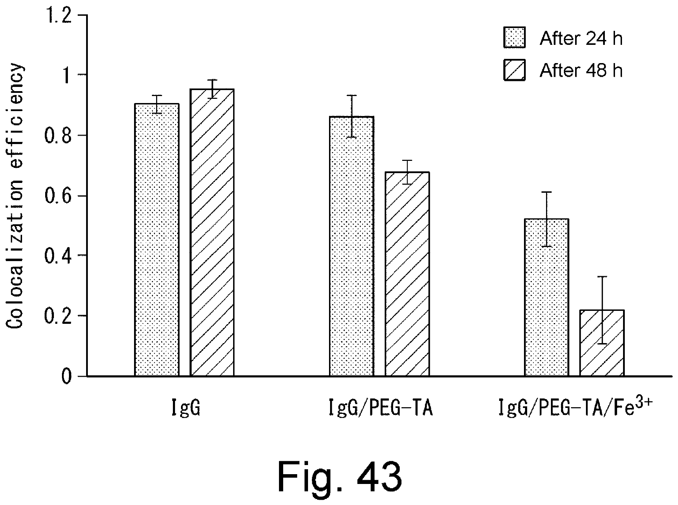

4T1-Luc cells were seeded in 35 mm² glass based dishes at 5.0 × 10⁴ cells/dish and precultured at 37°C under 5% CO₂ for 24 hours. 500 µL of the above antibody sample solution and 500 µL of RPMI medium were added to each dish, followed by incubation for 24 and 48 hours. After washing with 1 mL of D-PBS(-), 1 mL of a 100 nM LysoTracker (registered trademark) Red DND-99/(D-PBS(-):RPMI = 1:9) solution was added, followed by incubation for 30 minutes. After washing with 1 mL of D-PBS(-), incubation was performed with a 4% paraformaldehyde/D-PBS(-) solution for 4 minutes. After washing with 1 mL of D-PBS(-), 1 mL of a 5.0 µg/mL Hoechst/D-PBS(-) solution was added, followed by incubation for 5 minutes. After washing twice with 1 mL of D-PBS(-), 2 mL of RPMI was added, followed by incubation for 24 hours or 48 hours and observation using a confocal microscope. Figure 41 shows the microscopic images captured after 24-hour incubation. Figure 42 shows the microscopic images captured after 48-hour incubation. Figure 43 is a graph showing the results of measuring the colocalization efficiency of fluorescence derived from Lysotracker and fluorescence derived from Cy5 labeled to the antibody.

With the antibody alone and antibody/PEG-TA, fluorescence derived from the antibody was observed to be colocalized with lysosomes both after 24-hour incubation and after 48-hour incubation. In contrast, with the antibody/PEG-TA/Fe³⁺ MPN complex, fluorescence derived from the antibody was observed to be dispersed throughout the cytoplasm. In particular, after 48-hour incubation, marked dispersion throughout the cells was observed, and the colocalization efficiency of fluorescence derived from Cy5 and Lysotracker for the antibody/PEG-TA/Fe³⁺ MPN complex was markedly decreased compared with the antibody alone and antibody/PEG-TA.

### (7.7 Functional Evaluation of Anti-NPC Antibody-Loaded MPN Complex by Confocal Microscopy)

### <Final Concentrations of Antibody and PEG-TA>

- Anti-NPC antibody: 1.7 µM
- PEG₁₀ₖ-TA: 42.5 µM (stock concentration: 200 µM)
- Fe³⁺: 141 µM (stock concentration: 20 mM)

The antibody and each PEG-TA were prepared by dissolution in 10 mM HEPES, and Fe³⁺ was prepared by dissolution in ultrapure water.

The anti-NPC antibody solution, PEG₁₀ₖ-TA solution, and Fe³⁺ solution were mixed by pipetting. In doing so, the molar ratio of PEG-TA/antibody was set to 25. In addition, the molar ratio of iron ions was set to 3.3 equivalents relative to TA in PEG-TA.

### <Observation by Confocal Microscopy>

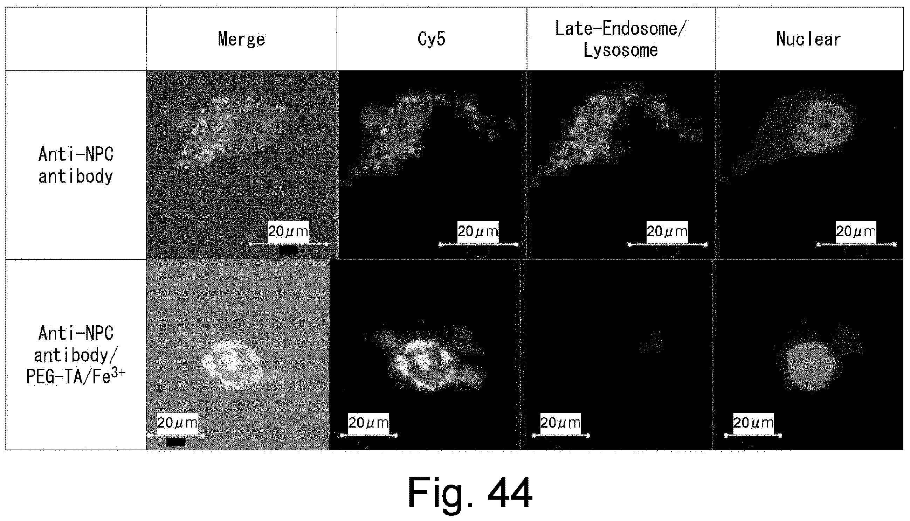

4T1 cells were seeded in 35 mm² glass based dishes at 5.0 × 10⁴ cells/dish and precultured at 37°C under 5% CO₂ for 24 hours. 100 µL of the above antibody sample solution and 100 µL of RPMI medium were added to each dish, followed by incubation for 48 hours. After washing with 1 mL of D-PBS(-), 1 mL of a 100 nM LysoTracker (registered trademark) Red DND-99/ (D-PBS(-) :RPMI = 1:9) solution was added, followed by incubation for 30 minutes. After washing with 1 mL of D-PBS(-), incubation was performed with a 4% paraformaldehyde/D-PBS(-) solution for 4 minutes. After washing with 1 mL of D-PBS(-), 1 mL of a 5.0 µg/mL Hoechst/D-PBS(-) solution was added, followed by incubation for 5 minutes. After washing twice with 1 mL of D-PBS(-), 2 mL of RPMI was added, and observation was performed using a confocal microscope. Figure 44 shows the captured microscopic images.

With the anti-NPC antibody alone, fluorescence derived from the antibody was colocalized with lysosomes, and no colocalization of the anti-NPC antibody with the nucleus was observed. In contrast, with the anti-NPC antibody/PEG₁₀ₖ-TA/Fe³⁺ MPN complex, the anti-NPC antibody was observed to be colocalized with the nucleus. These results indicate that the anti-NPC antibody/PEG₁₀ₖ-TA/Fe³⁺ MPN complex can translocate into the cytoplasm and target the nucleus.

### [Experimental Example 8]

### <Functional Evaluation of RNP-Loaded MPN Complex in Cultured Cells>

### (8.1 Overview)

A complex using Luc gene-editing RNP was introduced into HeLa-Luc cells, and toxicity evaluation by CCK assay and measurement of knockout efficiency based on luciferase luminescence intensity were performed.

### (8.2 Reagents and Cell Lines)

- Cas9 Nuclease protein NLS (Cas9) (NIPPON GENE)
- Luciferase knock gene knockout sgRNA (sgRNA) (GenScript)
- Iron(III) chloride hexahydrate (FeCl₃·6H₂O) (Fe³⁺) (FUJIFILM Wako Pure Chemical Corporation)
- PEG₁₀ₖ-TA (Mn = 11,700)
- PEG₅ₖ-TA (Mn = 6,700)
- D-PBS(-) (FUJIFILM Wako Pure Chemical Corporation)
- Luciferase Assay System (Promega)
- Cell Culture Lysis 5x Reagent (Promega)
- Cell Counting Kit-8 (Dojindo Laboratories)
- Roswell Park Memorial Institute medium (RPMI) (Sigma-Aldrich)
- Fetal bovine serum (FBS) (Biosera)
- Trypsin-EDTA solution (Sigma-Aldrich)
- Penicillin/streptomycin (Sigma-Aldrich)
- HeLa-Luc cells (aneuploid epithelial-like cell line stably expressing luciferase) (American Type Culture Collection, ATCC)
- ProDeliverIn (OZ Biosciences)

### (8.3 Measurement Instruments)

- Glomax 96 microplate luminometer (Promega)
- Multi-plate reader (Spark) (Tecan)

### (8.4 Cytotoxicity Evaluation)

### <Final Concentrations of Cas9-sgRNA, PEG₁₀ₖ-TA, and FeCl₃·6H₂O>

- Cas9: 90 nM
- sgRNA: 90 nM
- PEG₁₀ₖ-TA (Mn = 11,700): 9 µM
- Fe³⁺: 30 µM

Cas9-sgRNA and PEG₁₀ₖ-TA were prepared by dissolution in D-PBS(-). Fe³⁺ was prepared by dissolution in pure water.

Cas9 and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, PEG₁₀ₖ-TA was added, followed by standing at room temperature for 15 minutes to prepare an RNP/PEG₁₀ₖ-TA solution. Fe³⁺ was further added, followed by standing at room temperature for 15 minutes to prepare an RNP/PEG₁₀ₖ-TA/Fe³⁺ MPN complex solution. In addition, as a positive control, ProDeliverIn, which is a cationic reagent, was mixed at a ratio of ProDeliverIn/RNP = 1 µg/1 µL to prepare an RNP/ProDeliverIn solution.

### <Cytotoxicity Evaluation by CCK Assay>

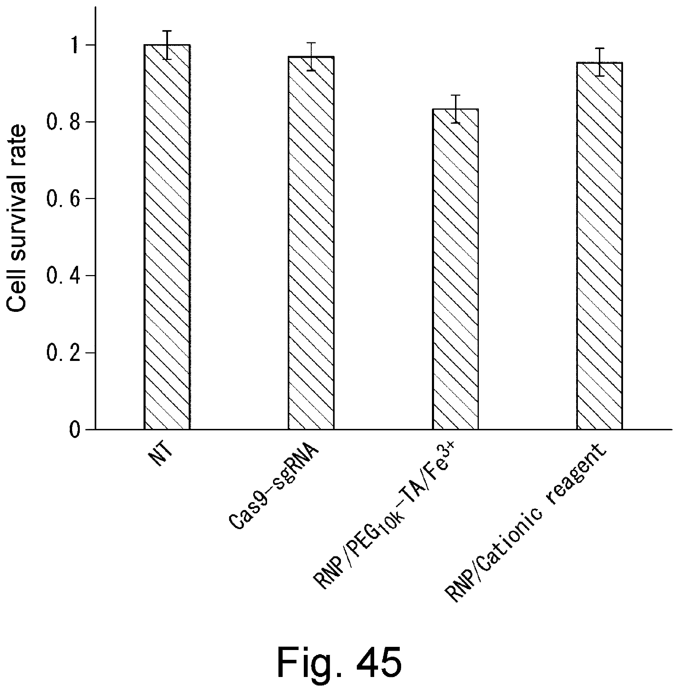

HeLa-Luc cells were seeded in a 96-well plate at 3.0 × 10³ cells/plate and incubated at 37°C under 5% CO₂ for 24 hours. 100 µL/well each of the above RNP solution, RNP/PEG₁₀ₖ-TA solution, RNP/PEG₁₀ₖ-TA/Fe³⁺ MPN complex solution, and RNP/ProDeliverIn solution were added to each well, followed by incubation for 72 hours. After washing with 150 µL/well of D-PBS(-), 110 µL/well of a CCK measurement solution prepared by mixing 10 µL of CCK and 100 µL of PBS(-) was added, followed by incubation for 1 hour. Thereafter, the absorbance at 460 nm was measured using a plate reader to evaluate cytotoxicity. Figure 45 is a graph showing the results of measuring cell survival rate by a CCK assay.

In the group to which the RNP/PEG₁₀ₖ-TA/Fe³⁺ MPN complex was added, no significant decrease in cell survival rate was observed compared with NT (untreated cells), and no marked cytotoxicity was confirmed.

### (8.5. Evaluation of Knockout Efficiency)

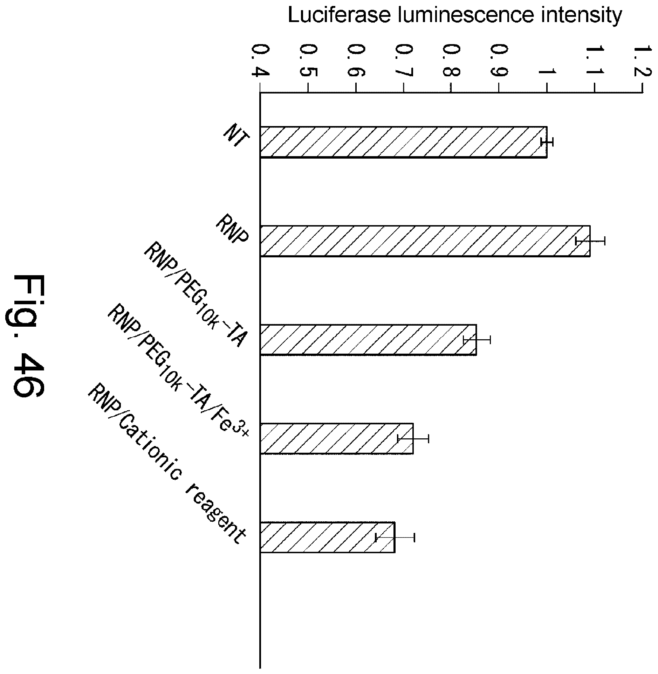

After cytotoxicity evaluation, the medium was collected, and after washing with D-PBS(-), 50 µL of Cell Culture Lysis was added to each well, followed by incubation for 15 minutes. Thereafter, 20 µL of the lysate was transferred to a dish for fluorescence measurement, to which luciferin was added, and the knockout efficiency of the luciferase gene was evaluated by measuring luciferase luminescence intensity using a plate reader. Figure 46 is a graph showing the results of measuring luciferase luminescence intensity.

In cells to which the RNP/PEG₁₀ₖ-TA/Fe³⁺ MPN complex solution was added, a marked decrease in luciferase luminescence intensity was observed compared with untreated cells and cells administered with RNP alone or RNP/PEG₁₀ₖ-TA. These results confirmed that knockout efficiency was improved by forming the RNP into an MPN complex. In addition, the RNP/PEG₁₀ₖ-TA/Fe³⁺ MPN complex exhibited gene knockout efficiency comparable to that obtained using the commercially available product ProDeliverIn.

### [Experimental Example 9]

### <Effects on Subcutaneous Tumor Mouse Model (In Vivo Kinetics, Intratumoral Kinetics, and Antitumor Effects)>

### (9.1 Overview)

The function of the antibody/PEG-TA/Fe³⁺ complex was evaluated in a 4T1-Luc (mouse colon cancer cell) subcutaneous tumor mouse model. Specifically, *in vivo* kinetics, intratumoral kinetics, and antitumor effects upon loading a therapeutic antibody were evaluated.

### (9.2 Reagents, Cells, and Animals)

- Cy5-labeled antibody (antibody)
- Alexa Fluor^{(R)} 647 anti-Nuclear Pore Complex Proteins Antibody (anti-NPC antibody) (BioLegend)
- Mouse Anti-S100A4 Recombinant Antibody (TAB-0136CL; hereinafter, anti-S100A4 antibody) (Creative Biolabs)
- PEG₁₀ₖ-TA (Mn = 11,701)
- PEG₅ₖ-TA (Mn = 6,701)
- Tannic acid (Mw: 1,701) (FUJIFILM Wako Pure Chemical Corporation)
- 4T1-Luc cells (mouse breast cancer-derived cell line) (American Type Culture Collection, ATCC)
- BALB/c mice (Charles River)

### (9.3 Instruments and Equipment)

- Countess (Thermo Fisher Scientific)
- Multi-beads shocker (Yasui Kikai)
- Multi-plate reader (Spark) (Tecan)
- *In vivo* imaging system (PerkinElmer)
- Cryostat (Leica Biosystems)
- Fluorescence microscope (BZX-700) (Keyence)

### (9.4 In Vivo Kinetics of Antibody-Loaded MPN Complexes)

In order to evaluate blood retention and tumor accumulation of the antibody/PEG-TA/Fe³⁺ MPN complex, an antibody sample was intravenously injected into a 4T1-Luc subcutaneous tumor mouse model, and after a predetermined period of time, the antibody contents in blood and tumor were measured by fluorescence intensity measurement. In the present Experimental Example, antibody alone, antibody/PEG₁₀ₖ-TA, and the antibody/TA/Fe³⁺ MPN complex were evaluated as comparators for the antibody/PEG₁₀ₖ-TA/Fe³⁺ MPN complex.

### <Final Concentrations of Antibody and PEG-TA>

- Antibody: 3.3 µM
- PEG₁₀ₖ-TA: 82.5 µM (stock concentration: 200 µM)
- Fe³⁺: 272 µM (stock concentration: 20 mM)

The antibody and PEG₁₀ₖ-TA were prepared by dissolution in 10 mM HEPES, and Fe³⁺ was prepared by dissolution in ultrapure water.

The antibody solution, PEG₁₀ₖ-TA solution, and Fe³⁺ solution were mixed by pipetting. In doing so, the molar ratio of PEG₁₀ₖ-TA/antibody was set to 25. In addition, the molar ratio of iron ions was set to 3.3 equivalents relative to TA in PEG-TA.

### <Preparation of 4T1-Luc Subcutaneous Tumor Mouse Model>

100 µL of a 4T1-Luc cell suspension (4.0 × 10⁷ cells/mL) was subcutaneously injected into BALB/c mice.

### <Evaluation of In Vivo Kinetics>

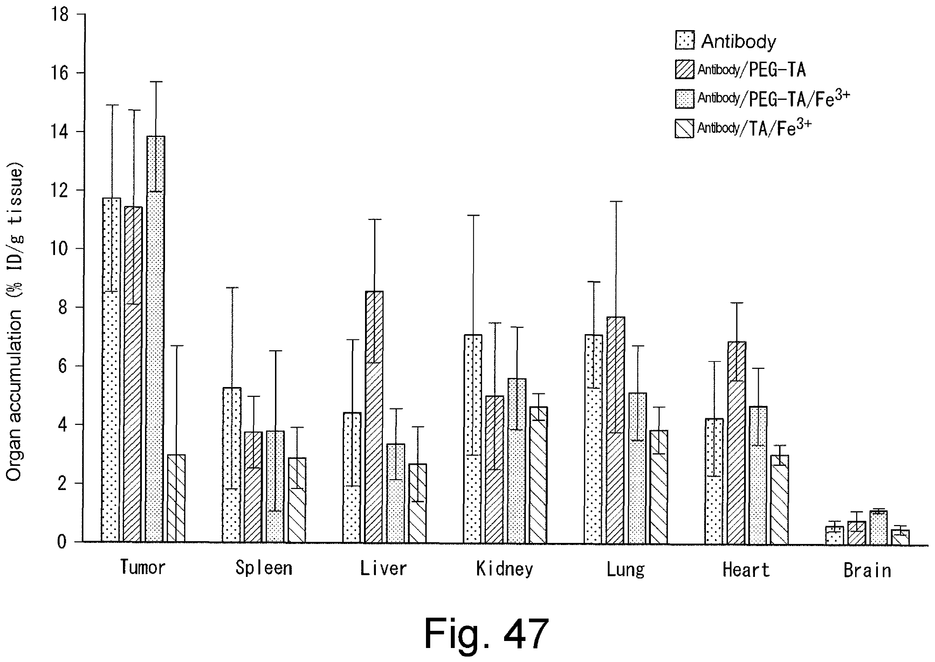

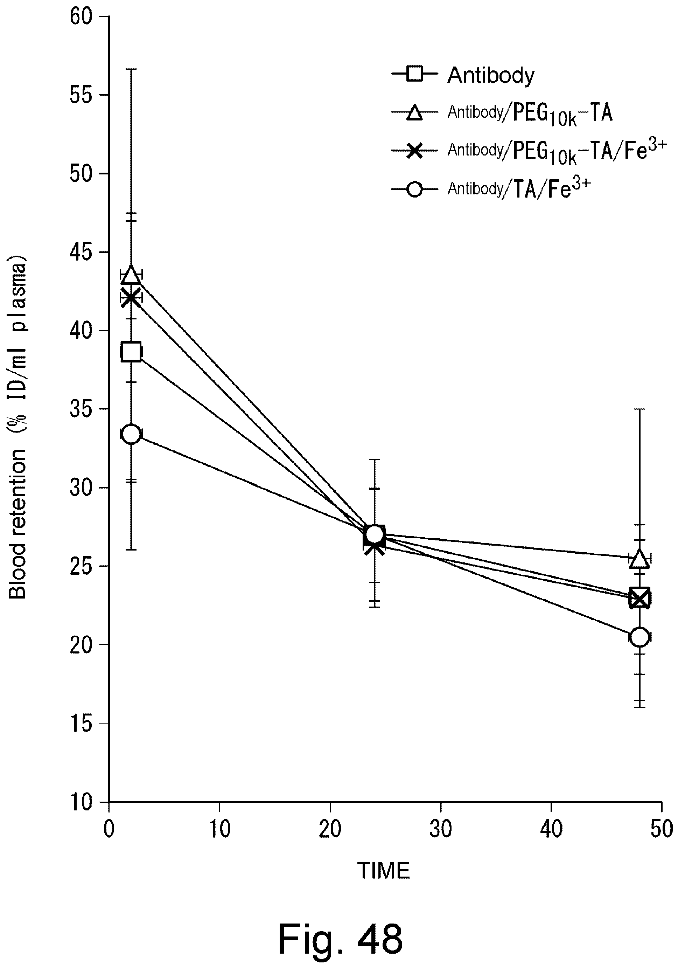

To model mice whose tumor size had reached approximately 200 mm³, 100 µL of the above prepared sample solution was administered via the tail vein. Forty-eight hours after sample administration, the mice were dissected, blood and various organs were collected, and Passive Lysis Buffer was added thereto in an amount corresponding to five times the weight, followed by homogenization using a multi-beads shocker. Blood was also collected from the tail vein after 2 hours and after 24 hours. Thereafter, centrifugation was performed at 10,000 × g for 5 minutes at 4°C, and 50 µL of the supernatant was transferred to a 96-well plate, after which the fluorescence intensity of the antibody was measured using a multi-plate reader to evaluate *in vivo* kinetics. Figure 47 is a graph showing the results of evaluation of organ accumulation. Figure 48 is a graph showing the results of evaluation of blood retention.

The *in vivo* kinetics of the antibody/PEG₁₀ₖ-TA/Fe³⁺ MPN complex exhibited behavior comparable to the *in vivo* kinetics of the antibody alone, which has high blood retention and tumor accumulation. The antibody/PEG₁₀ₖ-TA/Fe³⁺ MPN complex showed a tendency toward improved tumor accumulation compared with the antibody alone. The blood retention and tumor accumulation of the antibody/TA/Fe³⁺ MPN complex, which served as a comparator, showed lower values than those of the antibody/PEG-TA/Fe³⁺ MPN complex. These results indicate that PEG is effective for improving blood retention and tumor accumulation.

### (9.5 Intratumoral Behavior of Anti-NPC Antibody MPN Complex)

In order to evaluate the intratumoral behavior of the antibody MPN complex, an anti-NPC antibody/PEG-TA/Fe³⁺ MPN complex sample was intravenously injected into a 4T1-Luc subcutaneous tumor mouse model, and after a predetermined period of time, the tumor was excised, followed by section observation to observe the intratumoral behavior of the antibody. In the present Experiment, antibody alone, antibody/PEG-TA, and the antibody/TA/Fe³⁺ MPN complex were evaluated as comparators for the antibody/PEG-TA/Fe³⁺ MPN complex.

### <Final Concentrations of Antibody, PEG₁₀ₖ-TA, TA, and Fe³⁺>

- Anti-NPC antibody: 1.7 µM
- PEG₁₀ₖ-TA: 41.7 µM (stock concentration: 200 µM)
- Fe³⁺: 138 µM (stock concentration: 20 mM)
- TA: 41.7 µM (stock concentration: 200 µM)

The antibody, PEG₁₀ₖ-TA, and TA were prepared by dissolution in PBS(-), and Fe³⁺ was prepared by dissolution in ultrapure water.

The antibody solution, PEG₁₀ₖ-TA solution, TA solution, and Fe³⁺ solution were mixed by pipetting. In doing so, the molar ratio of PEG₁₀ₖ-TA/antibody and TA/antibody were set to 25. In addition, the molar ratio of iron ions was set to 3.3 equivalents relative to TA in

### PEG-TA.

### <Preparation of 4T1-Luc Subcutaneous Tumor Mouse Model>

100 µL of a 4T1-Luc cell suspension (4.0 × 10⁷ cells/mL) was subcutaneously injected into BALB/c mice.

### <Evaluation of In Vivo Kinetics>

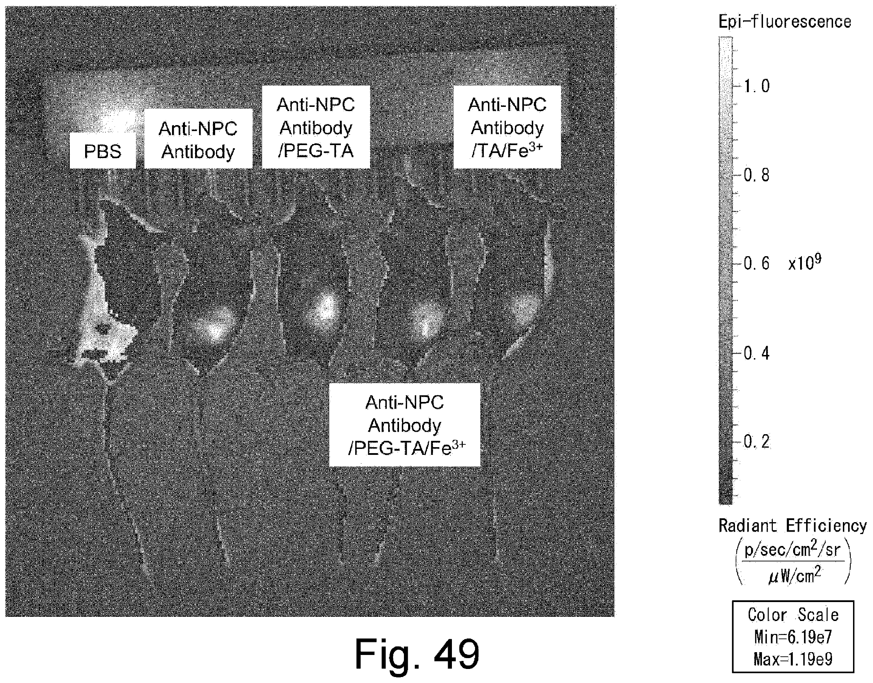

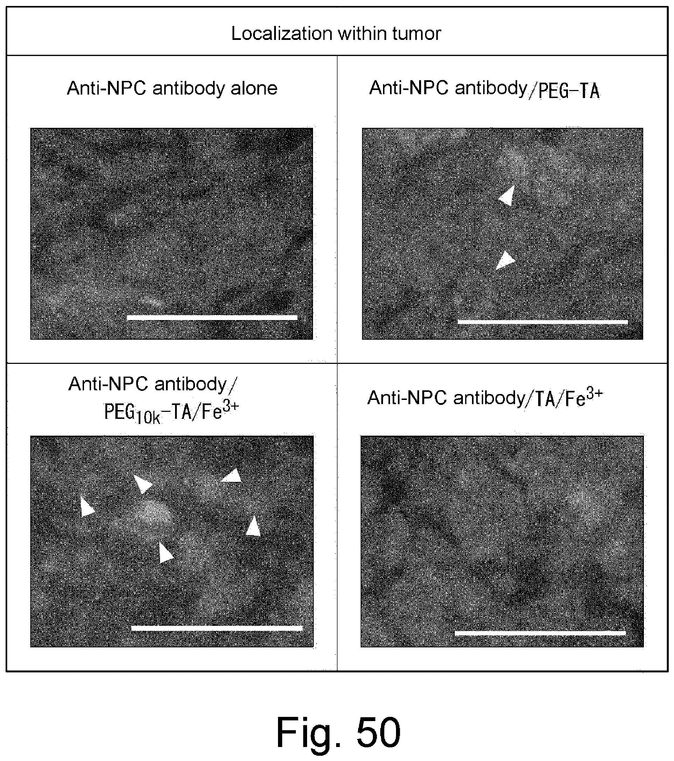

To model mice whose tumor size had reached approximately 200 mm³, 100 µL of the above sample solution was administered via the tail vein. Forty-eight hours after sample administration, *in vivo* fluorescence imaging (IVIS) was performed. Figure 49 is an image showing the results of *in vivo* fluorescence imaging. Immediately after *in vivo* fluorescence imaging, the mice were dissected and the tumors were excised. The tumors were sectioned into 6 µm slices using a cryostat and then fixed with 4% paraformaldehyde. The sections were then stained with Hoechst and observed using a fluorescence microscope. Figure 50 shows fluorescence microscope images of the sections. In Figure 50, the arrowheads indicate regions in the nucleus where antibody-derived fluorescence was detected.

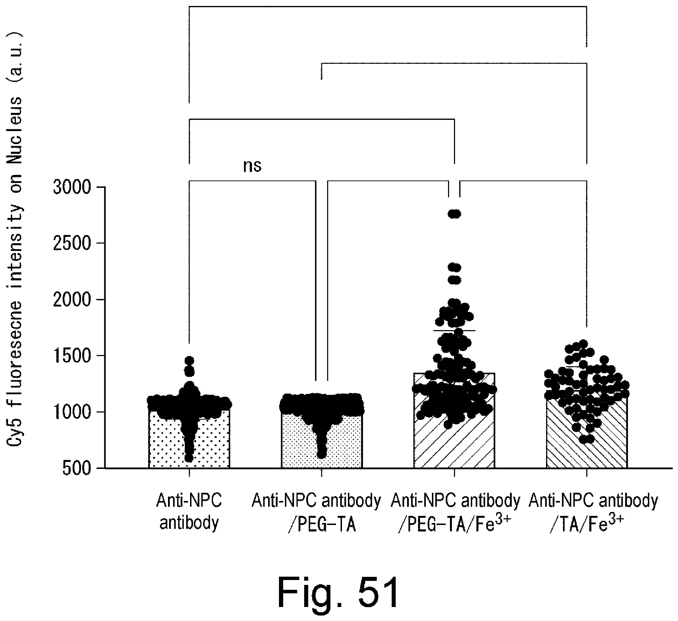

Figure 51 is a graph showing fluorescence intensity derived from the antibody observed in the nucleus.

From Figure 49, it was confirmed that all anti-NPC antibody samples accumulated in the tumor. Among them, the fluorescence intensity of the anti-NPC antibody/TA/Fe³⁺ MPN complex was the lowest, which was consistent with the quantitative results of tumor accumulation shown in Figure 47. From Figures 50 and 51, the anti-NPC antibody/PEG₁₀ₖ-TA/Fe³⁺ MPN complex exhibited higher fluorescence intensity of the antibody in the nucleus than the other samples, indicating that translocation into the cytoplasm and into the nucleus occurred sufficiently even *in vivo*. In addition, although the anti-NPC antibody/TA/Fe³⁺ MPN complex exhibited lower tumor accumulation than the anti-NPC antibody alone and the anti-NPC antibody/PEG-TA, it exhibited a higher colocalization efficiency with the nucleus. These results indicated that TA and Fe³⁺ are extremely important for translocation into the cytoplasm.

### (9.6 Antitumor Effects of MPN Complex Loaded with Therapeutic Antibody)

In order to demonstrate the function of the antibody MPN complex, the antitumor effect of an MPN complex loaded with an anti-S100A4 antibody that activates the tumor suppressor gene (p53) was evaluated. An anti-S100A4 antibody/PEG-TA/Fe³⁺ MPN complex sample was intravenously injected into a 4T1-Luc subcutaneous tumor mouse model, and the tumor size was measured. In the present Experimental Example, in addition to antibody/PEG-TA/Fe³⁺ MPN complexes using PEG-TA having PEG molecular weights of 10k and 5k, anti-S100A4 antibody alone, anti-S100A4 antibody/PEG₁₀ₖ-TA, control antibody/PEG₁₀ₖ-TA, and an anti-S100A4 antibody/TA/Fe³⁺ MPN complex were evaluated as comparators.

### <Final Concentrations of Antibody, PEG-TA, TA, and Fe³⁺>

- Anti-S100A4 antibody: 3.3 µM
- PEG₁₀ₖ-TA: 82.5 µM (stock concentration: 200 µM)
- PEG₅ₖ-TA: 82.5 µM (stock concentration: 200 µM)
- Fe³⁺: 272 µM (stock concentration: 20 mM)
- TA: 82.5 µM (stock concentration: 200 µM)

The antibody, PEG-TA, and TA were prepared by dissolution in PBS(-), and Fe³⁺ was prepared by dissolution in ultrapure water.

The antibody solution, the two PEG-TA solutions, the TA solution, and the Fe³⁺ solution were mixed by pipetting. In doing so, the molar ratio of PEG-TA/antibody and TA/antibody were set to 25. In addition, the molar ratio of iron ions was set to 3.3 equivalents relative to TA in PEG-TA.

### <Preparation of 4T1-Luc Subcutaneous Tumor Mouse Model>

100 µL of a 4T1-Luc cell suspension (5.0 × 10⁷ cells/mL) was subcutaneously injected into BALB/c mice.

### <Evaluation of In Vivo Kinetics>

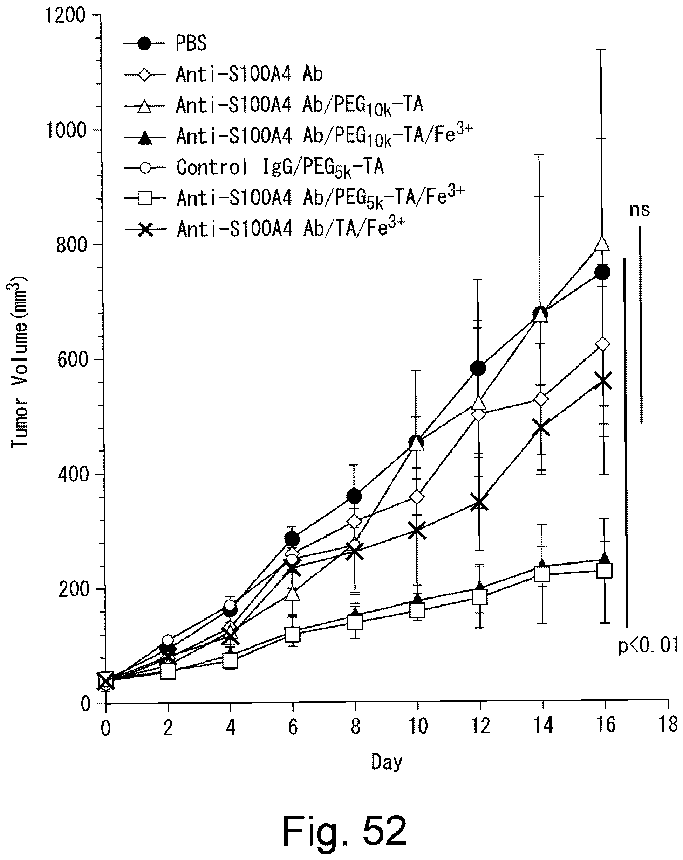

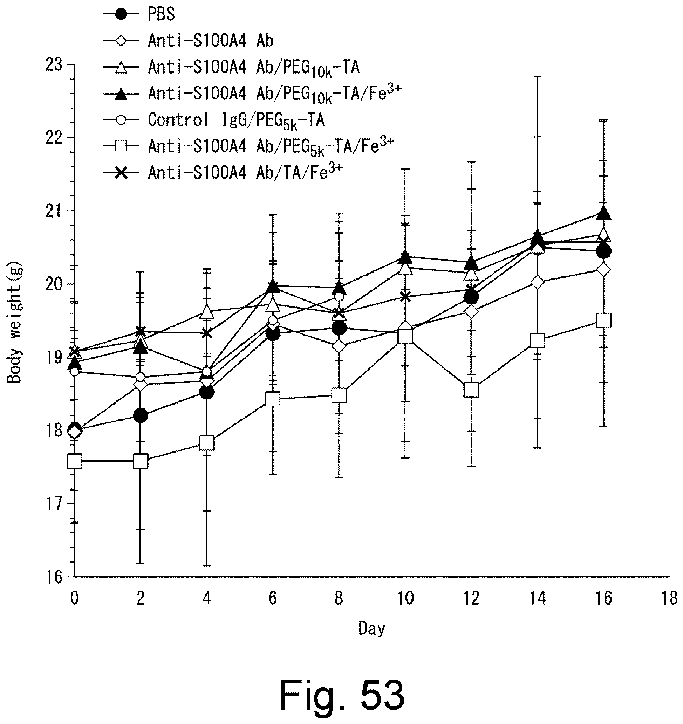

Using the time point at which the tumor size of the model mice reached approximately 30 mm³ as Day 0, 100 µL of the above sample solution (anti-S100A4 antibody: 50 µg/mouse) was administered via the tail vein on Days 0, 4, and 8. In addition, a group administered with D-PBS(-) was also provided for comparison. Tumor size was evaluated by measuring the tumor size using calipers every two days until Day 16. At the same time, the body weight of the mice was measured, and adverse events were also monitored. Figure 52 shows changes in tumor size over time, and Figure 53 shows changes in body weight over time. Tumor size was calculated according to the following equation (F1). $V = \left({W}^{2}\times L\right) / 2 \left(\begin{matrix}V : volume , W : short diameter , and L : \\ long diameter\end{matrix}\right)$ (F1)

Figure 52 showed that the tumor sizes in both groups administered with the anti-S100A4 antibody/PEG-TA/Fe³⁺ MPN complex were significantly suppressed compared with the tumor sizes in the groups administered with other samples and with PBS(-). In addition, because the MPN complex loaded with the control antibody exhibited a tumor growth curve similar to that of the group administered with PBS(-), it was confirmed that the antitumor effect was exhibited as a result of the anti-S100A4 antibody functioning within the tumor. In addition, Figure 53 showed that all sample-administered groups exhibited similar changes in body weight.

### [Experimental Example 10]

### <Effects on a Subcutaneous Tumor Mouse Model (Evaluation of Knockout Efficiency)>

### (10.1 Overview)

Evaluation of the knockout efficiency of the RNP/PEG₁₀ₖ-TA/Fe³⁺ MPN complex was performed in a 4T1-Luc subcutaneous tumor mouse model.

### (10.2 Reagents, Cells, and Animals)

- Cas9 Nuclease protein NLS (Cas9) (NIPPON GENE)
- sgRNA (Luciferase-2) (GenScript)
- Iron(III) chloride hexahydrate (FeCl₃·6H₂O) (Fe³⁺) (FUJIFILM Wako Pure Chemical Corporation)
- PEG₁₀ₖ-TA (Mn = 11,700)
- 4T1-Luc cells (mouse breast cancer-derived cell line stably expressing luciferase) (American Type Culture Collection, ATCC)
- BALB/c mice (Charles River)
- Passive Lysis 5× Buffer (Promega)
- Micro BCA^{™} Protein Assay Kit (Thermo Fisher Scientific)

### (10.3 Measurement Instruments)

- Glomax 96 microplate luminometer (Promega)
- Multi-plate reader (Spark) (Tecan)
- Multi-beads shocker (Yasui Kikai)

### (10.4 Confirmation of In Vivo Function of RNP/PEG₁₀ₖ-TA/Fe³⁺ MPN Complex)

In order to evaluate the gene knockout efficiency of the RNP/PEG₁₀ₖ-TA/Fe³⁺ MPN complex, the RNP/PEG₁₀ₖ-TA/Fe³⁺ MPN complex was intravenously injected into a 4T1-Luc subcutaneous tumor mouse model, and the luciferase luminescence intensity of the tumor was measured after a predetermined period of time.

### <Final Concentrations of Cas9-sgRNA, PEG₁₀ₖ-TA, and Fe³⁺>

- Cas9 Nuclease protein NLS: 1.0 µM
- sgRNA: 1.0 µM
- PEG₁₀ₖ-TA (Mn = 11,700): 100 µM
- Fe³⁺: 333 µM

Cas9-sgRNA and PEG₁₀ₖ-TA were prepared by dissolution in D-PBS(-). Fe³⁺ was prepared by dissolution in pure water.

Cas9 and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, PEG₁₀ₖ-TA was added, followed by standing at room temperature for 15 minutes to prepare an RNP/PEG₁₀ₖ-TA solution. Fe³⁺ was further added, followed by standing at room temperature for 15 minutes to prepare an RNP/PEG₁₀ₖ-TA/Fe³⁺ MPN complex solution.

### <Preparation of 4T1-Luc Subcutaneous Tumor Mouse Model>

100 µL of a 4T1-Luc cell suspension (1.0 × 10⁶ cells/mL) was subcutaneously injected into BALB/c mice.

### <Evaluation of Knockout Efficiency>

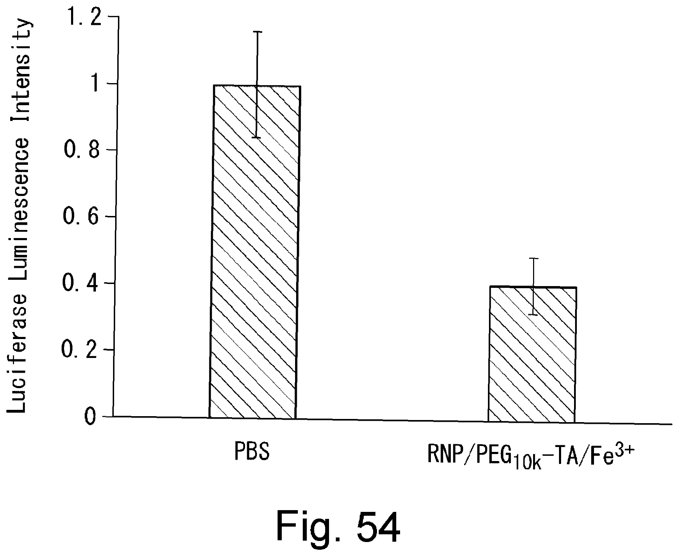

To the 4T1-Luc subcutaneous tumor mouse model, 200 µL of the above prepared solution and D-PBS(-) were administered via the tail vein. After 8 days had elapsed from sample administration, the tumors were collected, Passive Lysis Buffer was added in an amount corresponding to three times the weight, and disruption was performed using a multi-beads shocker at 1,500 rpm for 30 seconds, 8 times. Thereafter, the suspension was centrifuged at 10,000 rpm for 5 minutes, and the supernatant was collected. Thereafter, the supernatant was diluted 50-fold with PBS(-), and BCA assay standard reagent and the sample were mixed at a sample:standard reagent ratio of 1:15, followed by incubation at 37°C for 20 minutes. The absorbance at 562 nm was measured using a plate reader, and the protein amount in each sample was calculated from a calibration curve. Furthermore, 10 µL of the supernatant was transferred to a plate for fluorescence measurement, to which 100 µL of luciferin was added, and the knockout efficiency of the luciferase gene was evaluated by measuring luciferase luminescence intensity using a plate reader. Figure 54 is a graph showing the results of measuring luciferase luminescence intensity.

In mice administered with the RNP/PEG₁₀ₖ-TA/Fe³⁺ MPN complex, a decrease in luciferase luminescence intensity was observed compared with mice administered with PBS(-), confirming that knockout of the luciferase gene had been achieved.

### [Summary]

To improve the activity of bioactive proteins, a protein delivery system driven by complex formation between a polyphenol-introduced polymer and metal ions was constructed in the present invention. The antibody/PEG-TA/Fe³⁺ MPN complex loaded with an antibody promoted cytoplasmic translocation of the antibody and enhanced the antibody-derived antitumor effect. In addition, the RNP-loaded MPN complex loaded with Cas9/sgRNA RNP enhanced gene knockout efficiency. Furthermore, this RNP-loaded MPN complex successfully achieved gene knockout in tumors even *in vivo.*

### [Experimental Example 11]

### <Physical Property Evaluation of PEG-TA>

### (11.1 Overview)

The structures of PEG₅ₖ-TA (Mₙ = 6,700) and PEG₁₀ₖ-TA (Mₙ = 11,700) were evaluated. Using a JASCO V-650 spectrophotometer, UV absorption due to the structure was confirmed, and the presence of specific functional groups and the like was confirmed by FT-IR measurement.

### (11.2 Reagents)

Unless otherwise specified, commercially available reagents and solvents were used as purchased.
- PEG₅ₖ-TA (Mₙ = 6,700)
- PEG₁₀ₖ-TA (Mₙ = 11,700)
- D-PBS(-) (FUJIFILM Wako Pure Chemical Corporation)
- Tannic acid (Mw: 1,701) (FUJIFILM Wako Pure Chemical Corporation)
- TA (quinone)
- α-Methoxy-ω-amino-poly(ethylene glycol) (PEG-NH₂) [Mn: 5k] (NOF Corporation)
- α-Methoxy-ω-amino-poly(ethylene glycol) (PEG-NH₂) [Mn: 10k] (NOF Corporation)
- Sodium hydrogen carbonate (Tokyo Chemical Industry)

### (11.3 Measurement Instruments)

- JASCO V-650 spectrophotometer (JASCO)
- FT-IR: JASCO

### (11.4 Preparation of Oxidized TA)

### <Concentration of TA>

- TA: 0.26 mg/mL

TA was dissolved in 50 mM sodium hydrogen carbonate buffer (pH 8.5) in a beaker and stirred for 24 hours. Thereafter, the reaction solution transferred into a dialysis membrane (MWCO: 3,500 Da) was dialyzed for 24 hours against 2 L of pure water subjected to ultrasonication for 10 minutes and Ar bubbling for 5 minutes. The dialyzed reaction solution was passed through a 0.45 µm filter, and then the solution was freeze-dried to obtain TA (quinone).

### (11.5 Measurement of UV Spectra of TA, TA (Quinone), PEG₅ₖ-TA, and PEG₁₀ₖ-TA)

### <Final Concentrations of TA, TA (Quinone), PEG₅ₖ-TA, and PEG₁₀ₖ-TA>

- TA: 0.073 mg/mL
- TA (quinone): 0.073 mg/mL
- PEG₅ₖ-TA: 0.29 mg/mL
- PEG₁₀ₖ-TA: 0.5 mg/mL

Each was prepared by dissolution in D-PBS(-).

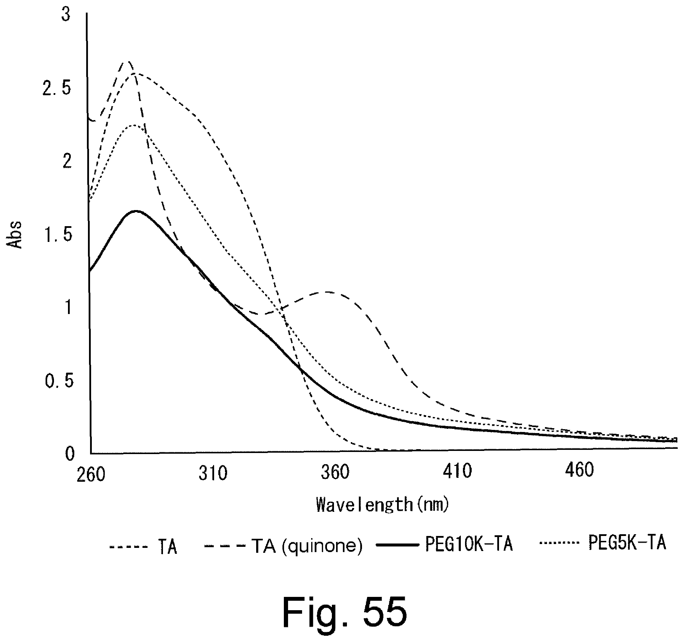

Using a JASCO V-650 spectrophotometer, the TA solution, TA (quinone) solution, PEG₅ₖ-TA solution, and PEG₁₀ₖ-TA solution were each measured, and UV absorption was confirmed. Figure 55 shows the respective measurement results.

The PEG₅ₖ-TA solution and the PEG₁₀ₖ-TA solution each exhibited UV absorption similar to that of TA, indicating that PEG₅ₖ-TA and PEG₁₀ₖ-TA contain the structure of TA. In addition, the UV absorption at wavelengths of 360 nm or greater observed in the TA (quinone) solution, PEG₅ₖ-TA solution, and PEG₁₀ₖ-TA solution suggested that a portion of the galloyl group structures of PEG₅ₖ-TA and PEG₁₀ₖ-TA had become quinone structures. Formula (3) below shows the chemical formula of tannic acid-modified polyethylene glycol in which a part of the galloyl group structure is in a quinone structure. In Formula (3) below, n represents the number of polyethylene glycol units.

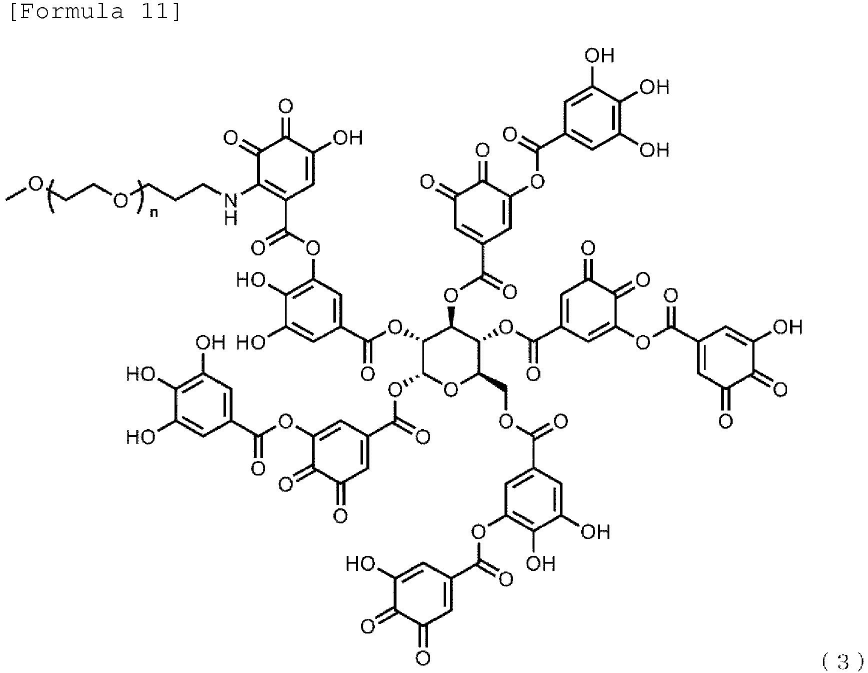

(11.6 Preparation of PEG-NH₂ (Mn: 10k) + TA and PEG-NH₂ (Mn: 5k) + TA)

### <Final Concentrations of TA, PEG-NH₂ (Mn: 10k), and PEG-NH₂ (Mn: 5k)>

- TA: 0.17 mg/mL
- PEG-NH₂ (Mn: 10k): 1 mg/mL
- PEG-NH₂ (Mn: 5k): 0.5 mg/mL

Each was prepared by dissolution in ultrapure water.

TA was dissolved in the same solution as PEG₁₀ₖ-NH₂ (Mn: 10k) or PEG₅ₖ-NH₂ (Mn: 5k). In doing so, the molar ratio of TA to PEG-NH₂ was set to 1:1 for both. Thereafter, the reaction solutions were freeze-dried to obtain PEG₁₀ₖ-NH₂ (Mn: 10k) + TA and PEG₃ₖ-NH₂ (Mn: 5k) + TA.

### (11.7 FT-IR Measurement)

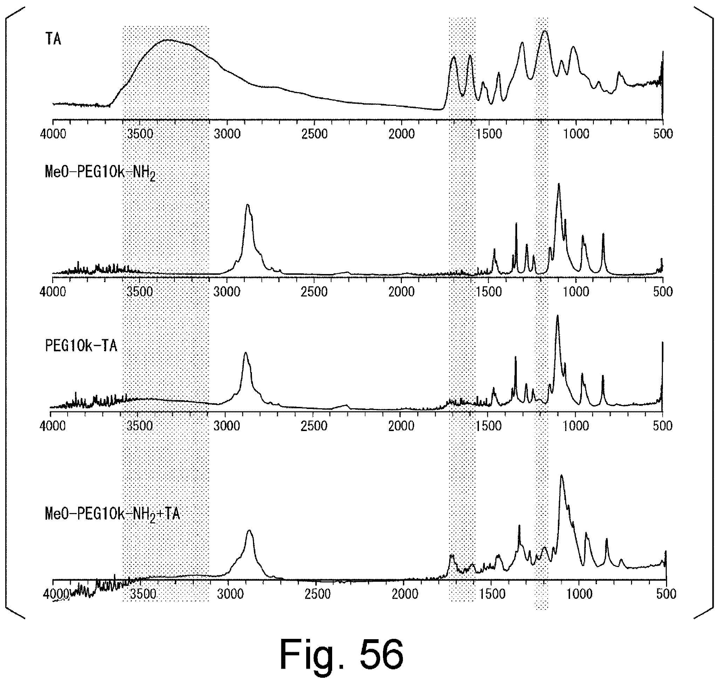

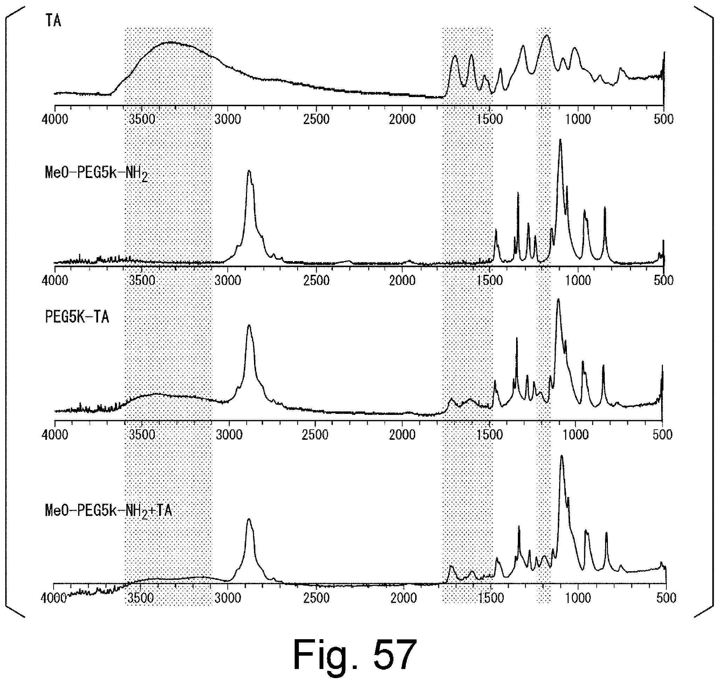

Using FT-IR, TA, MeO-PEG-NH₂ (Mn: 10k and 5k), PEG₁₀ₖ-TA, PEG₅ₖ-TA, and MeO-PEG-NH₂ (Mn: 10k and 5k) + TA were measured. Figures 56 and 57 show the respective measurement results.

PEG₁₀ₖ-TA and PEG₅ₖ-TA each exhibited peaks derived from TA (1,200 cm⁻¹, 1,600 to 1,800 cm⁻¹, and 3,100 to 3,600 cm⁻¹) and PEG (2,800 to 3,000 cm⁻¹), suggesting that both PEG-NH₂ and TA structures were present in PEG₁₀ₖ-TA and PEG₅ₖ-TA.

### [Experimental Example 12]

### <Synthesis of PEG-TA Introduced with Hepatic Parenchymal Cell-Targeting Molecule>

### (12.1. Overview)

To impart functionality to PEG-TA, PEG-TA into which a hepatic parenchymal cell-targeting molecule (N-acetylgalactosamine) had been introduced was synthesized. As shown in Scheme (5) below, α-GalNAc-PEG3-Thiol was introduced into ACA-PEG-NH₂, after which TA was introduced.

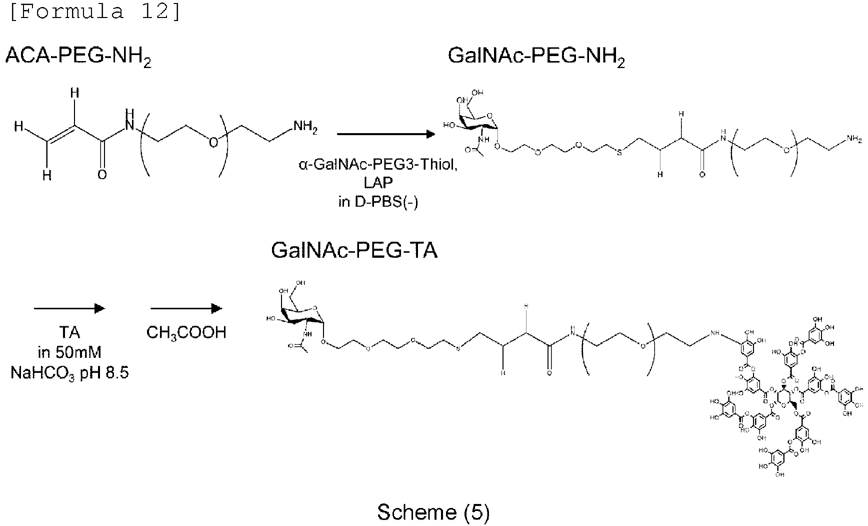

### (12.2. Reagents)

- ACA-PEG₁₀ₖ-NH₂ (HE010005-10K, Mn: 10K, Biochempeg Scientific Inc.)
- Tannic acid (Mw: 1,701, FUJIFILM Wako Pure Chemical Corporation)
- D-PBS(-) (FUJIFILM Wako Pure Chemical Corporation)
- α-GalNAc-PEG3-Thiol: Sussex Research
- Lithium phenyl(2,4,6-trimethylbenzoyl)phosphinate (LAP) (Sigma Aldrich)
- Acetic acid (CH₃COOH) (Nacalai Tesque) Sodium hydrogen carbonate (NaHCO₃) (FUJIFILM Wako Pure Chemical Corporation)

### (12.3. Measurement Instruments)

- Nuclear magnetic resonance (NMR) instrument (BRUKER AVANCE III 400 (400 MHz, BRUKER BioSpin))
- NanoDrop One nanospectrophotometer (Thermo Fisher Scientific)

### (12.4. Synthesis of GalNac-Introduced PEG-TA)

### <Synthesis of GalNac-PEG₁₀ₖ-TA>

### <Binding of GalNac to ACA-PEG₁₀ₖ-NH₂>

20 mg (0.05 mmol) of α-GalNAc-PEG₃-Thiol, 200 mg (0.025 mmol) of ACA-PEG₁₀ₖ-NH₂, and 15 mg (0.05 mmol) of LAP were weighed into a 30 mL beaker, and irradiated with 380 nm ultraviolet light at 20 W for 5 hours. Thereafter, the reaction solution transferred to a dialysis membrane (MWCO: 6 to 8 kDa) was dialyzed three times against water. The resulting solution was freeze-dried to obtain white solid GalNac-PEG₁₀ₖ-NH₂ in a yield of 200 mg.

### <Binding of TA to GalNac-PEG₁₀ₖ-NH₂>

52 mg (0.030 mmol) of TA was weighed into a 200 mL beaker and dissolved in 80 mL of 50 mM NaHCO₃ (pH 8.5). To this was added dropwise 100 mg (0.010 mmol) of GalNac-PEG₁₀ₖ-NH₂ dissolved in 40 mL of 50 mM NaHCO₃ (pH 8.5), followed by stirring at room temperature for 5 hours. Thereafter, the reaction solution transferred into a dialysis membrane (MWCO: 6 to 8 kDa) was dialyzed for 1 hour against 2 L of pure water subjected to ultrasonication for 10 minutes and Ar bubbling for 5 minutes.

### <Reduction of Side-Chain Ketone Groups of TA>

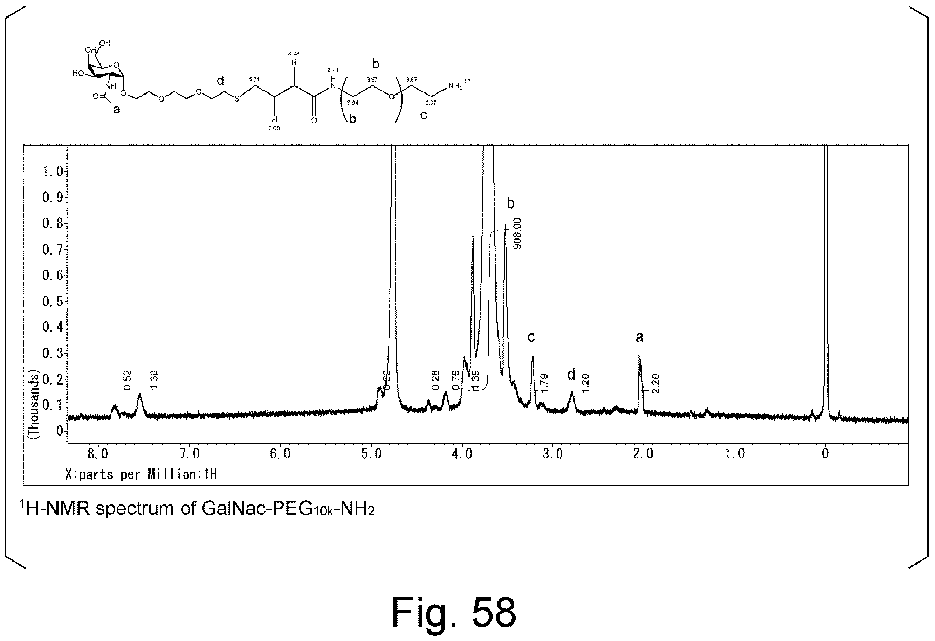

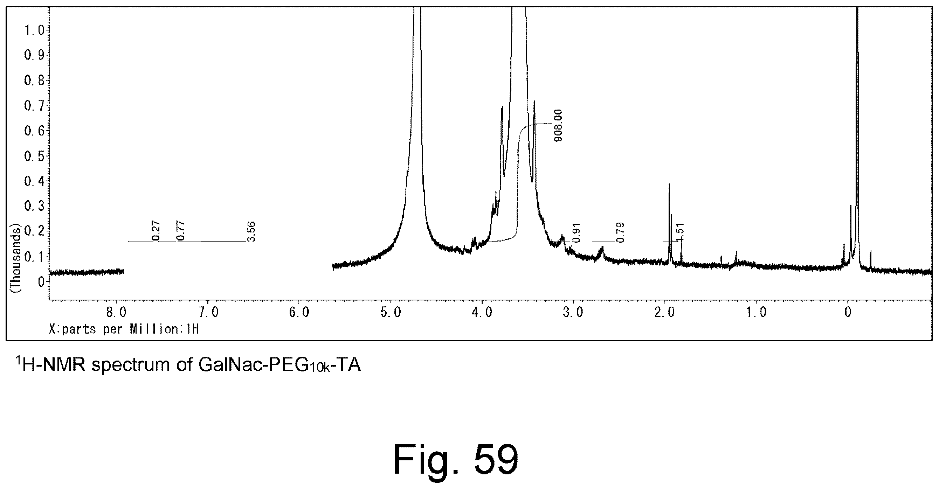

The reaction solution was transferred to a 200 mL beaker, and 2 mL of acetic acid was added dropwise followed by stirring for 5 minutes. Thereafter, the reaction solution transferred into a dialysis membrane (MWCO: 6 to 8 kDa) was dialyzed twice against 2 L of pure water subjected to ultrasonication for 10 minutes and Ar bubbling for 5 minutes. The resulting solution was freeze-dried to obtain yellow solid PEG₁₀ₖ-TA in a yield of 68 mg. Figure 58 shows the ¹H-NMR spectrum of GalNac-PEG₁₀ₖ dissolved in D₂O, and Figure 59 shows the ¹H-NMR spectrum of GalNac-PEG₁₀ₖ-TA dissolved in d-DMSO.

### (Analysis)

### <Binding of GalNac to ACA-PEG₁₀ₖ-NH₂>

In the ¹H-NMR spectrum, an initiator-derived peak (3.3 to 4.0 ppm) and a GalNac-derived peak (2.0 ppm) were confirmed. The GalNac introduction ratio was calculated to be 90%.

### <Binding of TA to GalNac-PEG₁₀ₖ-NH₂>

From the ¹H-NMR spectrum of GalNac-PEG-NH₂, a peak derived from TA was confirmed, confirming quantitative introduction of TA.

### [Experimental Example 13]

### <Synthesis of PEG-TA Imparted with Cationic Molecule>

### (13.1. Overview)

To impart functionality to PEG-TA, PEG-TA into which the ethylenediamine molecules triethylenetetramine (TET), tetraethylenepentamine (TEP), and spermine (S) had been introduced was synthesized. As shown in Schemes (6) to (11) below, an ethylenediamine-based molecule was introduced into MeO-PEG₁₀ₖ-COOH, followed by binding of TA.

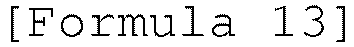

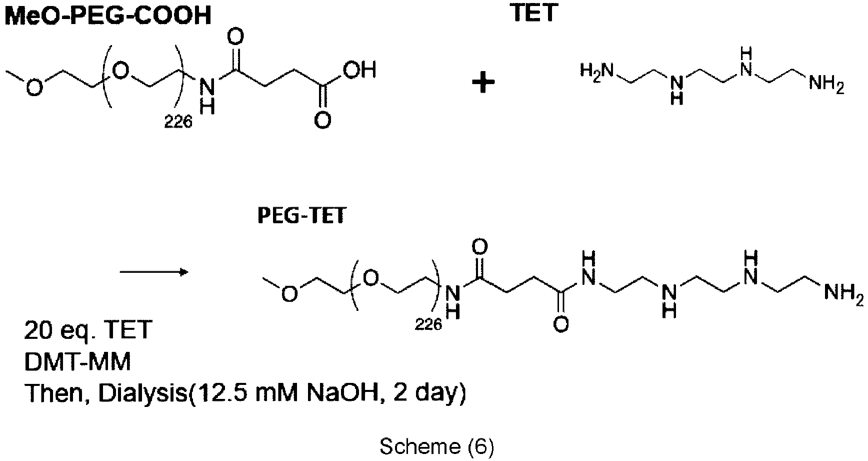

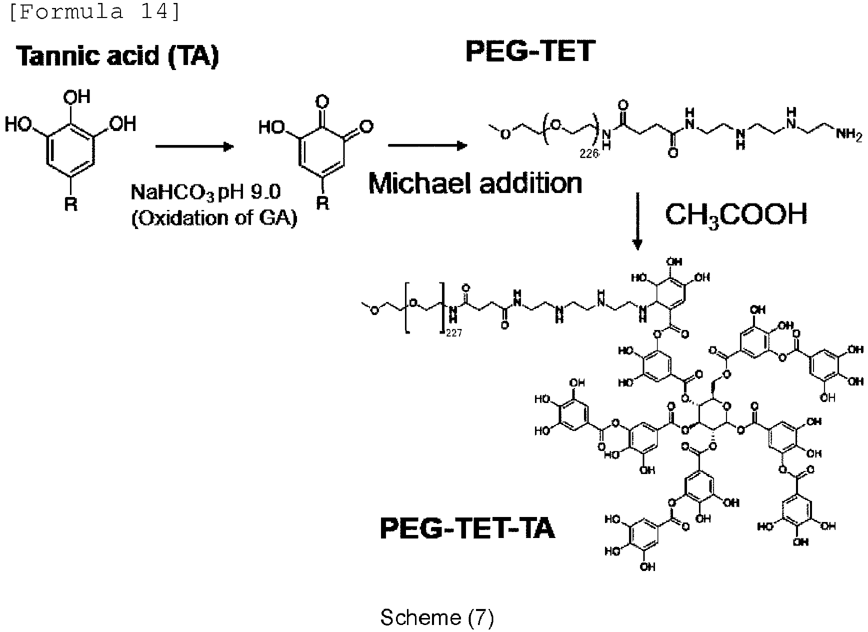

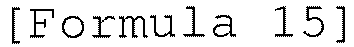

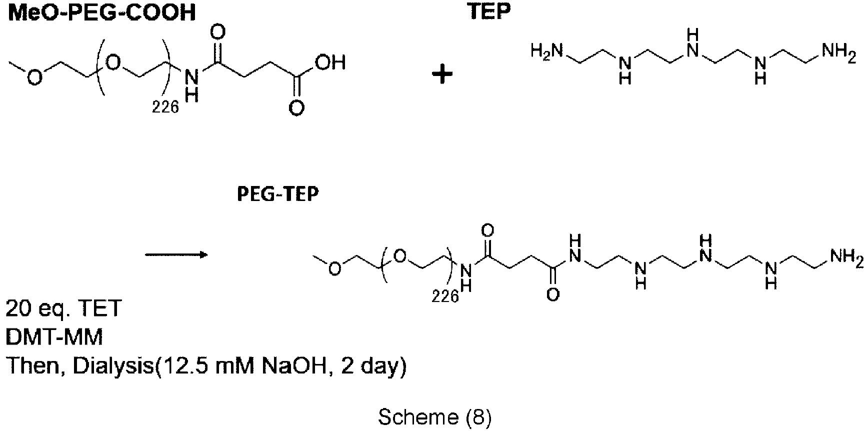

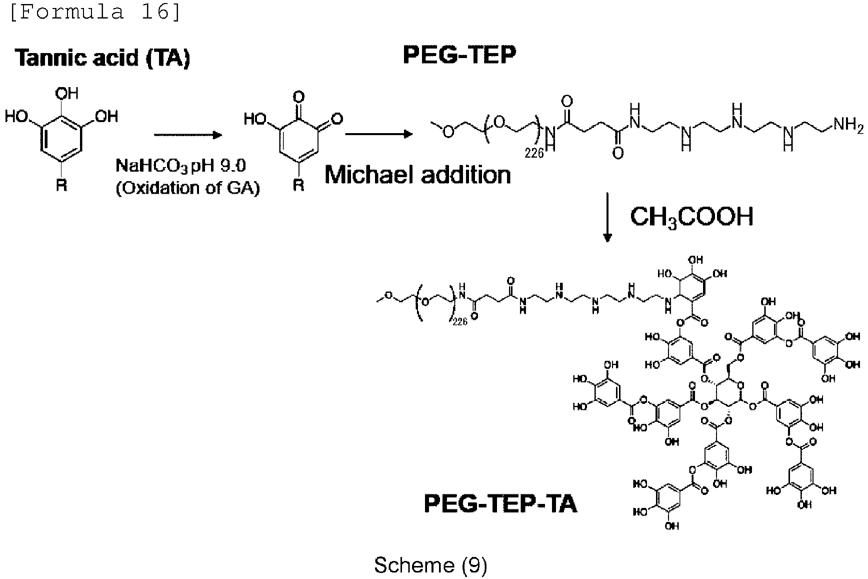

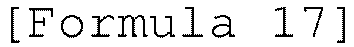

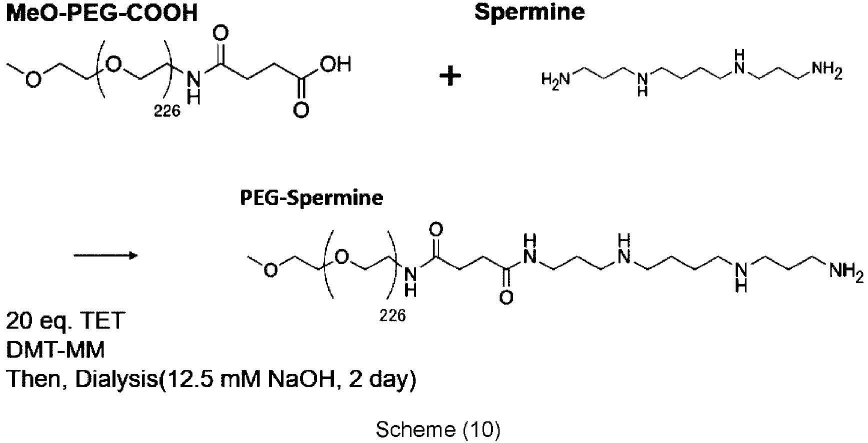

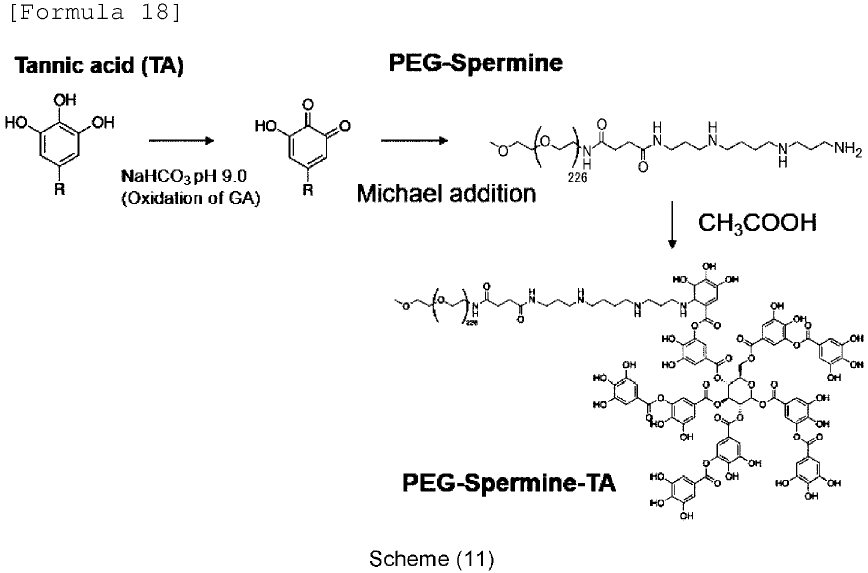

### (13.2. Reagents)

· α-Hydroxyl-ω-carboxyl-poly(ethylene glycol) (MeO-PEG₁₀ₖ-COOH, Mn: 10k, NOF Corporation)
· Tannic acid (FUJIFILM Wako Pure Chemical Corporation)
• N,N'-Bis(2-aminoethyl)-1,2-ethanediamine (TET, Tokyo Chemical Industry)
• N(2-Aminoethyl)-N-(2-((2-aminoethyl)amino)ethyl-1,2-ethanediamine (TEP, Tokyo Chemical Industry)
• N,N'-Bis(3-aminopropyl)butane-1,4-diamine (Spermine, FUJIFILM Wako Pure Chemical Corporation)
• Sodium hydrogen carbonate (NaHCO₃, FUJIFILM Wako Pure Chemical Corporation)
• Acetic acid (CH₃COOH) (Nacalai Tesque)
• {2-[4-(2-Hydroxyethyl)-1-piperazinyl]ethanesulfonic acid} (HEPES, Nacalai Tesque)
• Sodium chloride (NaCl, FUJIFILM Wako Pure Chemical Corporation)
• Sodium hydroxide (NaOH, Nacalai Tesque)

### (13.3. Measurement Instruments)

- Nuclear magnetic resonance (NMR) instrument (BRUKER AVANCE III 400 (400 MHz, BRUKER BioSpin))
- Gel permeation chromatography (GPC) instrument (JASCO International)
   Column: Superdex 200 Increase 10/300 GL (Cytiva)
   Column: Superdex 75 Increase 10/300 GL (Cytiva)
   Detectors: RI-2031, UV-2030
- JASCO V-650 spectrophotometer (JASCO)

### (13.4. Polymer Synthesis)

### <Synthesis of PEG₁₀ₖ-TET-TA>

### [Binding of TET to PEG₁₀ₖ-COOH]

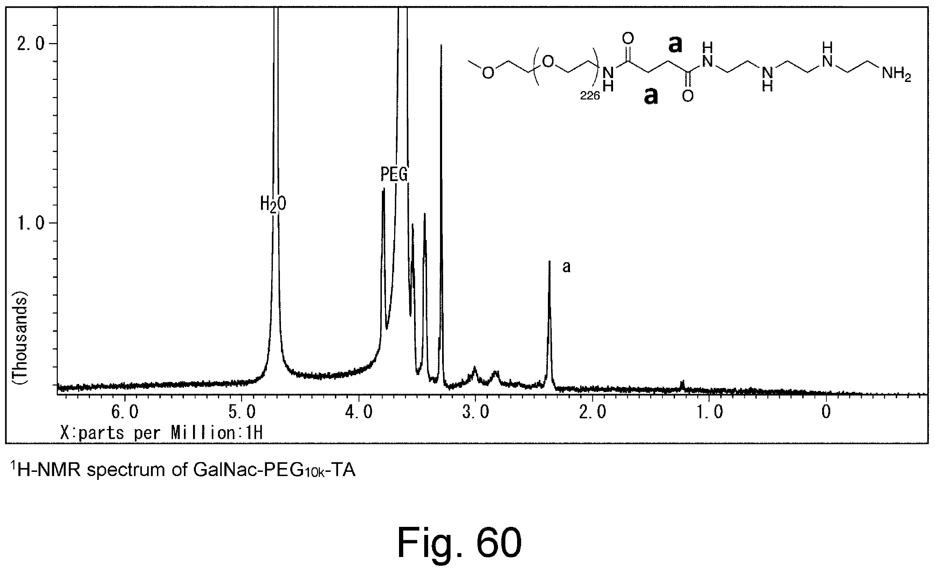

29 mg (0.2 mmol) of TET was weighed into a 200 mL beaker and dissolved in 80 mL of 10 mM NaHCO₃ (pH 8.5). To this was added dropwise 100 mg (0.010 mmol) of PEG₁₀ₖ-COOH dissolved in 40 mL of 10 mM NaHCO₃ (pH 8.5), 1.5 mmol of DMT-MM was added, and the mixture was stirred at room temperature for 5 hours. Thereafter, dialysis was performed against 0.5 mL of NaOH and 2 L of distilled water, followed by dialysis against distilled water alone. Figure 60 shows the ¹H-NMR spectrum of the sample dissolved in D₂O.

### [Binding of TA to PEG₁₀ₖ-TET]

10 mg (0.006 mmol) of TA was weighed into a 200 mL beaker and dissolved in 60 mL of 50 mM NaHCO₃ (pH 8.5). To this was added dropwise 30 mg (0.003 mmol) of PEG₁₀ₖ-TET dissolved in 40 mL of 50 mM NaHCO₃ (pH 8.5), followed by stirring at room temperature for 5 hours. Thereafter, the reaction solution transferred into a dialysis membrane (MWCO = 6 to 8 kDa) was dialyzed for 1 hour against 2 L of pure water subjected to ultrasonication for 10 minutes and Ar bubbling for 5 minutes.

### [Reduction of Side-Chain Ketone Groups of TA]

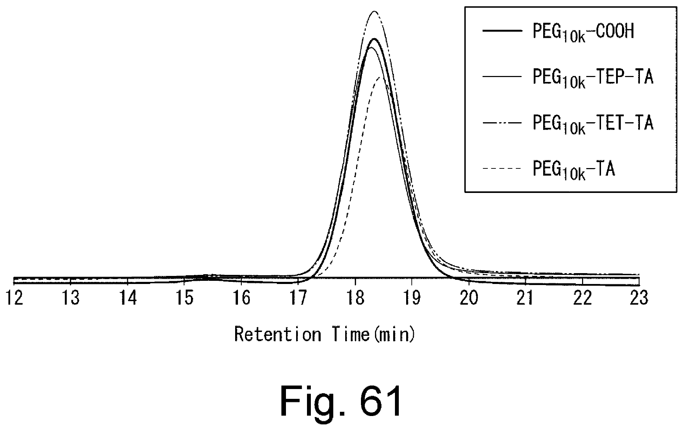

The reaction solution was transferred to a 200 mL beaker, and 5 mL of a 10-fold dilution of 0.5 mL of acetic acid was added dropwise, followed by stirring for 5 minutes. Thereafter, the reaction solution transferred into a dialysis membrane (MWCO = 6 to 8 kDa) was dialyzed twice against 2 L of pure water subjected to ultrasonication for 10 minutes and Ar bubbling for 5 minutes. The resulting solution was freeze-dried to obtain yellow solid PEG₁₀ₖ-TET in a yield of 30 mg. Figure 61 shows the GPC curve (obtained using a Superdex^{™} 200 Increase 10/300 GL column; eluent: 10 mM HEPES, 140 mM NaCl (pH 7.4); flow rate: 0.75 mL/min; detector: UV-2030; detection wavelength: 254 nm; measurement temperature: room temperature).

### <Synthesis of PEG-TEP-TA>

### [Binding of TEP to PEG₁₀ₖ-COOH]

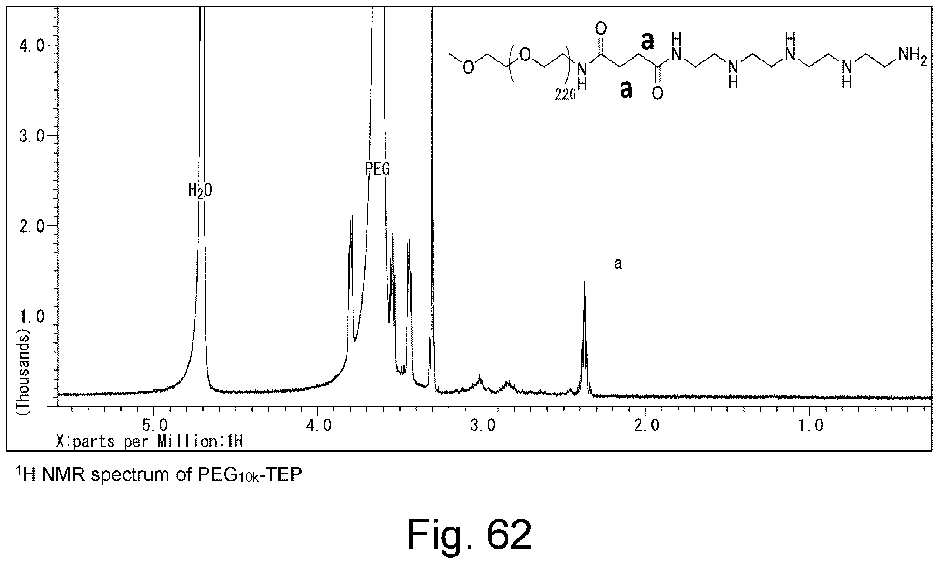

38 mg (0.2 mmol) of TEP was weighed into a 200 mL beaker and dissolved in 80 mL of 10 mM NaHCO₃ (pH 8.5). To this was added dropwise 100 mg (0.010 mmol) of PEG₁₀ₖ-COOH dissolved in 40 mL of 10 mM NaHCO₃ (pH 8.5), 1.5 mmol of DMT-MM was added, and the mixture was stirred at room temperature for 5 hours. Thereafter, dialysis was performed against 0.5 mL of NaOH and 2 L of distilled water, followed by dialysis against distilled water alone. Figure 62 shows the ¹H-NMR spectrum of the sample dissolved in D₂O.

### [Binding of TEP to PEG₁₀ₖ-TEP]

10 mg (0.006 mmol) of TA was weighed into a 200 mL beaker and dissolved in 60 mL of 50 mM NaHCO₃ (pH 8.5). To this was added dropwise 30 mg (0.003 mmol) of PEG₁₀ₖ-TEP dissolved in 40 mL of 50 mM NaHCO₃ (pH 8.5), followed by stirring at room temperature for 5 hours. Thereafter, the reaction solution transferred into a dialysis membrane (MWCO = 6 to 8 kDa) was dialyzed for 1 hour against 2 L of pure water subjected to ultrasonication for 10 minutes and Ar bubbling for 5 minutes.

### [Reduction of Side-Chain Ketone Groups of TA]

The reaction solution was transferred to a 200 mL beaker, and 5 mL of a 10-fold dilution of 0.5 mL of acetic acid was added dropwise, followed by stirring for 5 minutes. Thereafter, the reaction solution transferred into a dialysis membrane (MWCO = 6 to 8 kDa) was dialyzed twice against 2 L of pure water subjected to ultrasonication for 10 minutes and Ar bubbling for 5 minutes. The resulting solution was freeze-dried to obtain yellow solid PEG₁₀ₖ-TEP in a yield of 30 mg. Figure 61 shows the GPC curve (obtained using a Superdex^{™} 200 Increase 10/300 GL column; eluent: 10 mM HEPES, 140 mM NaCl (pH 7.4); flow rate: 0.75 mL/min; detector: UV-2030; detection wavelength: 254 nm; measurement temperature: room temperature).

### <Synthesis of PEG-S-TA>

### [Binding of Spermine to PEG₁₀ₖ-COOH]

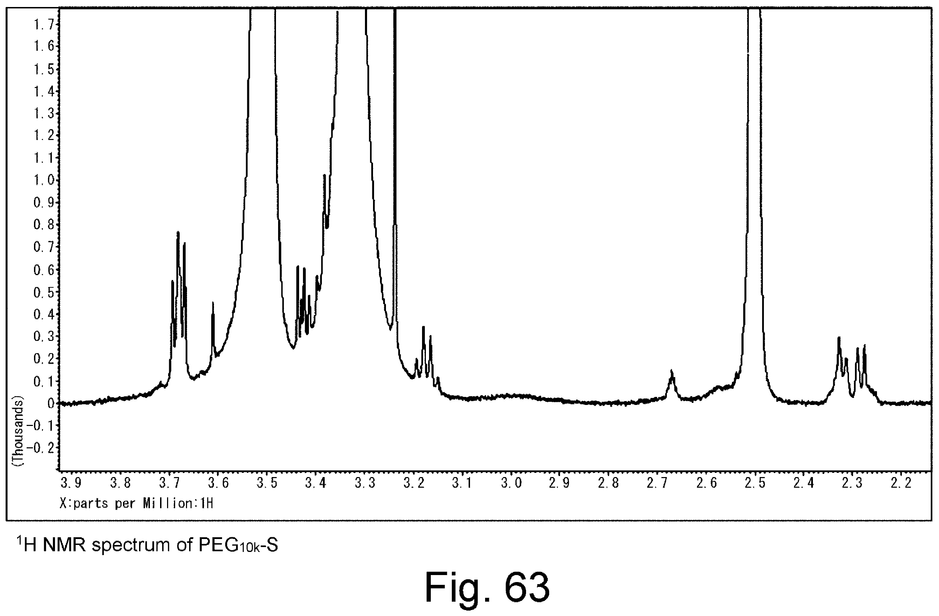

40 mg (0.2 mmol) of Spermine was weighed into a 200 mL beaker and dissolved in 80 mL of 10 mM NaHCO₃ (pH 8.5). To this was added dropwise 100 mg (0.010 mmol) of PEG₁₀ₖ-COOH dissolved in 40 mL of 10 mM NaHCO₃ (pH 8.5), 1.5 mmol of DMT-MM was added, and the mixture was stirred at room temperature for 5 hours. Thereafter, dialysis was performed against 0.5 mL of NaOH and 2 L of distilled water, followed by dialysis against distilled water alone. Figure 63 shows the ¹H-NMR spectrum of the sample dissolved in D₂O.

### [Binding of TA to PEG₁₀ₖ-Spermine]

10 mg (0.006 mmol) of TA was weighed into a 200 mL beaker and dissolved in 60 mL of 50 mM NaHCO₃ (pH 8.5). To this was added dropwise 30 mg (0.003 mmol) of PEG₁₀ₖ-TET dissolved in 40 mL of 50 mM NaHCO₃ (pH 8.5), followed by stirring at room temperature for 5 hours. Thereafter, the reaction solution transferred into a dialysis membrane (MWCO = 6 to 8 kDa) was dialyzed for 1 hour against 2 L of pure water subjected to ultrasonication for 10 minutes and Ar bubbling for 5 minutes.

### [Reduction of Side-Chain Ketone Groups of TA]

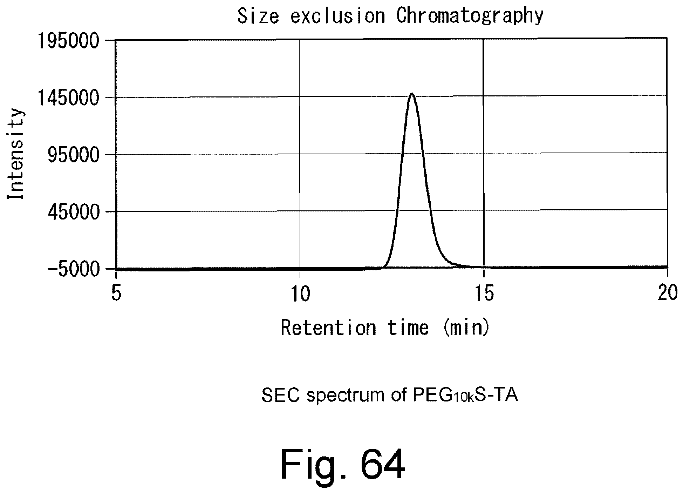

The reaction solution was transferred to a 200 mL beaker, and 5 mL of a 10-fold dilution of 0.5 mL of acetic acid was added dropwise, followed by stirring for 5 minutes. Thereafter, the reaction solution transferred into a dialysis membrane (MWCO = 6 to 8 kDa) was dialyzed twice against 2 L of pure water subjected to ultrasonication for 10 minutes and Ar bubbling for 5 minutes. The resulting solution was freeze-dried to obtain yellow solid PEG₁₀ₖ-TET in a yield of 30 mg. Figure 64 shows the GPC curve (obtained using a Superdex^{™} 75 Increase 10/300 GL column; eluent: 10 mM HEPES, 140 mM NaCl (pH 7.4); flow rate: 0.75 mL/min; detector: UV-2030; detection wavelength: 280 nm; measurement temperature: room temperature).

### (13.5. Analysis)

### <PEG₁₀ₖ-TET-TA>

From the GPC curve, it was confirmed that PEG₁₀ₖ-TET-TA had a narrow molecular weight distribution. In the ¹H-NMR spectrum of PEG₁₀ₖ-TET, a peak derived from PEG and a peak derived from TET were confirmed, confirming quantitative introduction. In addition, the introduction ratio of TA was quantitatively confirmed from the absorbance at 290 nm, an absorption wavelength derived from the GA groups.

### <PEG₁₀ₖ-TEP-TA>

From the GPC curve, it was confirmed that PEG₁₀ₖ-TEP-TA had a narrow molecular weight distribution. In the ¹H-NMR spectrum of PEG₁₀ₖ-TEP, a peak derived from PEG and a peak derived from TEP were confirmed, confirming quantitative introduction. In addition, the introduction ratio of TA was quantitatively confirmed from the absorbance at 290 nm, an absorption wavelength derived from the GA groups.

### <PEG₁₀ₖ-Spermine-TA>

From the GPC curve, it was confirmed that PEG₁₀ₖ-Spermine-TA had a narrow molecular weight distribution. In the ¹H-NMR spectrum of PEG₁₀ₖ-Spermine, a peak derived from PEG and a peak derived from Spermine (around 2.3 ppm) were confirmed, confirming quantitative introduction. In addition, the introduction ratio of TA was quantitatively confirmed from the absorbance at 290 nm, an absorption wavelength derived from the GA groups.

### [Experimental Example 14]

### <Confirmation of Complex Formation Between Synthetic Polymer and Metal Ions>

### (14.1. Overview)

GA groups and metal ions form a metal ion-polyphenol complex (MPN) having buffering ability by crosslinking. Therefore, complex formation between each polymer and iron ions was confirmed using a spectrophotometer.

### (14.2. Reagents)

- Iron(III) chloride hexahydrate (FeCl₃·6H₂O) (Fe³⁺) (FUJIFILM Wako Pure Chemical Corporation)
- PEG₁₀ₖ-TA (Mn = 11,701)
- PEG₁₀ₖ-TET-TA (Mn = 11,847)
- PEG₁₀ₖ-TEP-TA (Mn = 11,890)
- PEG₁₀ₖ-S-TA (Mn = 11,903)
- Tannic acid (FUJIFILM Wako Pure Chemical Corporation)
- D-PBS(-) (FUJIFILM Wako Pure Chemical Corporation)

### (14.3. Measurement Instruments)

- JASCO V-650 spectrophotometer (JASCO)

### (4.4. Confirmation of Complex Formation)

### <Final Concentrations of Polymers and FeCl₃·6H₂O>

- PEG₁₀ₖ-TA: 0.04 mM
- PEG₁₀ₖ-TET-TA: 0.04 mM
- PEG₁₀ₖ-TEP-TA: 0.04 mM
- PEG₁₀ₖ-S-TA: 0.04 mM
- FeCl₃·6H₂O (Fe³⁺): 0.12 mM

PEG₁₀ₖ-TA, PEG₁₀ₖ-TET-TA, PEG₁₀ₖ-TEP-TA, and PEG₁₀ₖ-S-TA were prepared by dissolution in D-PBS(-), and Fe³⁺ was prepared by dissolution in ultrapure water.

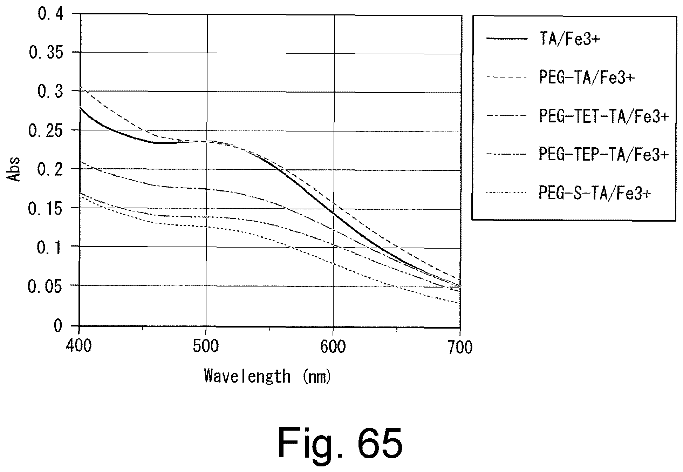

The polymer solutions and Fe³⁺ were mixed at the above concentrations to prepare solutions. After incubation at room temperature for 15 minutes, absorption spectra were measured using a spectrophotometer. Figure 65 is a graph showing the results of measuring the absorption spectra.

### (14.5. Analysis)

From the absorption spectra shown in Figure 65, peaks were observed at around 550 nm for all samples, confirming that the various polymers and Fe³⁺ formed complexes.

### [Experimental Example 15]

### <Evaluation of Formation of GalNac-Modified β-Gal-Loaded MPN Complex>

### (15.1. Overview)

MPN complexes were formed using not only proteins but also inorganic/organic microparticles, nucleic acids, and lipid nanoparticles, and changes in particle size were measured. Particle size was measured using dynamic light scattering (DLS) or fluorescence correlation spectroscopy (FCS).

### (15.2. Reagents)

- D-PBS(-) (FUJIFILM Wako Pure Chemical Corporation)
- β-D-Galactosidase (β-Gal, Mw: 540 kDa; FUJIFILM Wako Pure Chemical Corporation)
- PEG₁₀ₖ-TA
- GalNac-PEG₁₀ₖ-TA

### (15.3. Measurement Instruments)

- LSM710 (Carl Zeiss Co., Ltd.)
- Zetasizer Ultra (Malvern Co., Ltd.)

### (15.4. Evaluation of Formation of GalNac-Modified β-Gal-Loaded MPN Complex)

### <Introduction of Alexa647 into β-Gal Complex>

- β-Gal: 10 mg
- Alexa Flour 647-NHS: 0.12 mg

10 mg of β-Gal was weighed into a 20 mL vial and dissolved in 10 mL of 50 mM NaHCO₃ (pH 8.0). 0.12 mg of Alexa Flour 647-NHS dissolved in DMSO was added thereto, followed by stirring at room temperature for 4 hours. After the reaction solution was subjected twice to ultrafiltration (MWCO: 10 kDa) using PBS, unreacted Alexa Flour 647-NHS was removed using a PD-10 column (PBS as the solvent). Subsequently, ultrafiltration (MWCO: 10 kDa) was performed twice again using PBS, and Alexa Flour 647-modified β-Gal (Alexa647-β-Gal) was recovered as a solution. Thereafter, the β-Gal concentration was calculated from the absorbance at 280 nm, a protein-derived absorption wavelength.

### <Final Concentrations of Alexa647-β-Gal, PEG-TA, GalNac-PEG-TA, and Fe³⁺>

- Alexa647-β-Gal (β-Gal): 370 nM
- PEG-TA: 92.5 µM
- GalNac-PEG₁₀ₖ-TA: 92.5 µM
- Fe³⁺: 305 µM (stock concentration: 6.1 mM)

Alexa647-β-Gal, PEG-TA, and GalNac-PEG-TA were prepared by dissolution in PBS(-), and Fe³⁺ was prepared by dissolution in ultrapure water.

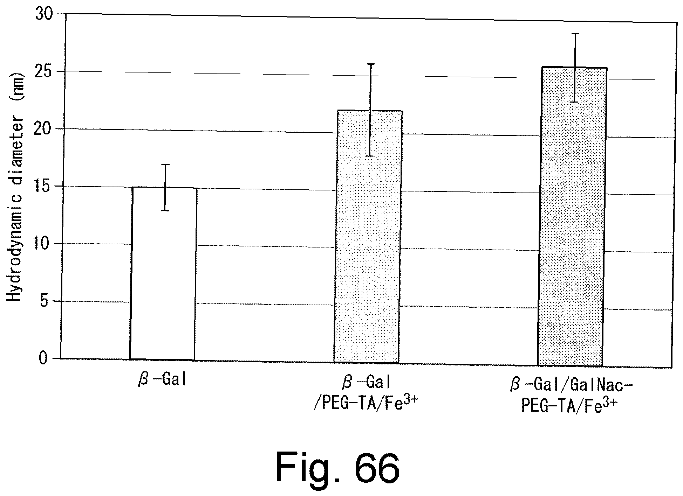

The Alexa647-β-Gal solution, PEG-TA solution, GalNac-PEG₁₀ₖ-TA solution, and Fe³⁺ solution were mixed by pipetting. In doing so, the polymer/β-Gal molar ratio was set to 250. In addition, the molar ratio of iron ions was set to 3.3 equivalents relative to TA in the polymer. Finally, after the concentration was adjusted by adding PBS(-), particle size was measured by fluorescence correlation spectroscopy using LSM710. Figure 66 shows the results of particle size measurement.

The particle sizes of the β-Gal/PEG₁₀ₖ-TA/Fe³⁺ MPN complex and the β-Gal/GalNac-PEG-TA/Fe³⁺ MPN complex were significantly increased compared with the particle size of β-Gal alone. These results indicated formation of the β-Gal MPN complexes.

### [Experimental Example 16]

### <Confirmation of Formation of MPN Complexes Loaded with Various Molecules>

### (16.1. Overview)

MPN complexes loaded with not only proteins but also inorganic/organic microparticles, nucleic acids, and lipid nanoparticles were formed, and changes in particle size were measured. Particle size was measured with dynamic light scattering (DLS) or fluorescence correlation spectroscopy (FCS).

### (16.2. Reagents)

Unless otherwise specified, commercially available reagents and solvents were used as purchased.
- D-PBS(-) (FUJIFILM Wako Pure Chemical Corporation)
- β-D-Galactosidase (β-Gal, Mw: 540 kDa; FUJIFILM Wako Pure Chemical Corporation)
- LipidLaunch^{™} SORT LNP Exploration Kit (Cayman Chemical)
- Iron(III) chloride hexahydrate (FeCl₃·6H₂O) (Fe³⁺) (FUJIFILM Wako Pure Chemical Corporation)
- D-PBS(-) (FUJIFILM Wako Pure Chemical Corporation)
- Sodium acetate (FUJIFILM Wako Pure Chemical Corporation)
- Alexa Flouor 647-TUG1 (8058.7 g/mol) (GeneDesign. Inc.)
- PEG₅ₖ-TA (Mₙ = 6,700)
- PEG₁₀ₖ-TA (Mₙ = 11,700)
- GalNac-PEG₁₀ₖ-TA (Mₙ = 12,400)
- PS Particle-COOH (50 nm) (Abvigen)
- PEG-P[Lys₁₁/(Lys-GA)14]
- Alexa Flouor 647-TUG1 (TUG1, 8058.7 g/mol) (GeneDesign. Inc.)
- Zirconium(IV) chloride (ZrCl₄) (Zr⁴⁺) (FUJIFILM Wako Pure Chemical Corporation)
- UltraPure^{™} DNase/Rnase-Free Distilled Water (Thermo Fisher Scientific)

### (16.3 Measurement Instruments)

- Zetasizer Ultra (red) (Malvern Panalytical)
- LSM710 (Carl Zeiss)

### (16.4 Evaluation of Physicochemical Properties of LNP MPN Complex)

When an MPN complex is formed by mixing LNP, PEG₅ₖ-TA (Mₙ = 6,700), and metal ions, the particle size increases compared with that of LNP alone. Particle size was measured using a Zetasizer Ultra (red). Fe³⁺ was used as the metal ion.

### [LNP Formation]

In accordance with the kit instructions, the respective solutions of the LipidLaunch^{™} SORT LNP Exploration Kit were dispensed into a 1.5 mL tube so that the total volume was 100 µL. Thereafter, 12.5 µL of Alexa Flouor 647-TUG1 (2 mg/mL) was added to a separate 1.5 mL tube, and 285.7 µL of 50 mM sodium acetate (pH 4.0) was added thereto. To this solution, 100 µL of the LipidLaunch^{™} SORT LNP Exploration Kit solution was added, and the mixture was stirred by pipetting for 15 seconds. The solution was allowed to stand for 10 minutes, after which the reaction solution was added to an ultrafiltration tube (MWCO: 30 kDa) and centrifuged twice at 2,300 × rpm for 20 minutes to remove unreacted lipids and the like. Finally, the volume was made up to 500 µL using D-PBS(-) to obtain LNPs into which Alexa Flouor 647-TUG1 had been introduced.

### [Confirmation of Formation of LNP MPN Complex]

### <Final Concentrations of LNP, PEG₅ₖ-TA, and FeCl₃·6H₂O>

- LNP: solution used during preparation, diluted 20-fold
- PEG₅ₖ-TA: 11.2 µM (stock concentration: 224 µM)
- FeCl_{3'}6H₂O: 12.9 µM (stock concentration: 370 µM)

LNP and PEG₅ₖ-TA were prepared by dissolution in D-PBS(-), and FeCl₃·6H₂O was prepared by dissolution in ultrapure water.

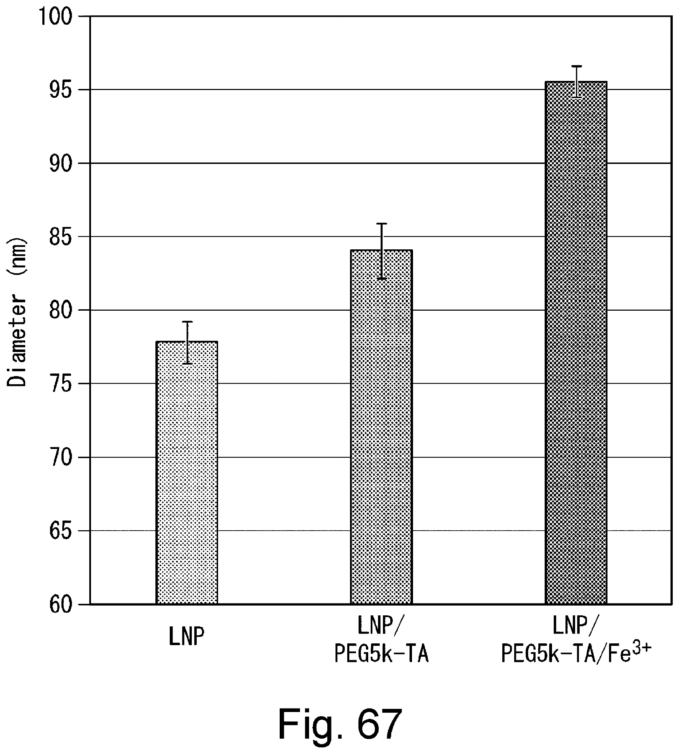

The LNP solution, PEG₅ₖ-TA solution, and FeCl₃·6H₂O solution were mixed by vortexing. In doing so, the molar ratio of PEG₅ₖ-TA to FeCl₃·6H₂O was set to 1:1.2. In addition, a mixture of only the LNP solution and the PEG₅ₖ-TA solution was also prepared. After the concentrations of these solutions and the LNP solution were adjusted by adding D-PBS(-) to each, particle size was measured using a Zetasizer Ultra (red). Figure 67 shows the results of particle size measurement.

The particle size of the LNP/PEG₅ₖ-TA/Fe³⁺ complex was significantly increased compared with those of LNP and LNP/PEG₅ₖ-TA, indicating formation of the LNP/PEG₅ₖ-TA/Fe³⁺ complex.

### [Evaluation of LNP MPN Complex Formation Upon Varying Ratio of PEG₅ₖ-TA and FeCl₃·6H₂O]

### <Final Concentrations of LNP, PEG₅ₖ-TA, and FeCl₃·6H₂O>

- LNP: solution used during preparation, diluted 20-fold
- PEG₅ₖ-TA: 11.2 to 22.4 µM (stock concentration: 224 µM)
- FeCl₃·6H₂O: 12.9 to 33.6 µM (stock concentration: 370 µM)

LNP and PEG₅ₖ-TA were prepared by dissolution in D-PBS(-), and FeCl₃·6H₂O was prepared by dissolution in ultrapure water.

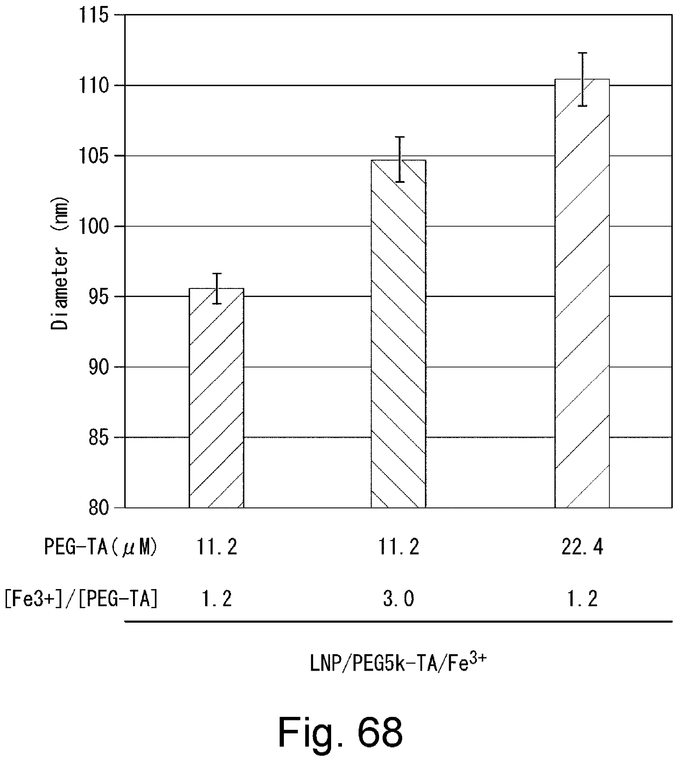

The LNP solution, PEG₅ₖ-TA solution, and FeCl₃·6H₂O solution were mixed by vortexing. In doing so, the final concentration of PEG₅ₖ-TA was set to 11.2 µM, and the molar ratio of PEG₅ₖ-TA to FeCl₃·6H₂O was set to 1:1.2 or 1:3. In addition, a sample in which the final concentration of PEG₅ₖ-TA was set to 22.4 µM and the molar ratio of PEG₅ₖ-TA to FeCl₃·6H₂O was set to 1:1.2 was also prepared. After the concentrations of these solutions were adjusted by adding D-PBS(-) to each, particle size was measured using a Zetasizer Ultra (red). Figure 68 shows the results of particle size measurement.

The particle sizes of all LNP/PEG₅ₖ-TA/Fe³⁺ complexes were significantly increased compared with those of LNP, suggesting formation of the LNP/PEG₅ₖ-TA/Fe³⁺ complex. In addition, as the amounts of PEG₅ₖ-TA and FeCl₃·6H₂O mixed increased, an increase in particle size was observed.

### (16.5. Evaluation of Physicochemical Properties of PS Particle MPN Complex)

When an MPN complex is formed by mixing PS particles, metal ions, and PEG-TA, the particle size increases compared with that of PS particles alone. Particle size was measured using a Zetasizer Ultra (red). Fe³⁺ was used as the metal ion.

### [Confirmation of Formation of PS Particle MPN Complex]

### <Final Concentrations of PS Particles, PEG₅ₖ-TA, PEG₁₀ₖ-TA, and FeCl₃·6H₂O>

- PS particles: 0.86 mg/mL
- PEG₅ₖ-TA: 60 µM (stock concentration: 880 µM)
- PEG₁₀ₖ-TA: 60 µM (stock concentration: 880 µM)
- FeCl₃·6H₂O: 76 to 180 µM (stock concentration: 370 µM)

Each was prepared by dissolution in ultrapure water.

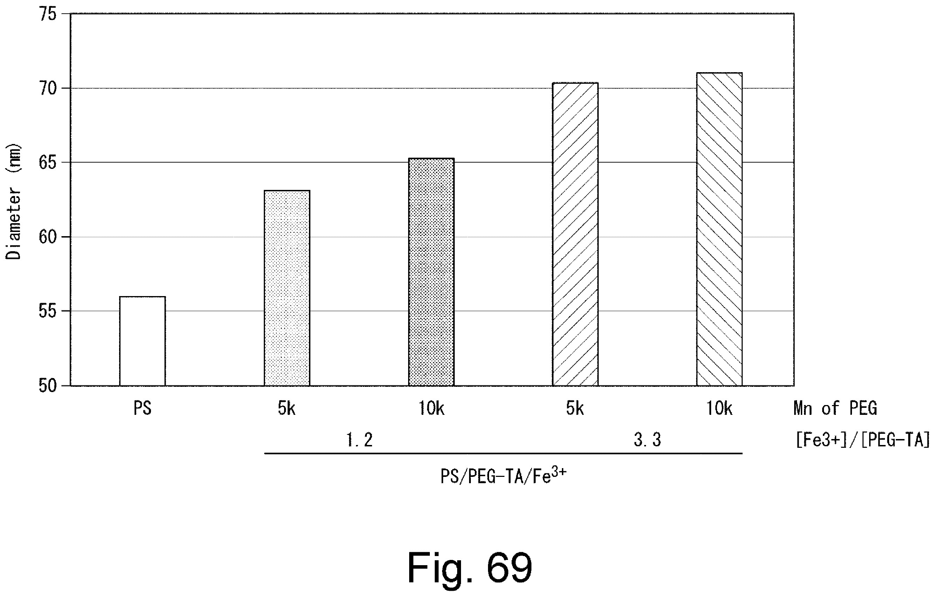

The PS particle solution, PEG₅ₖ-TA solution, and FeCl₃·6H₂O solution, or the PS particle solution, PEG₁₀ₖ-TA solution, and FeCl₃·6H₂O solution, were each mixed by vortexing. In doing so, the final concentration of PEG-TA was set to 60 µM, and the molar ratio of PEG-TA to FeCl₃·6H₂O was set to 1:1.2 or 1:3. In addition, a solution of PS particles alone was also prepared, and ultrapure water was added to each of these solutions to adjust the concentrations, after which particle size was measured using a Zetasizer Ultra (red). Figure 69 shows the results of particle size measurement.

The particle sizes of the respective PS/PEG₅ₖ-TA/Fe³⁺ complexes and PS/PEG₁₀ₖ-TA/Fe³⁺ complexes were all significantly increased compared with that of the PS particles, suggesting formation of the PS/PEG₅ₖ-TA/Fe³⁺ complex and the PS/PEG₁₀ₖ-TA/Fe³⁺ complex. In addition, as the amounts of FeCl₃·6H₂O mixed increased, an increase in particle size was observed.

### (16.6. Evaluation of Physicochemical Properties of Nucleic Acid MPN Complex)

When an MPN complex is formed by mixing a nucleic acid and a metal ion, and then mixing PEG-TA or PEG-P[Lys_{11/}(Lys-GA)₁₄] therewith, the particle size increases compared with that of the nucleic acid alone. Therefore, particle size measurement was performed using fluorescence correlation spectroscopy. Zr⁴⁺ was used as the metal ion.

### [Confirmation of Formation of Nucleic Acid MPN Complex]

### <Final Concentrations of TUG1, PEG₁₀ₖ-TA, PEG-P[Lys₁₁/(Lys-GA)₁₄], and ZrCl₄>

- TUG1: 3.36 µM
- PEG₁₀ₖ-TA: 672 µM (stock concentration: 940 µM)
- PEG-P[Lys₁₁/(Lys-GA)₁₄]: 672 µM (stock concentration: 940 µM)
- ZrCl₄: 307 to 613 µM (stock concentration: 2,146 µM)

These were all prepared by dissolution in UltraPure^{™} DNase/Rnase-Free Distilled Water.

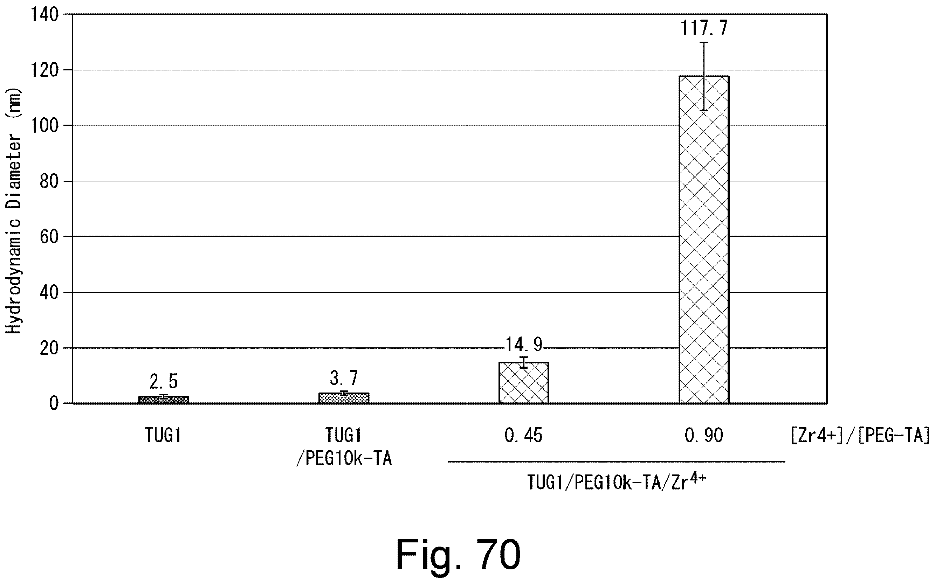

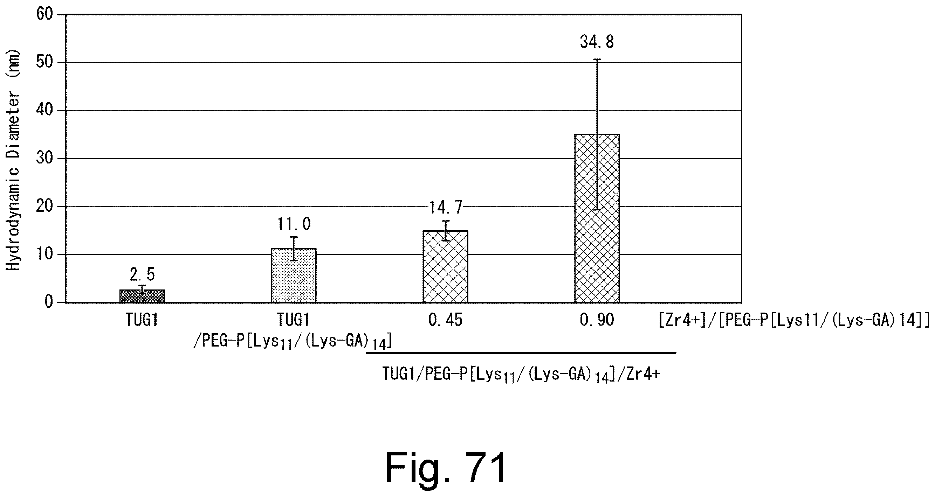

The TUG1 solution, PEG₁₀ₖ-TA solution, and ZrCl₄ solution, or the TUG1 solution, PEG-P[Lys₁₁/(Lys-GA)₁₄] solution, and ZrCl₄ solution, were each mixed by vortexing. In doing so, the molar ratios of PEG₁₀ₖ-TA to ZrCl₄ and of PEG-P[Lys₁₁/(Lys-GA)₁₄] to ZrCl₄ were each set to 1:0, 1:0.45, and 1:0.9. In addition, a solution of TUG1 alone was also prepared, and D-PBS(-) was added to each of these solutions to adjust the concentrations, after which particle size was measured using LSM710. Figures 70 and 71 show the results of particle size measurement.

The particle sizes of the respective TUG1/PEG₁₀ₖ-TA/Zr⁴⁺ complexes and TUG1/PEG-P[Lys₁₁/(Lys-GA)14]/Zr⁴⁺ complexes were all significantly increased compared with that of TUG1. These results suggested formation of the TUG1/PEG₁₀ₖ-TA/Zr⁴⁺ complex and the TUG1/PEG-P[Lys₁₁/(Lys-GA)₁₄]/Zr⁴⁺ complex. In addition, whereas the particle size of the TUG1/PEG₁₀ₖ-TA complex did not increase substantially compared with that of TUG1, the particle size of the TUG1/PEG-P[Lys₁₁/(Lys-GA)₁₄] complex was significantly increased. These results suggested that only the TUG1/PEG-P[Lys₁₁/(Lys-GA)₁₄] complex was formed. In addition, as the amounts of ZrCl₄ mixed increased, an increase in particle size was observed.

### [Experimental Example 7]

### <Evaluation of Physicochemical Properties of Nucleic Acid Complex (RNP)-Loaded MPN Complex>

### (17.1. Overview)

Formation of a Cas9-sgRNA RNP-loaded MPN complex was confirmed using newly synthesized PEG-ethylenediamine-TA. Specifically, particle size increase was measured by fluorescence correlation spectroscopy.

### (17.2. Reagents)

- Cas9-GFP protein (Sigma-Aldrich)
- sgRNA (Luciferase-2) (GenScript)
- D-PBS(-) (FUJIFILM Wako Pure Chemical Corporation)
- PEG₁₀ₖ-TET-TA (Mn = 11,847)
- PEG₁₀ₖ-TEP-TA (Mn = 11,890)
- PEG₁₀ₖ-S-TA (Mn = 11,903)
- Iron(III) chloride hexahydrate (FeCl₃·6H₂O) (Fe³⁺) (FUJIFILM Wako Pure Chemical Corporation)

### (17.3. Measurement Instruments)

- LSM710 (Carl Zeiss)

### (17.4. Evaluation of Formation of RNP MPN Complex)

### <Final Concentrations of Cas9-sgRNA, Polymer, and FeCl₃·6H₂O>

- Cas9-GFP: 0.2 µM
- sgRNA: 0.2 µM
- Polymer (PEG-TET-TA, PEG-TEP-TA, PEG-S-TA): 20 µM
- FeCl₃·6H₂O(Fe³⁺): 67 µM

Cas9-sgRNA and the polymers were prepared by dissolution in D-PBS(-). FeCl₃·6H₂O was prepared by dissolution in pure water.

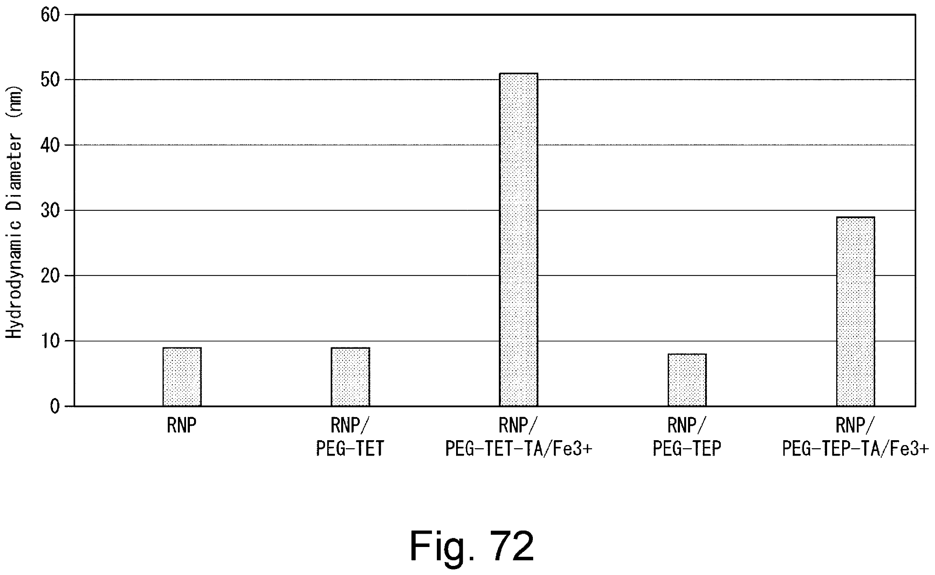

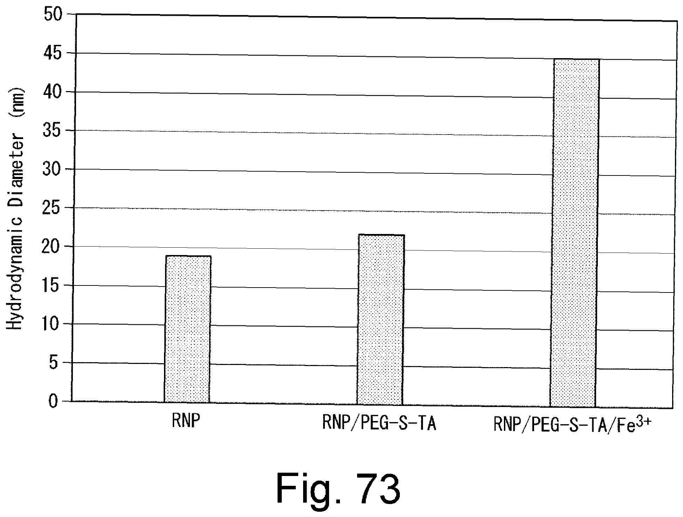

Cas9-GFP and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, a polymer (PEG-TET-TA, PEG-TEP-TA, or PEG-S-TA) was added, followed by standing at room temperature for 15 minutes to prepare both RNP/polymer solutions. Fe³⁺ was further added, followed by standing at room temperature for 15 minutes to prepare both RNP/polymer/Fe³⁺ MPN complex solutions. In doing so, the polymer/RNP molar ratio was set to 100. In addition, the molar ratio of iron ions was set to 3.3 equivalents relative to TA contained in the polymer. Finally, particle size was measured by fluorescence correlation spectroscopy using LSM710. Figures 72 and 73 show the results of particle size measurement.

### (17.5. Analysis)

As shown in Figures 72 and 73, the particle sizes of both RNP/polymer/Fe³⁺ were significantly increased compared with the particle sizes of RNP and RNP/PEG-TA. These results suggested formation of both RNP/polymer/Fe³⁺ MPN complexes.

### [Experimental Example 18]

### <Evaluation of Function of Antibody-Loaded MPN Complex>

### (18.1. Overview)

Additional confirmation of formation of the antibody MPN complex and evaluation of its functionality were performed. Specifically, electron microscopic observation and confirmation of buffering effects, therapeutic effects in an orthotopically transplanted breast cancer model, and the mechanism were performed.

### (18.2. Reagents)

- Anti-S100A4 antibody (Creative Biolabs)
- Anti-NPC Antibody (BioLegend)
- PEG-TA
- TA: Wako Pure Chemical Industries
- p53 (D2H9O) Rabbit mAb #32532 (Anti-p53 antibody, Cell Signaling)
- HRP-Conjugated Anti-Rabbit mAb (Cell Signaling)
- TUNEL Assay Kit-HRP-DAB (ab206386, Abcam)
- FUJI DRI-CHEM SLIDE (GOT, GPT) (FUJIFILM Wako Pure Chemical Corporation)
- Roswell Park Memorial Institute medium (RPMI, Sigma-Aldrich Co., llc.)
- Fetal bovine serum (FBS, Biosera Inc.)
- Trypsin-EDTA solution (Trp, Sigma Life Science Co., Ltd.)
- Penicillin/streptomycin (PS, Sigma Life Science Co., Ltd.)
- PBST (FUJIFILM Wako Pure Chemical Corporation)
- TMB solution (FUJIFILM Wako Pure Chemical Corporation)
- Ab206386-TUNEL Assay Kit-HRP-DAB (Abcam)
- 5 M Hydrochloric Acid (Nacalai Tesque)

### (18.3. Measurement Instruments)

- JEM-1400 (JEOL)
- LSM710 (Carl Zeiss Co., Ltd.)
- FUJI DRI-CHEM NX500 (FUJIFILM Wako Pure Chemical Corporation)
- pH meter (HORIBA)
- All-in-one fluorescence microscope BZ-X800 (Keyence)
- Cryostat (Leica)

### (18.4. Electron Microscopic Observation of Antibody-Loaded MPN Complex)

### <Final Concentrations of Antibody, PEG-TA, and Fe³⁺>

- Anti-NPC antibody: 660 nM
- PEG-TA: 16 µM (stock concentration: 80 µM)
- Fe³⁺: 55 µM (stock concentration: 2.2 mM)

Each was prepared by dissolution in 1 mM HEPES buffer (pH 7.4). Fe³⁺ was prepared by dissolution in ultrapure water.

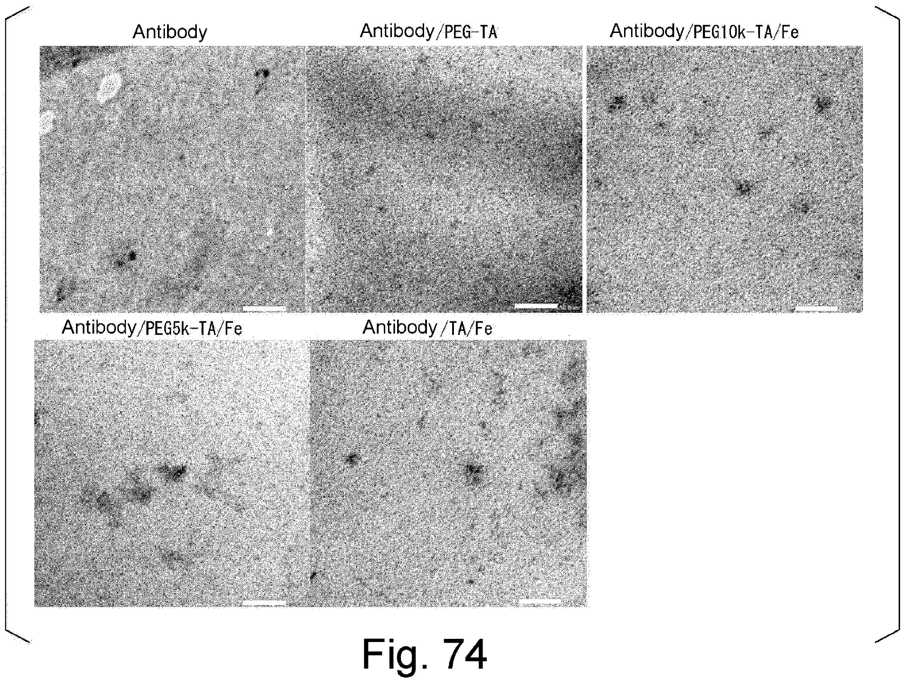

The antibody solution, the two PEG-TA solutions, the TA solution, and the Fe³⁺ solution were mixed by pipetting. In doing so, the molar ratio of PEG-TA/antibody and TA/antibody were set to 25. In addition, the molar ratio of iron ions was set to 3.3 equivalents relative to TA in PEG-TA. Thereafter, 5 µL of the sample solution was dropped onto a copper grid, and the solution was removed after 1 minute. Thereafter, staining was performed using a gadolinium acetate solution, followed by washing with ultrapure water and then air drying for 1 day. TEM images of the sample-coated microgrids were captured using JEM1400. Figure 74 is a TEM image of the antibody sample. The scale bar represents 100 nm.

From the TEM images of the antibody/PEG₁₀ₖ-TA/Fe³⁺ MPN complex and the antibody/PEG₅ₖ-TA/Fe³⁺ MPN complex, spherical particles of approximately 30 nm were observed. From the TEM image of the antibody/TA/Fe³⁺ MPN complex, aggregated particles were observed. These confirmed formation of dispersed antibody/PEG₁₀ₖ-TA/Fe³⁺ MPN complexes and antibody/PEG₅ₖ-TA/Fe³⁺ MPN complexes.

### (18.5. Measurement of Buffering Effect of Antibody-Loaded MPN Complex)

### <Final Concentrations of Antibody, PEG₁₀ₖ-TA, TA, and Fe³⁺>

- Anti-NPC antibody: 660 nM
- PEG₁₀ₖ-TA: 16 µM (stock concentration: 80 µM)
- TA: 16 µM (stock concentration: 80 µM)
- Fe³⁺: 55 µM (stock concentration: 2.2 mM)

Each was prepared by dissolution in D-PBS(-) (pH 7.4). Fe³⁺ was prepared by dissolution in ultrapure water.

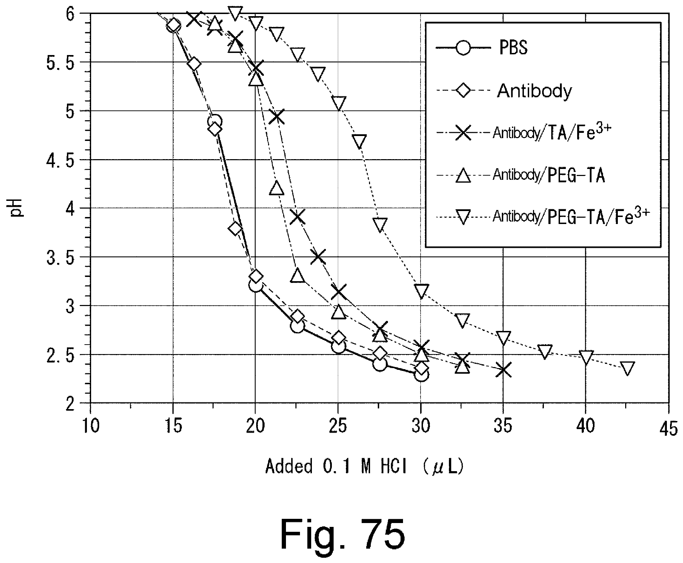

The antibody solution, PEG₁₀ₖ-TA solution, and Fe³⁺ solution, or antibody solution, TA solution, and Fe³⁺ solution were mixed by pipetting. In doing so, the molar ratio of PEG₁₀ₖ-TA/antibody and TA/antibody were set to 25. In addition, the molar ratio of iron ions was set to 3.3 equivalents relative to TA in PEG-TA. 0.1 N hydrochloric acid was added dropwise to the sample solution in 5 µL portions, and changes in pH were measured. Measurement was terminated once the pH had fallen to 3 or less and stabilized. The results are shown in Figure 75.

The antibody/TA/Fe³⁺ MPN complex and the antibody/PEG₁₀ₖ-TA/Fe³⁺ MPN complex were confirmed to suppress the decrease in pH upon addition of HCl. In particular, the antibody/PEG₁₀ₖ-TA/Fe³⁺ MPN complex was shown to markedly suppress the decrease in pH.

### (18.6. Evaluation of Blood Stability of Antibody-Loaded MPN Complex)

### <Final Concentrations of Antibody, PEG-TA, TA, and Fe³⁺>

- Anti-NPC antibody: 3.3 µM
- PEG₁₀ₖ-TA: 82.5 µM (stock concentration: 200 µM)
- Fe³⁺: 272 µM (stock concentration: 20 mM)
- TA: 82.5 µM (stock concentration: 200 µM)

The antibody, PEG₁₀ₖ-TA, and TA were prepared by dissolution in PBS(-), and Fe³⁺ was prepared by dissolution in ultrapure water.

### <Evaluation of Blood Stability>

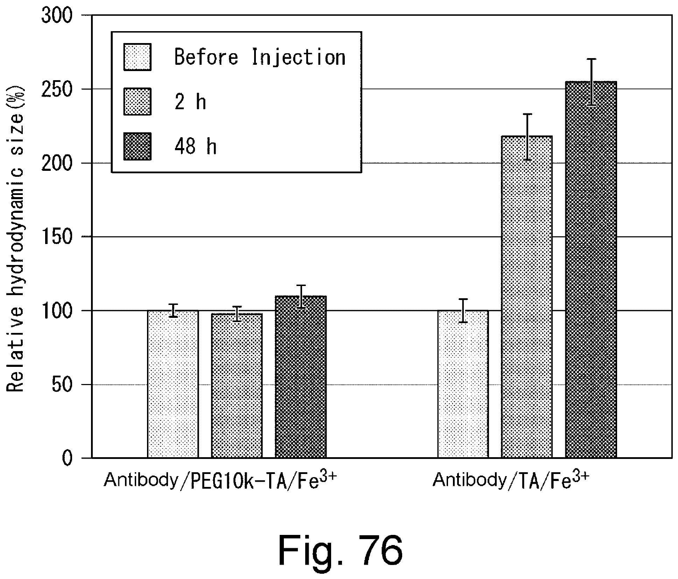

To Balb/C mice, 200 µL of the above prepared solution was administered via the tail vein. At 2 hours and 48 hours after sample administration, the mice were dissected and blood was collected. The blood was centrifuged at 5,000 × g for 10 minutes at 20°C, diluted 10-fold with D-PBS(-), and the particle size of the sample was measured by FCS. The results are shown in Figure 76.

When administered into the bloodstream, the particle size of the antibody/TA/Fe³⁺ MPN complex increased to 200% or more. This was considered to result from interaction of the antibody/TA/Fe³⁺ MPN complex with blood components. On the other hand, no change in the particle size of the antibody/PEG-TA/Fe³⁺ MPN complex was observed. These results showed that this MPN complex is stable even in the bloodstream, a dynamic environment.

### (18.7. Evaluation of Toxicity of Antibody-Loaded MPN Complex)

### <Final Concentrations of Antibody, PEG₁₀ₖ-TA, TA, and Fe³⁺>

- Anti-S100A4 antibody: 3.3 µM
- PEG₁₀ₖ-TA: 82.5 µM (stock concentration: 200 µM)
- Fe³⁺: 272 µM (stock concentration: 20 mM)
- TA: 82.5 µM (stock concentration: 200 µM)

The antibody, PEG₁₀ₖ-TA, and TA were prepared by dissolution in PBS(-), and Fe³⁺ was prepared by dissolution in ultrapure water.

### <Toxicity Evaluation>

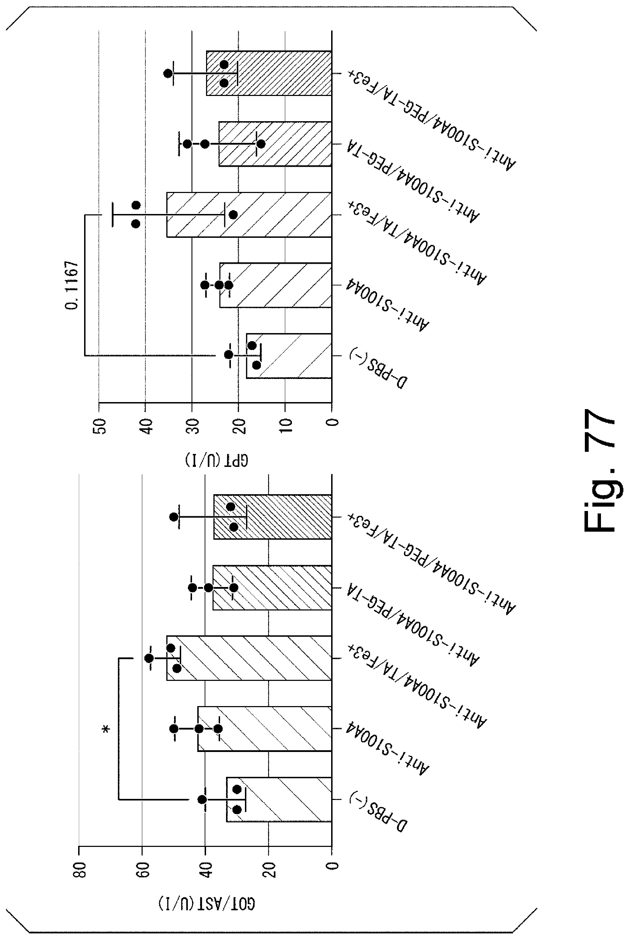

To model mice, 200 µL of the above prepared solution was administered via the tail vein three times every 4 days. Four days after the third administration, the mice were dissected and blood was collected. The blood was centrifuged at 5,000 × g for 10 minutes at 20°C, and the serum was collected. 10 µL of the serum was dropped onto the FUJI DRI-CHEM Slide (GOT or GPT) appropriate for each blood marker, and measured using DRI-CHEM. The results are shown in Figure 77.

Upon administration of the antibody/TA/Fe³⁺ MPN complex, increases in GOT and GPT were observed, and hepatotoxicity was observed. On the other hand, no increases in GOT or GPT were observed upon administration of the antibody/PEG-TA/Fe³⁺ MPN complex. These results confirmed that the antibody/PEG-TA/Fe³⁺MPN complex does not exhibit toxicity.

### (18.8 Therapeutic Effect of Therapeutic Intracellular Antibody-Loaded MPN Complex in Orthotopically Transplanted Breast Cancer Model)

### <Final Concentrations of Antibody, PEG-TA, TA, and Fe³⁺>

- Anti-S100A4 antibody: 3.3 µM
- PEG₁₀ₖ-TA: 82.5 µM (stock concentration: 200 µM)
- PEG₅ₖ-TA: 82.5 µM (stock concentration: 200 µM)
- Fe³⁺: 272 µM (stock concentration: 20 mM)
- TA: 82.5 µM (stock concentration: 200 µM)

The antibody, PEG-TA, and TA were prepared by dissolution in PBS(-), and Fe³⁺ was prepared by dissolution in ultrapure water.

The antibody solution, PEG-TA solution, and Fe³⁺ solution, or antibody solution, TA solution, and Fe³⁺ solution were mixed by pipetting. In doing so, the molar ratio of PEG₁₀ₖ-TA/antibody and TA/antibody were set to 25. In addition, the molar ratio of iron ions was set to 3.3 equivalents relative to TA in PEG-TA.

### <Preparation of 4T1-Luc Subcutaneous Tumor Mouse Model>

50 µL of a 4T1-Luc cell suspension (5.0 × 10⁷ cells/mL) was injected into the mammary gland of BALB/c mice.

### <Evaluation of Antitumor Effect>

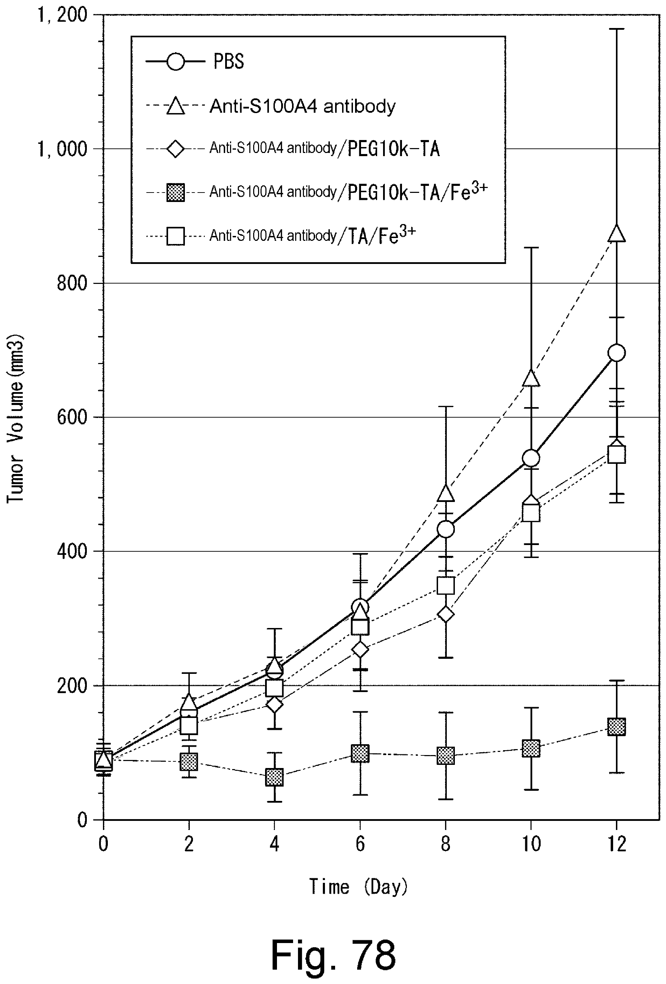

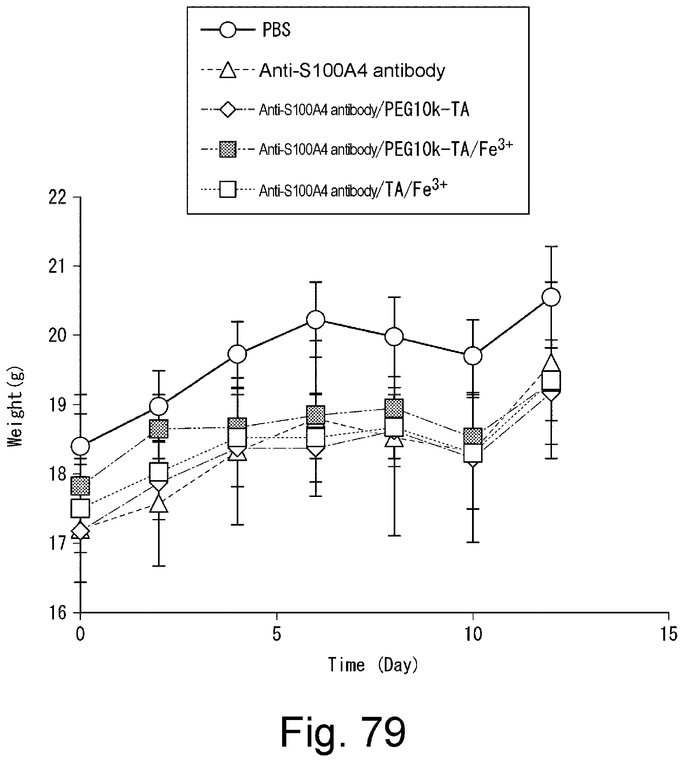

Using the time point at which the tumor size of the model mice reached approximately 20 mm³ as Day 0, 100 µL of the above sample solution (anti-S100A4 antibody: 50 µg/mouse) was administered via the tail vein on Days 0, 4, and 8. In addition, a group administered with D-PBS(-) was also provided for comparison. Tumor size was evaluated by measuring the tumor size using calipers every two days until Day 16. At the same time, the body weight of the mice was measured, and adverse events were also monitored. Figure 78 shows changes in tumor size over time, and Figure 79 shows changes in body weight over time. Tumor size was calculated according to the following equation (F1). $V = \left({W}^{2}\times L\right) / 2 \left(\begin{matrix}V : volume , W : short diameter , and L : \\ long diameter\end{matrix}\right)$ (F1)

Figure 78 showed that the tumor sizes in the group administered with the anti-S100A4 antibody/PEG-TA/Fe³⁺ MPN complex were significantly suppressed compared with the tumor sizes in the groups administered with other samples and with PBS(-). In addition, Figure 79 showed that all sample-administered groups exhibited similar changes in body weight.

### (18.9. Confirmation of Mechanism of Anticancer Effect by Immunostaining)

### <Immunostaining Observation>

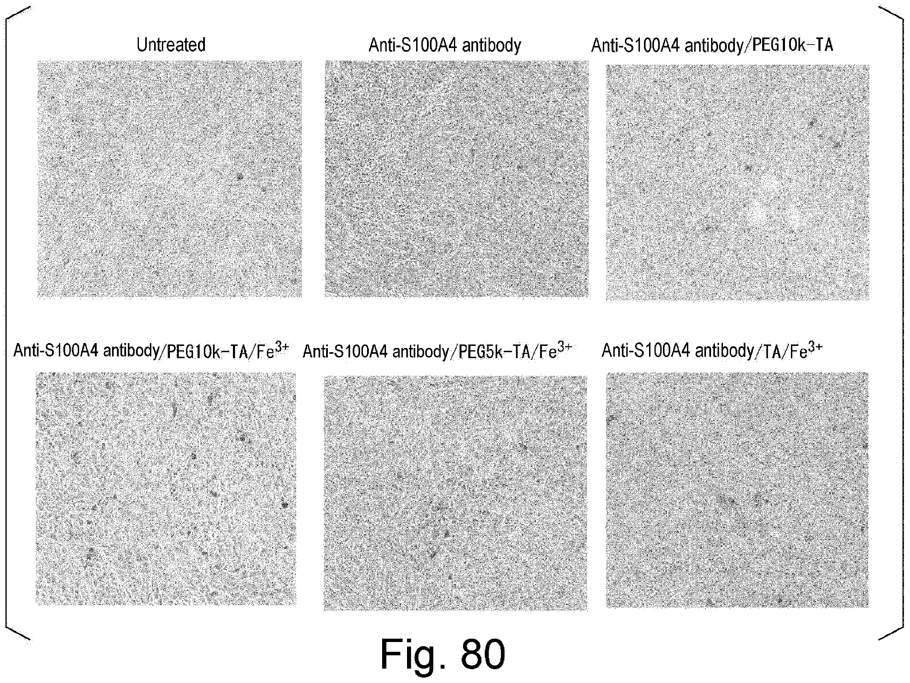

Paraffin blocks were prepared from the tumors prepared in Section 18.8. above. Sections of the paraffin blocks with a thickness of 10 µm were prepared using a cryostat, and deparaffinized with xylene and alcohol. The sections were incubated in 30% hydrogen peroxide for 10 minutes to remove endogenous peroxidase. Thereafter, the sections were washed with a PBST solution and a 10% normal goat serum solution, and incubated overnight at 4°C with Anti-p53 antibody diluted 50-fold with PBST. Thereafter, the sections were washed with PBST and incubated at room temperature for 30 minutes with an HRP-labeled Anti-Rabbit antibody diluted 1,000-fold with PBST. Finally, HRP was developed with TMB and the tissue was stained with hematoxylin for observation. Figure 80 shows the observed images.

In the images of the tumor samples administered with the anti-S100A4 antibody/PEG-TA/Fe³⁺ MPN complex, brown spots derived from the p53 protein were observed. These results confirmed increased expression of the p53 protein.

### (18.10. Confirmation of Mechanism of Anticancer Effect by TUNEL Assay)

### <TUNEL Assay>

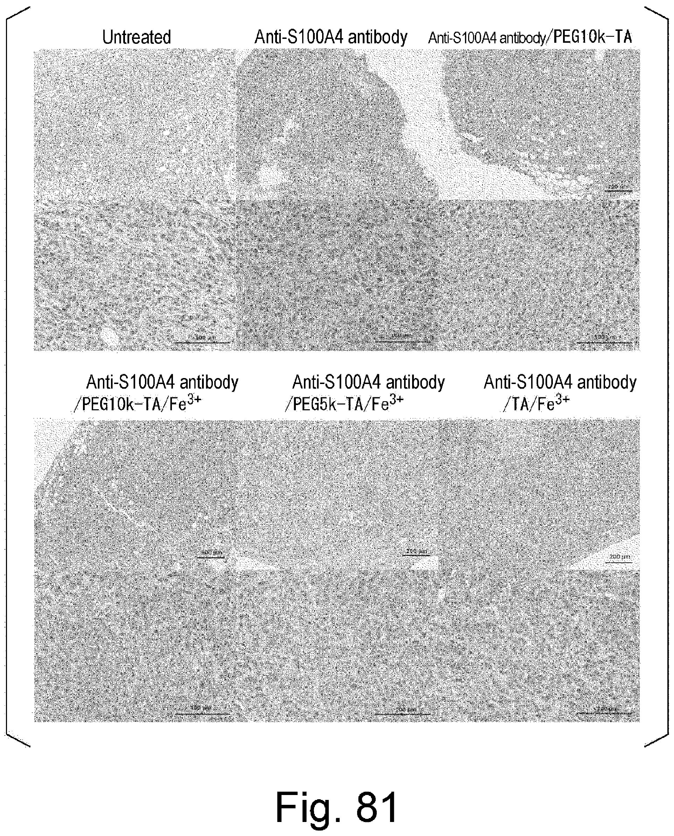

Paraffin blocks were prepared from the tumors prepared in Section 18.8. above using paraffin. Sections of the paraffin blocks with a thickness of 10 µm were prepared using a cryostat, and deparaffinized with xylene and alcohol. Thereafter, in accordance with the TUNEL assay kit protocol, the section samples were subjected to proteinase K treatment, endogenous peroxidase inactivation, terminal deoxynucleotidyl transferase reaction, labeling reaction, DAB staining, and counterstaining. Thereafter, observation was performed using an all-in-one fluorescence microscope. The results are shown in Figure 81.

In the images of the tumor samples administered with the anti-S100A4 antibody/PEG-TA/Fe³⁺ MPN complex, brown spots derived from the apoptotic cells were observed. These results confirmed that the anti-S100A4 antibody/PEG-TA/Fe³⁺ MPN complex induced apoptosis in cancer cells.

### [Experimental Example 19]

### <Evaluation of Blood Retention of GalNac-Modified β-Gal-Loaded MPN Complex>

### (19.1. Overview)

Blood retention of the β-Gal ternary complex (β-Gal/PEG-TA/Fe³⁺) was evaluated.

### (19.2. Reagents)

Unless otherwise specified, commercially available reagents were used as purchased.
- β-D-Galactosidase (β-Gal, Mw: 540 kDa; FUJIFILM Wako Pure Chemical Corporation)
- PEG-TA (Mn = 11,700)
- Alexa Fluor 647-β-Gal
- D-PBS(-) (FUJIFILM Wako Pure Chemical Corporation)
- BALB/c mice (Charles River Japan Inc.)
- Passive Lysis Buffer (Promega)

### (9.3. Measurement Instruments)

- Multi-plate reader (Spark, Tecan Group Ltd.)

### (9.4. Evaluation of In Vivo Kinetics)

The Alexa Fluor 647-β-Gal solution, PEG₁₀ₖ-TA solution, and Fe³⁺ solution were mixed by pipetting. In doing so, the polymer/β-Gal molar ratio was set to 250. In addition, the molar ratio of iron ions was set to 3.3 equivalents relative to TA in the polymer. Finally, PBS(-) was added to adjust the concentration.

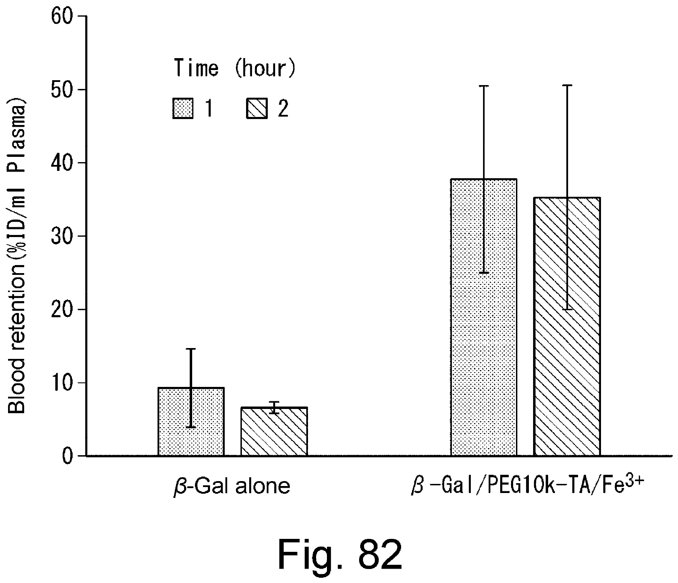

To model mice, 200 µL of the above prepared solution was administered via the tail vein. At 1 hour and 2 hours after sample administration, blood was collected, centrifuged at 5,000 × g for 10 minutes at 20°C, and 100 µL of the plasma component was collected, to which 200 µL of Passive Lysis Buffer was added. Thereafter, the fluorescence intensity of the solution (Ex/Em: 640 nm/680 nm) was measured using TECAN to evaluate blood retention. The results are shown in Figure 82.

Blood retention of the Alexa647-β-Gal-loaded MPN complex was improved 4-fold at 1 hour and 6-fold at 2 hours compared with Alexa647-β-Gal.

### [Experimental Example 20]

### <Evaluation of Targeting Function of GalNac-Modified β-Gal-Loaded MPN Complex>

### (20.1. Overview)

Functional evaluation of the β-Gal ternary complex was performed. Specifically, the amount of cellular uptake of the GalNac-loaded MPN complex was measured in the presence and absence of an inhibitor.

### (10.2. Reagents)

Unless otherwise specified, commercially available reagents were used as purchased.
- Alexa Fluor 647-β-Gal
- PEG-TA (Mn = 11,700)
- Tannic acid (Mw: 1,701) (FUJIFILM Wako Pure Chemical Corporation)
- D-PBS(-) (FUJIFILM Wako Pure Chemical Corporation)
- Dulbecco's modified Eagle's medium (DMEM, Sigma Aldrich Co., llc.)
- Fetal bovine serum (FBS, Biosera Inc.)
- Trypsin-EDTA solution (Trp, Sigma Life Science Co., Ltd.)
- Penicillin/streptomycin (PS, Sigma Life Science Co., Ltd.)
- Passive Lysis Buffer (Promega)
- N-Acetylgalactosamine (GalNac, FUJIFILM Wako Pure Chemical Corporation)
- Human liver cancer cells (HepG2 cells, cells overexpressing ASGPR (asialoglycoprotein receptor))

### (American Type Culture Collection, ATCC)

### (10.3. Measurement Instruments)

- Flow cytometer (Guava easyCyte 6-2L, Merck Millipore, Ex/Em: 642/661 nm)

### (10.4. Examination of Cellular Uptake Efficiency of β-Gal-Loaded MPN Complex)

### <Final Concentrations of Alexa647-β-Gal, PEG-TA, GalNac-PEG-TA, Fe³⁺, and GalNac>

- Alexa647-β-Gal (β-Gal): 460 nM
- PEG-TA: 115 µM
- GalNac-PEG-TA: 115 µM
- Fe³⁺: 380 µM (stock concentration: 6.1 mM)
- GalNac: 10 mM (stock concentration: 20 mM)

Alexa647-β-Gal, PEG-TA, GalNac-PEG-TA, and GalNac were prepared by dissolution in PBS(-), and Fe³⁺ was prepared by dissolution in ultrapure water.

The Alexa647-β-Gal solution, PEG-TA solution or GalNac-PEG-TA solution, and Fe³⁺ solution were mixed by pipetting. In doing so, the polymer/β-Gal molar ratio was set to 250. In addition, the molar ratio of iron ions was set to 3.3 equivalents relative to TA in the polymer. Finally, PBS(-) was added to adjust the concentration.

### [Evaluation of Cellular Uptake of β-Gal Ternary Complex]

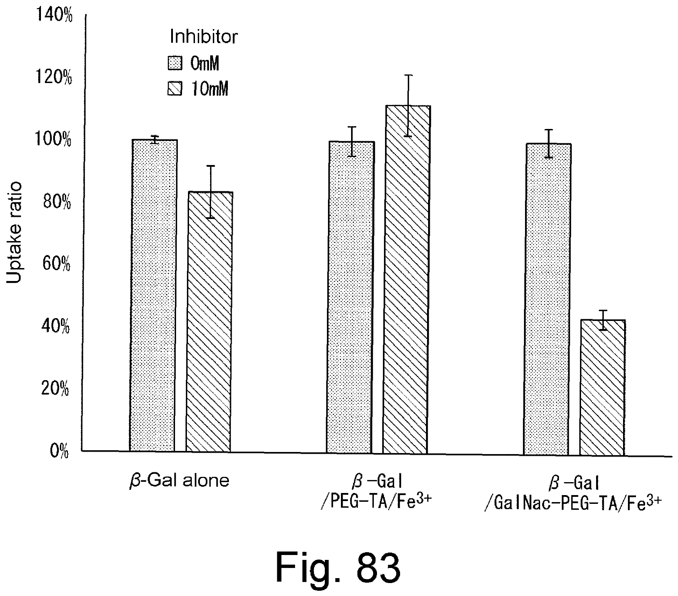

FBS and PS were added to DMEM so as to achieve 10 wt% and 2 wt%, respectively, to prepare a cell culture medium. HepG2 cells were suspended in the cell culture medium to prepare a cell suspension of 1.25 × 10⁵ cells/mL. 400µL of the cell suspension was seeded into a 24-well plate (5.0 × 10⁴ cells per well) and incubated at 37°C for 24 hours. After removal of the medium, the cells were washed once with PBS, after which 200 µL of each prepared Alexa647-β-Gal-loaded MPN complex solution, 200 µL of a GalNac solution as an inhibitor, and a PBS solution were added, and the cells were incubated at 37°C for 24 hours. After incubation for a predetermined period of time, the solution was removed, the cells were washed twice with PBS, and 150 µL of Trp was added, followed by incubation at 37°C for 7 minutes. Subsequently, 150 µL of 10% FBS-PBS was added, and fluorescence derived from Alexa647 (Ex/Em: 642/664 nm) was measured with a flow cytometer (FCM) to evaluate the cellular uptake of each sample. The uptake amount of Alexa647-β-Gal in the absence of the inhibitor was taken as 100%. The obtained results are shown in Figure 83.

As a result, the cellular uptake of Alexa647-β-Gal/PEG-TA/Fe³⁺ was not reduced even in the presence of GalNac (inhibitor). In contrast, the cellular uptake of Alexa647-β-Gal/GalNac-PEG-TA/Fe³⁺ was reduced in the presence of GalNac (inhibitor).

### [Experimental Example 20]

### <Evaluation of In Vivo Kinetics of RNP/PEG-TA/Fe³⁺ Complex>

### (21.1. Overview)

The *in vivo* kinetics of the RNP/PEG-TA/Fe³⁺ complex was evaluated in a tumor-bearing mouse model subcutaneously implanted with 4T1-Luc cells (mouse colon cancer cells).

### (21.2. Reagents, Cells, and Animals)

- Label IT Nucleic Acid Labeling Kit, Cy5 (Mirus)
- Cas9 Nuclease protein NLS (Cas9) (NIPPON GENE)
- sgRNA (Luciferase-2) (GenScript)
- Iron(III) chloride hexahydrate (FeCl₃·6H₂O) (Fe³⁺) (FUJIFILM Wako Pure Chemical Corporation)
- PEG₁₀ₖ-TA (Mn = 11,701)
- PEG₅ₖ-TA (Mn = 6,701)
- 4T1-Luc cells (mouse breast cancer-derived cell line stably expressing luciferase) (American Type Culture Collection, ATCC)
- BALB/c mice (Charles River)

### (21.3. Instruments and Equipment)

- Countess (Thermo Fisher Scientific)
- Multi-beads shocker (Yasui Kikai)
- Multi-plate reader (Spark) (Tecan)

### (21.4. In Vivo Kinetics of RNP/PEG-TA/Fe³⁺ Complex)

Blood retention and organ accumulation of the RNP/PEG-TA/Fe³⁺ MPN complex were evaluated. An RNP sample labeled with Cy5 was prepared and intravenously administered to a 4T1-Luc subcutaneous tumor mouse model, and the contents in the blood and each organ after a predetermined period of time were measured by fluorescence intensity measurement. In the present Experimental Example, RNP alone was used as a comparator for the RNP/PEG-TA/Fe³⁺ MPN complex.

### <Preparation of Cy5-Labeled RNP>

Label IT Reagent and RNA were mixed at a Label IT Reagent:RNA ratio of 0.25:1 (v:w) and incubated at 37°C for 1 hour. Thereafter, purification was performed using a G50 column to prepare Cy5-labeled sgRNA.

Cas9 and Cy5-labeled sgRNA were mixed, and centrifugation was performed twice at 12,000 × g for 5 minutes using an ultrafiltration membrane (MWCO: 10 kDa) to prepare a Cas9-sgRNA solution (RNP solution).

### <Final Concentrations of RNP and PEG-TA>

- Cas9 Nuclease protein NLS: 1.0 µM
- sgRNA: 1.0 µM
- PEG₁₀ₖ-TA (Mn = 11,701): 100 µM
- PEG₅ₖ-TA (Mn = 6,701): 100 µM
- Fe³⁺: 333 µM

Cas9-sgRNA and PEG-TA were prepared by dissolution in D-PBS(-). Fe³⁺ was prepared by dissolution in pure water.

PEG-TA was added to the RNP, and the mixture was allowed to stand at room temperature for 15 minutes. Subsequently, Fe³⁺ was added, followed by standing at room temperature for 15 minutes to prepare an RNP/PEG-TA/Fe³⁺ MPN complex solution. In doing so, the molar ratio of PEG-TA/RNP was set to 100. In addition, the molar ratio of iron ions was set to 3.3 equivalents relative to TA in PEG-TA.

### <Preparation of 4T1-Luc Subcutaneous Tumor Mouse Model>

100 µL of a 4T1-Luc cell suspension (5.0 × 10⁷ cells/mL) was subcutaneously injected into BALB/c mice.

### <Evaluation of In Vivo Kinetics>

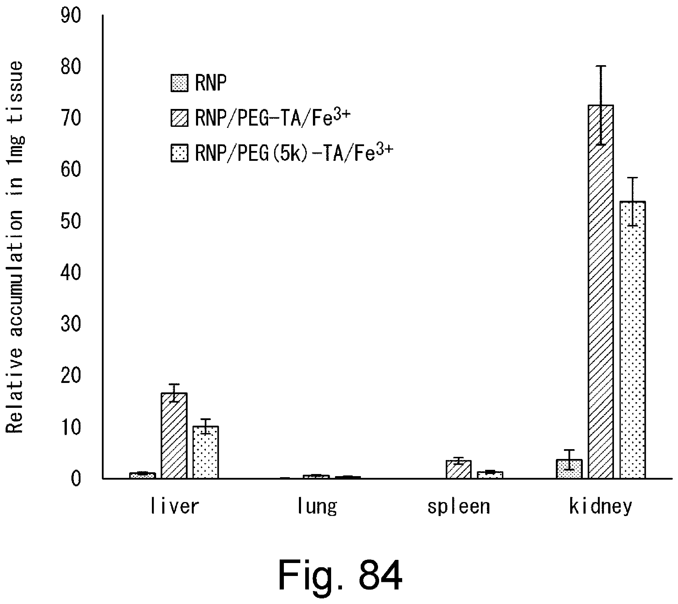

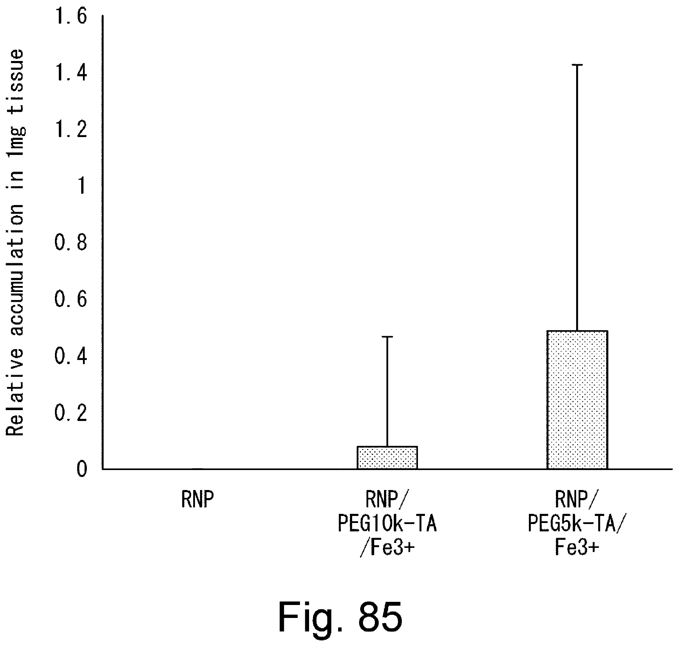

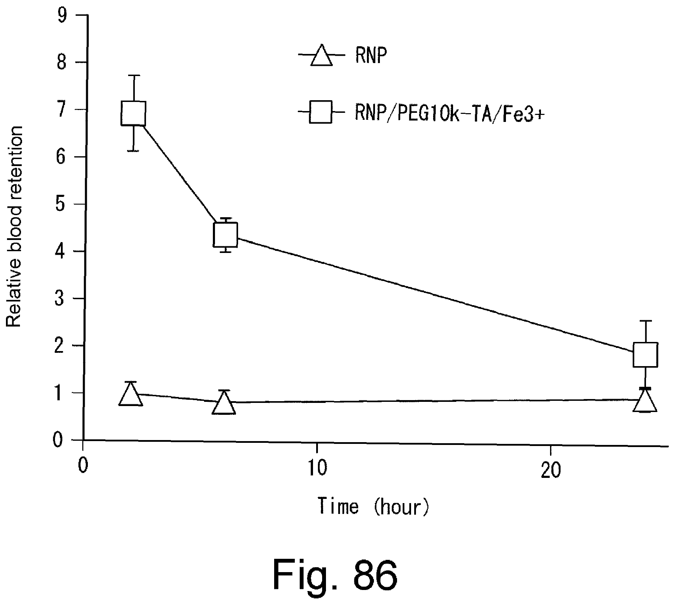

100 µL of the above prepared sample solution was administered via the tail vein to 4T1-Luc tumor-bearing model mice in which tumors had become visible by visual observation. Six hours after sample administration, the mice were dissected, blood and various organs were collected, and Passive Lysis Buffer was added thereto in an amount corresponding to three times the weight, followed by homogenization using a Multi-Beads Shocker. Blood was also collected from the tail vein after 2 hours and after 24 hours. Thereafter, centrifugation was performed at 10,000 × g for 5 minutes at 4°C, and 50 µL of the supernatant was transferred to a 96-well plate, after which the fluorescence intensity of RNP was measured using a multi-plate reader to evaluate *in vivo* kinetics. Figure 84 is a graph showing the results of evaluation of organ accumulation. Figure 85 is a graph showing the results of evaluation of tumor accumulation. Figure 86 is a graph showing the results of evaluation of blood retention.

### (11.5. Analysis)

The *in vivo* kinetics of the RNP/PEG-TA/Fe³⁺ MPN complex were improved relative to RNP alone in terms of blood retention, organ accumulation, and tumor accumulation.

### [Experimental Example 22]

### <Functional Evaluation of RNP-Loaded MPN Complex in Cultured Cells>

### (22.1. Overview)

An MPN complex using Luc gene-editing RNP was introduced into HeLa-Luc cells, and toxicity evaluation was performed by CCK assay. In addition, knockout efficiency was measured based on luciferase luminescence intensity.

### (22.2. Reagents and Cell Lines)

- Cas9 Nuclease protein NLS (Cas9) (NIPPON GENE)
- Luciferase knock gene knockout sgRNA (sgRNA) (GenScript)
- Iron(III) chloride hexahydrate (FeCl₃ · 6H₂O) (Fe³⁺) (FUJIFILM Wako Pure Chemical Corporation)
- PEG₁₀ₖ-TA (Mn = 11,701)
- PEG₁₀ₖ-TET-TA (Mn = 11,847)
- PEG₁₀ₖ-TEP-TA (Mn = 11,890)
- PEG₁₀ₖ-S-TA (Mn = 11,903)
- Luciferase Assay System (Promega)
- Cell Counting Kit-8 (Dojindo Laboratories)
- Roswell Park Memorial Institute medium (RPMI, Sigma-Aldrich)
- Fetal bovine serum (FBS, Biosera)
- Trypsin-EDTA solution (Sigma-Aldrich)
- Penicillin/streptomycin (Sigma-Aldrich)
- HeLa-Luc cells (aneuploid epithelial-like cell line stably expressing luciferase) (American Type Culture Collection, ATCC)
- ProDeliverIn (OZ Biosciences)

### (12.2. Measurement Instruments)

- Glomax 96 microplate luminometer (Promega)
- Multi-plate reader (Spark) (Tecan)

### (22.3. Evaluation of Knockout Efficiency)

### <Final Concentrations of RNP, Polymer, and FeCl₃·6H₂O>

- Cas9: 90 nM
- sgRNA: 90 nM
- Polymer (PEG₁₀ₖ-TA, PEG₁₀ₖ-TET-TA, PEG₁₀ₖ-TEP-TA, and PEG₁₀ₖ-Spermine-TA): 9 µM
- Fe³⁺: 30 µM

Cas9-sgRNA and the polymers were prepared by dissolution in D-PBS(-). Fe³⁺ was prepared by dissolution in pure water.

Cas9 and sgRNA were mixed, and centrifugation was performed twice at 12,000 × g for 5 minutes using an ultrafiltration membrane (MWCO: 10 kDa) to prepare a Cas9-sgRNA solution (RNP solution). Thereafter, the polymer was added, followed by standing at room temperature for 15 minutes to prepare an RNP/polymer solution. Fe³⁺ was further added, followed by standing at room temperature for 15 minutes to prepare an RNP/polymer/Fe³⁺ MPN complex solution. In addition, as a positive control, ProDeliverIn, which is a cationic reagent, was mixed at a ratio of ProDeliverIn/RNP = 1 µg/1 µL to prepare an RNP/ProDeliverIn solution.

### <Cell Survival Rate Measurement by CCK Assay>

HeLa-Luc cells were seeded in a 96-well plate at 2.0 × 10³ cells/plate and incubated at 37°C under 5% CO₂ for 24 hours. The above RNP solution, RNP/PEG₁₀ₖ-TA/Fe³⁺ MPN complex solution, RNP/PEG-TET-TA/Fe³⁺ MPN complex solution, RNP/PEG-TEP-TA/Fe³⁺ MPN complex solution, RNP/PEG-S-TA/Fe³⁺ MPN complex solution, and RNP/ProDeliverIn solution were added to each well at 100 µL/well, followed by incubation for 72 hours. After washing with 150 µL/well of D-PBS(-), 110 µL/well of a CCK measurement solution prepared by mixing 10 µL of CCK and 100 µL of PBS(-) was added, followed by incubation for 1 hour. Thereafter, the absorbance at 460 nm was measured using a plate reader to determine cell survival rate.

### <Evaluation of Knockout Efficiency>

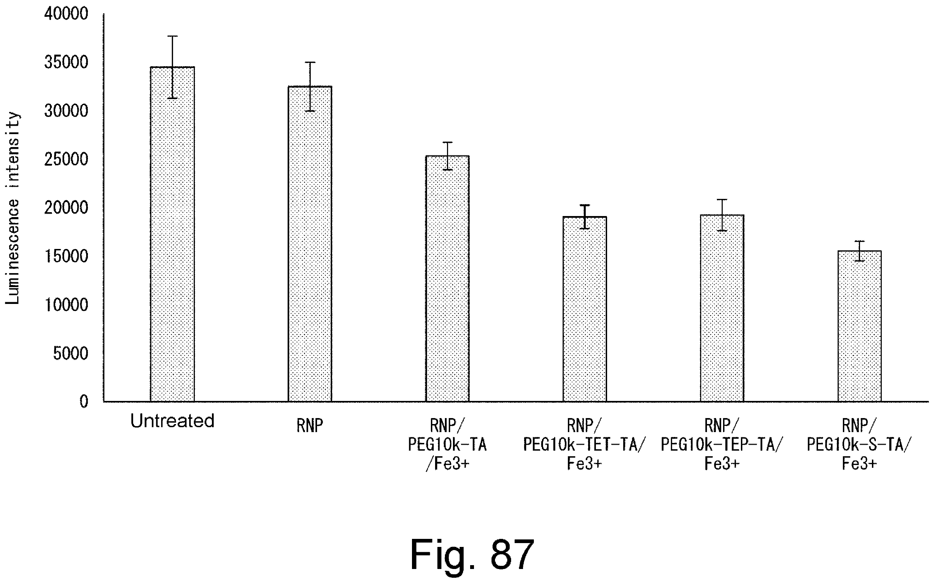

After cell survival rate had been determined, the medium was collected, the cells were washed with D-PBS(-), and 50 µL of Cell Culture Lysis was added to each well, followed by incubation for 15 minutes. Thereafter, 20 µL of the lysate was transferred to a dish for fluorescence measurement, to which luciferin was added, and the knockout efficiency of the luciferase gene was evaluated by measuring luciferase luminescence intensity using a plate reader and normalizing it to cell survival rate. Figure 87 is a graph showing the results of measuring luciferase luminescence intensity.

### (22.4. Analysis)

In cells to which the RNP/polymer/Fe³⁺ MPN complex solution was added, a marked decrease in luciferase luminescence intensity was observed compared with untreated cells and cells administered with RNP alone. In addition, binding of an ethylenediamine molecule to PEG-TA improved the gene knockout effect.

### [Experimental Example 23]

### <Evaluation of In Vivo Kinetics of RNP-Loaded MPN Complex Formation and In Vivo Genome Editing>

### (23.1. Overview)

Functional evaluation of the RNP ternary complex (RNP/PEG-TA/Fe³⁺) was performed. Specifically, an RNP sample was administered to genetically modified mice (Ai9 tdTomato mice) that emit red fluorescence upon genome editing, and the fluorescence intensity of each organ was measured.

### (23.2. Reagents)

- Cas9 Nuclease protein NLS (Cas9, NIPPON GENE)
- sgRNA (Ai9_L) (GenScript Japan)
- sgRNA (Ai9_R) (GenScript Japan)
- Iron(III) chloride hexahydrate (FeCl₃·6H₂O) (Fe³⁺) (FUJIFILM Wako Pure Chemical Corporation)
- PEG₁₀ₖ-TA (Mn = 11,701)
- PEG₁₀ₖ-S-TA (Mn = 11,903)
- GalNac-PEG-TA (Mₙ = 12,400)
- D-PBS(-) (FUJIFILM Wako Pure Chemical Corporation)
- LipidLaunch^{™} SORT LNP Exploration Kit (Cayman Chemical)
- Ai9 td-Tomato mouse (The Jackson Laboratory)

### (23.3. Measurement Instruments)

- Multi-plate reader (Spark) (Tecan)
- *In vivo* imaging system (PerkinElmer)

### (23.4. Measurement of Genome Editing Efficiency)

In order to evaluate the gene knockout efficiency of the RNP MPN complex, the RNP/polymer/Fe³⁺ MPN complex was intravenously injected into a 4T1-Luc subcutaneous tumor mouse model, and the luciferase luminescence intensity of the tumor was measured after a predetermined period of time. RNP alone and LNP-encapsulated RNP were used as comparators for the RNP/polymer/Fe³⁺ MPN complex.

### <Final Concentrations of Cas9-sgRNA, Polymer, and Fe³⁺>

- Cas9: 2 µM
- sgRNA: 2 µM (Ai9_L 1 µM + Ai9_R 1 µM)
- Polymer (PEG₁₀ₖ-TA and PEG-S-TA): 200 µM
- Fe³⁺: 660 µM

Cas9-sgRNA and the polymers were prepared by dissolution in D-PBS(-). Fe³⁺ was prepared by dissolution in pure water.

Cas9 and sgRNA were mixed, and centrifugation was performed twice at 12,000 × g for 5 minutes using an ultrafiltration membrane (MWCO: 10 kDa) to prepare a Cas9-gRNA solution (RNP solution). Thereafter, each polymer solution was mixed, followed by standing at room temperature for 15 minutes to prepare an RNP/polymer solution. Fe³⁺ was further added, followed by standing at room temperature for 15 minutes to prepare an RNP/polymer/Fe³⁺ MPN complex solution.

### <Final Concentrations of Cas9-sgRNA and LNP>

- Cas9: 288 nM
- sgRNA: 288 nM
- LNP: 800 µM

Cas9-sgRNA was prepared by dissolution in D-PBS(-).

In accordance with the kit instructions, the respective solutions of the LipidLaunch^{™} SORT LNP Exploration Kit were dispensed into a 5 mL tube so that the total volume was 1 mL. Thereafter, 20 mM MES buffer (pH 6.0) containing 50 mM NaCl, RNP, and LNP were mixed at 0.09 mL/min, 0.09 mL/min, and 0.02 mL/min, respectively. Thereafter, centrifugation was performed twice at 3,500 × g for 15 minutes to remove EtOH, and then centrifugation was further performed five times at 3,500 × g for 15 minutes to concentrate the LNP to the desired amount.

### <Evaluation of Gene Editing Efficiency and Blood Toxicity>

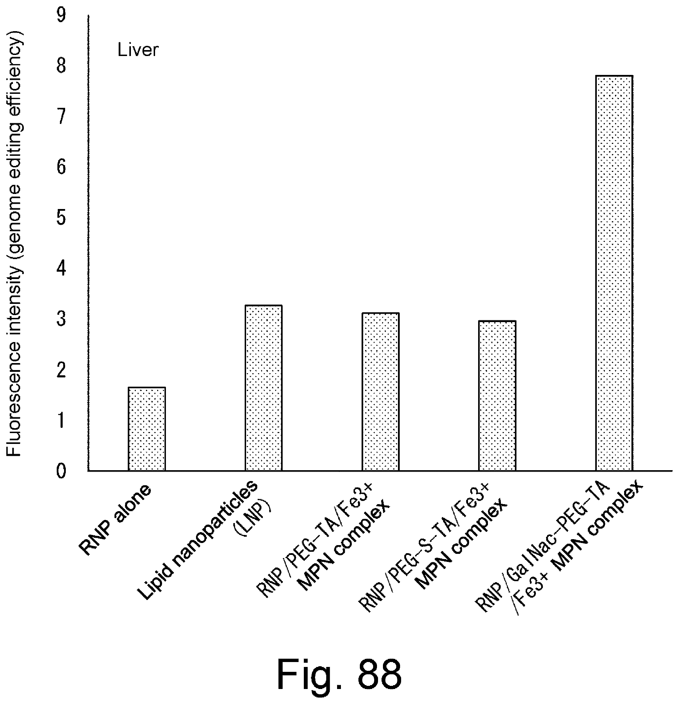

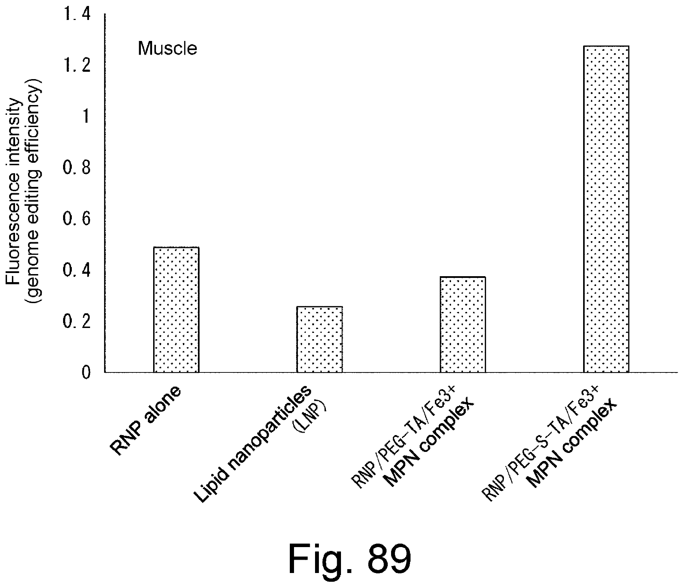

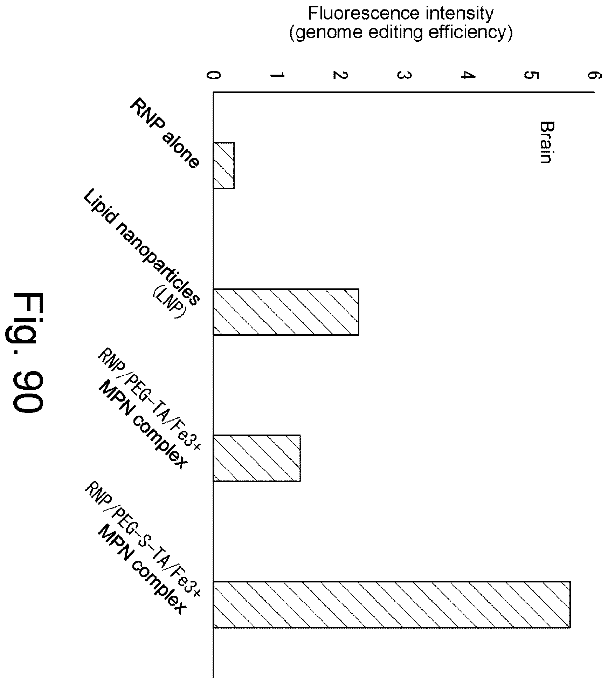

To Ai9 td-Tomato mice, 200 µL of the above prepared solution and D-PBS(-) were administered via the tail vein. Eight days after sample administration, various organs were collected and *in vivo* fluorescence imaging (IVIS) was performed. Genome editing efficiencies in the liver, muscle, and brain, calculated based on fluorescence intensity, are shown in Figures 88, 89, and 90, respectively.

By targeting hepatocytes, the GalNac-loaded RNP MPN complex exhibited higher genome editing efficiency in the liver than the RNP MPN complex, LNP, and RNP alone. In addition, the RNP MPN complex prepared using PEG-S-TA exhibited higher genome editing efficiency in muscle and brain than the RNP MPN complex, LNP, and RNP alone. In addition, the RNP MPN complex exhibited higher genome editing efficiency than RNP alone in various organs. These results demonstrated the potential of the RNP MPN complex as a tool capable of genome editing in various organs *in vivo.*

### [Summary]

The present drug delivery system composed of a polyphenol-modified polymer and metal ions was able to improve blood retention, blood stability, and cytoplasmic translocation of a bioactive protein. In addition, it was confirmed that the present drug delivery system can encapsulate not only proteins but also a wide variety of molecules and form MPN complexes. In addition, it was shown that use of the present drug delivery system can improve the *in vivo* pharmacokinetics and therapeutic effects of a wide variety of drugs. Furthermore, it was shown that introduction of a tissue-targeting molecule or a cationic molecule into the polyphenol-modified polymer can enhance the activity thereof.

The respective configurations in each embodiment and combinations thereof are merely examples, and additions, omissions, substitutions, and other modifications of the configurations may be made without departing from the spirit of the present invention. Furthermore, the present invention is not limited to the respective embodiments and is limited only by the scope of the claims.

### Industrial Applicability

According to the present invention, a technique for efficiently delivering a biomolecule into the cytoplasm can be provided.

## Claims

1. A complex of a biocompatible polymer-modified polyphenol, a metal ion, and a biomolecule.

2. The complex according to claim 1, wherein the polyphenol is at least one selected from the group consisting of tannic acid, gallic acid, and derivatives thereof.

3. The complex according to claim 1 or 2, wherein the biocompatible polymer comprises at least one selected from the group consisting of polyethylene glycol, acrylic resins, polyamino acids, polyvinylamine, polyallylamine, polynucleotides, polyacrylamide, polyethers, polyesters, polyurethanes, polysaccharides, and copolymers thereof.

4. The complex according to claim 1 or 2, wherein the biocompatible polymer has a weight-average molecular weight of 1,000 to 50,000.

5. The complex according to claim 1 or 2, wherein the metal ion is an iron(III) ion, a cobalt(II) ion, or a zirconium(IV) ion.

6. The complex according to claim 1 or 2, wherein the biomolecule is an antibody or an antigen-binding fragment thereof, a physiologically active protein, a nucleic acid, a lipid nanoparticle, or inorganic/organic microparticles.

7. A biocompatible polymer-modified polyphenol.

8. A kit for delivering a biomolecule into the cytoplasm by formation of a complex with the biomolecule and administration of the complex to a subject, the kit comprising a biocompatible polymer-modified polyphenol and a metal ion.
